# EUROPEAN PATENT SPECIFICATION

(11) **EP 1 037 977 B1**
(45) Date of publication and mention of the grant of the patent: **26.08.2009**
(21) Application number: 98959117.7
(22) Date of filing: 17.12.1998
(51) Int. Cl.: C12N 15/12, C07K 14/47, C07K 16/18, C12Q 1/68

(54) **EXTENDED cDNAs FOR SECRETED PROTEINS**
VERLÄNGERTE CDNS, DIE FÜR SEKRETIERTE PROTEINE KODIEREN
ADNc PROLONGES POUR PROTEINES SECRETEES

(30) Priority: 17.12.1997 US 69957 P; 09.02.1998 US 74121 P; 13.04.1998 US 81563 P; 10.08.1998 US 96116 P
(43) Date of publication of application: 27.09.2000
(73) Proprietor: Serono Genetics Institute S.A., 91030 Evry Cedex (FR)
(72) Inventor: BOUGUELERET, Lydie, F-92170 Vanves (FR); DUCLERT, Aymeric, F-94100 Saint-Maur (FR); DUMAS MILNE EDWARDS, Jean-Baptiste, F-75006 Paris (FR)
(74) Representative: Brasnett, Adrian Hugh
(86) International application number: PCT/IB1998/002122
(87) International publication number: WO 1999/031236

(56) References cited:
- EP-A- 0 625 572
- WO-A-96/34981
- WO-A-97/07198
- WO-A-99/06549
- DATABASE EMBL Human DNA sequence from clone 212A2 HUNT A.: XP002299551 Database accession no. Z95114
- DUCHATEAU P.N. ET AL.: "Apolipoprotein L, a new human high density lipoprotein apolipoprotein expressed by the pancreas. Identification, cloning, characterization, and plasma distribution" JOURNAL OF BIOLOGICAL CHEMISTRY, vol. 272, no. 41, 10 October 1997 (1997-10-10), pages 25576-25582, XP002136974 ISSN: 0021-9258
- Database EMBL, entry HS695112 Accession number R50695 24 May 1995 95% identity with Seq.ID:40 nt.1-384 XP002097725
- CARNINCI P. ET AL.: "High-efficiency full-length cDNA cloning by biotinylated CAP trapper" GENOMICS, vol. 37, no. 3, 1 November 1996 (1996-11-01), pages 327-336, XP002081729 cited in the application
- KATO S. ET AL.: "Construction of a human full-length cDNA bank" GENE, vol. 150, 1994, pages 243-250, XP002081364 cited in the application
- TASHIRO K. ET AL.: "Signal sequence trap: a cloning strategy for secreted proteins and type I membrane proteins" SCIENCE, vol. 261, 30 July 1993 (1993-07-30), pages 600-603, XP000673204
- YOKOYAMA-KOBAYASHI M. ET AL.: "A signal sequence detection system using secreted protease activity as an indicator" GENE, vol. 163, 1995, pages 193-196, XP002053953
- HEIJNE VON G.: "A new method for predicting signal sequence cleavage sites" NUCLEIC ACIDS RESEARCH, vol. 14, no. 11, 1986, pages 4683-4690, XP002053954 cited in the application
- LOCKHART D.J. ET AL.: "Expression monitoring by hybridization to high-density oligonucleotide arrays" BIO/TECHNOLOGY, no. 14, 14 December 1996 (1996-12-14), pages 1675-1680, XP002074420

<110> Dumas Milne Edwards. Jean-Bapciste
   Duclert, Aymeric
   Bougueleret, Lydie
<120> Extended cDNAS for Secreted proteins
<130> GENSET.019A
<160> 41
<170> Patent.pm
<210> 1
   <211> 47
   <212> RNA
   <213> Artificial Sequence
<220>
   <221> In vitro transcription produce
   <221> modified_base
   <222> (1)... (1)
   <223> m7g
<400> 1
   ngcauccuac ucccauccaa uuccacccua acuccuccca ucuccac 47
<210> 2
   <211> 46
   <212> RNA
   <213> Artificial Sequence
<220>
   <223> In vitro transcription product
<400> 2
   gcauccuacu cccauccaau uccacccuaa cuccucccau cuccac 46
<210> 3
   <211> 25
   <212> DNA
   <213> Artificial Sequence
<220>
   <223> In vitro transcription product
<400> 3
   atcaagaatt cgcacgagac catta 25
<210> 4
   <211> 25
   <212> DNA
   <213> Artificial Sequence
<220>
   <223> Oligonucleotide
<400> 4
   taatggtctc gtgcgaattc ttgat 25
<210> 5
   <211> 25
   <212> DNA
   <213> Artificial Sequence
<220>
   <223> Oligonucleotide
<400> 5
   ccgacaagac caacgtcaag gccgc 25
<210> 6
   <211> 25
   <212> DNA
   <213> Artificial Sequence
<220>
   <223> Oligonucleotide
<400> 6
   tcaccagcag gcagtggctt aggag 25
<210> 7
   <211> 25
   <212> DNA
   <213> Artificial Sequence
<220>
   <223> Oligonucleotide
<400> 7
   agtgattcct gctactttgg atggc 25
<210> 8
   <211> 25
   <212> DNA
   <213> Artificial Sequence
<220>
   <223> Oligonucleotide
<400> 8
   gcttggtctt gttctggagt ttaga 25
<210> 9
   <211> 25
   <212> DNA
   <213> Artificial Sequence
<220>
   <223> Oligonucleotide
<400> 9
   tccagaatgg gagacaagcc aattt 25
<210> 10
   <211> 25
   <212> DNA
   <213> Artificial Sequence
<220>
   <223> Oligonucleotide
<400> 10
   agggaggagg aaacagcgtg agtcc 25
<210> 11
   <211> 25
   <212> DNA
   <213> Artificial Sequence
<220>
   <223> Oligonucleotide
<400> 11
   atgggaaagg aaaagactca tatca 25
<210> 12
   <211> 25
   <212> DNA
   <213> Artificial Sequence
<220>
   <223> Oligonucleotide
<400> 12
   agcagcaaca atcaggacag cacag 25
<210> 13
   <211> 25
   <212> DNA
   <213> Artificial Sequence
<220>
   <223> Oligonucleotide
<400> 13
   atcaagaatt cgcacgagac catta 25
<210> 14
   <211> 67
   <212> DNA
   <213> Artificial Sequence
<220>
   <223> Oligonucleotide
<400> 14
   atcgttgaga ctcgtaccag cagagtcacg agagagacta cacggtactg gttttttttt 60
   tttttvn 67
<210> 15
   <211> 29
   <212> DNA
   <213> Artificial Sequence
<220>
   <223> Oligonucleotide
<400> 15
   ccagcagagt cacgagagag actacacgg 29
<210> 16
   <211> 25
   <212> DNA
   <213> Artificial Sequence
   <220>
   <223> Oligonucleotide
<400> 16
   cacgagagag actacacggt actgg 25
<210> 17
   <211> 526
   <212> DNA
   <213> Homo sapiens
<220>
   <221> misc_feature
   <222> complement(261..376)
   <223> blastn
<221> misc_feature
   <222> complement (380..486)
   <223> blastn
<221> misc_feature
   <222> complement (110..145)
   <223> blastn
<221> misc_feature
   <222> complement (196 .. 229)
   <223> blastn
<221> sig_peptide
   <222> 90..140
   <223> Von Heijne matrix
<400> 17
   aatatrarac agctacaata ttccagggcc artcacttgc catttctcat aacagcgtca    60
   gagagaaaga actgactgar acgtttgag atg aag aaa gtt ctc ctc ctg atc     113
                                   Met Lys Lys Val Leu Leu Leu Ile
                                           -15                 -10
   aca gcc atc ttg gca gtg gct gtw ggt ttc cca gtc tct caa gac cag     161
   Thr Ala Ile Leu Ala Val Ala Val Gly Phe Pro Val Ser Gln Asp Gln
                   -5                  1               5
   gaa cga gaa aaa aga agt atc agt gac agc gat gaa tta gct tca ggr     209
   Glu Arg Glu Lys Arg Ser Ile Ser Asp Ser Asp Glu Leu Ala Ser Gly
           10                  15                  20
   wtt ttt gtg ttc cct tac cca tat cca ttt cgc cca ctt cca cca att     257
   Xaa Phe Val Phe Pro Tyr Pro Tyr Pro Phe Arg Pro Leu Pro Pro Ile
       25                  30                  35
   cca ttt cca aga ttt cca tgg ttt aga cgt aan ttt cct att cca ata     305
   Pro Phe Pro Arg Phe Pro Trp Phe Arg Arg Xaa Phe Pro Ile Pro Ile
   40                  45                  50                  55
   cct gaa tct gcc cct aca act ccc ctt cct agc gaa aag taaacaaraa      354
   Pro Glu Ser Ala Pro Thr Thr Pro Leu Pro Ser Glu Lys
                   60                  65
   ggaaaagtca crataaacct ggtcacctga aattgaaatt gagccacttc cttgaaraat   414
   caaaattcct gttaataaaa raaaaacaaa tgtaattgaa atagcacaca gcattctcta   474
   gtcaatatct ttagtgatct tctttaataa acatgaaagc aaaaaaaaaa aa           526
<210> 18
   <211> 17
   <212> PRT
   <213> Homo sapiens
<220>
   <221> SIGNAL
   <222> 1..17
   <223> Von Heijne matrix
   score 8.2
   seq LLLITAILAVAVG/FP
<400> 18
   Met Lys Lys Val Leu Leu Leu Ile Thr Ala Ile Leu Ala Val Ala Val
   1               5                   10                  15
   Gly
<210> 19
   <211> 822
   <212> DNA
   <213> Homo sapiens
<220>
   <221> misc_feature
   <222> 260..464
   <223> blastn
<221> misc_feature
   <222> 118..184.
   <223> blastn
<221> miac_feature
   <222> 56..113
   <223> blastn
<221> misc_feature
   <222> 454..485
   <223> blastn
<221> misc_feature
   <222> 118..545
   <223> blastn
<221> misc_feature
   <222> 65..369
   <223> blastn
<221> misc_feature
   <222> 61..399
   <223> blastn
<221> misc_feature
   <222> 408..458
   <223> blastn
<221> misc_feature
   <222> 60..399
   <223> blastn
<221> misc_feature
   <222> 393..432
   <223> blastn
<221> aig_peptide
   <222> 346..408
   <223> Von Heijne matrix
<400> 19
   actcctttta gcataggggc ttcggcgcca gcggccagcg ctagtcggtc tggtaagtgc     60
   ctgatgccga gttccgtctc tcgcgtcttt tcctggtccc aggcaaagcg gasgnagatc    120
   ctcaaacggc ctagtgcttc gcgcttccgg agaaaatcag cggtctaatt aattcctctg    180
   gtttgttgaa gcagttacca agaatcttca accctttccc acaaaagcta attgagtaca    240
   cgttcctgtt gagtacacgt tcctgttgat ttacaaaagg tgcaggtatg agcaggtctg    300
   aagactaaca ttttgtgaag ttgtaaaaca gaaaacctgt tagaa atg tgg tgg ttt    357
                                                     Met Trp Trp Phe
                                                         -20
   cag caa ggc ctc agt ttc ctt cct tca gcc ctt gta att tgg aca tct      405
   Gln Gln Gly Leu Ser Phe Leu Pro Ser Ala Leu Val Ile Trp Thr Ser
       -15                 -10                 -5
   gct gct ttc ata ttt tca tac att act gca gta aca ctc cac cat ata      453
   Ala Ala Phe Ile Phe Ser Tyr Ile Thr Ala Val Thr Leu His His Ile
       1               5                   10                  15
   gac ccg gct tta cct tat atc agt gac act ggt aca gta gct cca raa      501
   Asp Pro Ala Leu Pro Tyr Ile Ser Asp Thr Gly Thr Val Ala Pro Xaa
                   20                  25                  30
   aaa tgc tta ttt ggg gca atg cta aat att gcg gca gtt tta tgt caa      549
   Lys Cys Leu Phe Gly Ala Met Leu Asn Ile Ala Ala Val Leu Cys Gln
               35                  40                  45
   aaa tagaaatcag gaarataatt caacttaaag aakttcattt catgaccaaa           602
   Lys
   ctcttcaraa acatgtcttt acaagcatat ctcttgtatt gctttctaca ctgttgaatt    662
   gtctggcaat atttctgcag tggaaaattt gatttarmta gttcttgact gataaatatg    722
   gtaaggtggg cttttccccc tgtgtaattg gctactatgt cttactgagc caagttgtaw    782
   tttgaaataa aatgatatga gagtgacaca aaaaaaaaaa                          822
<210> 20
   <211> 21
   <212> PRT
   <213> Homo sapiens
<220>
   <221> SIGNAL
   <222> 1..21
   <223> Von Heijne matrix
   score 5.5
   seq SFLPSALVIWTSA/AF
<400> 20
   Met Trp Trp Phe Gln Gln Gly Leu Ser Phe Leu Pro Ser Ala Leu Val
   1               5                   10                  15
   Ile Trp Thr Ser Ala
               20
<210> 21
   <211> 405
   <212> DNA
   <213> Homo sapiens
<220>
   <221> misc_feature
   <222> complement (103..398)
   <223> blastn
<221> sig_peptide
   <222> 185..295
   <223> Von Heijne matrix
<400> 21
   atcaccttct tctccatcct tstctgggcc agtccccarc ccagtccctc tcctgacctg      60
   cccagcccaa gtcagccttc agcacgcgct tttctgcaca cagatattcc aggcctacct     120
   ggcattccag gacctccgma atgatgctcc agtcccttac aagcgcttcc tggatgaggg     180
   tggc atg gtg ctg acc acc ctc ccc ttg ccc tct gcc aac agc cct gtg      229
        Met Val Leu Thr Thr Leu Pro Leu Pro Ser Ala Asn Ser Pro Val
                -35                 -30                 -25
   aac atg ccc acc act ggc ccc aac agc ctg agt tat gct agc tct gcc       277
   Asn Met Pro Thr Thr Gly Pro Asn Ser Leu Ser Tyr Ala Ser Ser Ala
           -20                 -15                 -10
   ctg tcc ccc tgt ctg acc gct cca aak tcc ccc cgg ctt gct atg atg       325
   Leu Ser Pro Cys Leu Thr Ala Pro Xaa Ser Pro Arg Leu Ala Met Met
       -5                  1               5                   10
   cct gac aac taaatatcct tatccaaatc aataaarwra raatcctccc               374
   Pro Asp Asn
   tccaraaggg tttctaaaaa caaaaaaaaa a                                    405
<210> 22
   <211> 37
   <212> PRT
   <213> Homo sapiens
<220>
   <221> SIGNAL
   <222> 1..37
   <223> Von Heijne matrix
   score 5.9
   seq LSYASSALSPCLT/AP
<400> 22
   Met Val Leu Thr Thr Leu Pro Leu Pro Ser Ala Asn Ser Pro Val Asn
   1               5                   10                  15
   Met Pro Thr Thr Gly Pro Asn Ser Leu Ser Tyr Ala Ser Ser Ala Leu
               20                  25                  30
   Ser Pro Cys Leu Thr
           35
<210> 23
   <211> 496
   <212> DNA
   <213> Homo sapiens
<220>
   <221> miac_feature
   <222> 149..331
   <223> blastn
<221> misc_feature
   <222> 328..485
   <223> blastn
<221> misc_feature
   <222> complement (182..496)
   <223> blastn
   <221> sig_peptide
   <222> 196..240
   <223> Von Heijne matrix
<400> 23
   aaaaaattgg tcccagtttt caccctgccg cagggctggc tggggagggc agcggtttag      60
   attagccgtg gcctaggccg tttaacgggg tgacacgagc ntgcagggcc gagtccaagg     120
   cccggagata ggaccaaccg tcaggaatgc gaggaatgtt tttcttcgga ctctatcgag     180
   gcacacagac agacc atg ggg att ctg tct aca gtg aca gcc tta aca ttt      231
                    Met Gly Ile Leu Ser Thr Val Thr Ala Leu Thr Phe
                    -15                 -10                 -5
   gcc ara gcc ctg gac ggc tgc aga aat ggc att gcc cac cct gca agt      279
   Ala Xaa Ala Leu Asp Gly Cys Arg Asn Gly Ile Ala His Pro Ala Ser
               1               5                   10
   gag aag cac aga ctc gag aaa tgt agg gaa ctc gag asc asc cac tcg      327
   Glu Lys His Arg Leu Glu Lys Cys Arg Glu Leu Glu Xaa Xaa His Ser
       15                  20                  25
   gcc cca gga tca acc cas cac cga aga aaa aca acc aga aga aat tat      375
   Ala Pro Gly Ser Thr Xaa His Arg Arg Lys Thr Thr Arg Arg Asn Tyr
   30                  35                  40                  45
   tct tca gcc tgaaatgaak ccgggatcaa atggttgctg atcaragccc               424
   Ser Ser Ala
   atatttaaat tggaaaagtc aaattgasca ttattaaata aagcttgttt aatatgtctc     484
   aaacaaaaaa aa                                                         496
<210> 24
   <211> 15
   <212> PRT
   <213> Homo sapiens
<220>
   <221> SIGNAL
   <222> 1..15
   <223> Von Heijne matrix
   score 5.5
   seq ILSTVTALTFAXA/LD
<400> 24
   Met Gly Ile Leu Ser Thr Val Thr Ala Leu Thr Phe Ala Xaa Ala
   1               5                   10                  15
<210> 25
   <211> 623
   <212> DNA
   <213> Homo sapiens
<220>
   <221> sig peptide
   <222> 49..96
   <223> Von Heijne matrix
<400> 25
   aaagatccct gcagcccggc aggagagaag gctgagcctt ctggcgtc atg gag agg       57
                                                        Met Glu Arg
                                                            -15
   ctc gtc cta acc ctg tgc acc ctc ccg ctg gct gtg gcg tct gct ggc       105
   Leu Val Leu Thr Leu Cys Thr Leu Pro Leu Ala Val Ala Ser Ala Gly
               -10                 -5                  1
   tgc gcc acg acg cca gct cgc aac ctg agc tgc tac cag tgc ttc aag       153
   Cys Ala Thr Thr Pro Ala Arg Asn Leu Ser Cys Tyr Gln Cys Phe Lys
       5                   10                  15
   gtc agc agc tgg acg gag tgc ccg ccc acc tgg tgc agc ccg ctg gac       201
   Val Ser Ser Trp Thr Glu Cys Pro Pro Thr Trp Cys Ser Pro Leu Asp
   20                  25                  30                  35
   caa gtc tgc atc tcc aac gag gtg gtc gtc tct ttt aaa tgg agt gta       249
   Gln Val Cys Ile Ser Asn Glu Val Val Val Ser Phe Lys Trp Ser Val
                   40                  45                  50
   cgc gtc ctg ctc agc aaa cgc tgt gct ccc aga tgt ccc aac gac aac       297
   Arg Val Leu Leu Ser Lys Arg Cys Ala Pro Arg Cys Pro Asn Asp Asn
               55                  60                  65
   atg aak ttc gaa tgg tcg ccg gcc ccc atg gtg caa ggc gtg atc acc       345
   Met Xaa Phe Glu Trp Ser Pro Ala Pro Met Val Gln Gly Val Ile Thr
           70                  75                  80
   agg cgc tgc tgt tcc tgg gct ctc tgc aac agg gca ctg acc cca cag       393
   Arg Arg Cys Cys Ser Trp Ala Leu Cys Asn Arg Ala Leu Thr Pro Gln
       85                  90                  95
   gag ggg cgc tgg gcc ctg cra ggg ggg ctc ctg ctc cag gac cct tcg       441
   Glu Gly Arg Trp Ala Leu Xaa Gly Gly Leu Leu Leu Gln Asp Pro Ser
   100                 105                 110                 115
   agg ggc ara aaa acc tgg gtg cgg cca cag ctg ggg ctc cca ctc tgc       489
   Arg Gly Xaa Lys Thr Trp Val Arg Pro Gln Leu Gly Leu Pro Leu Cys
                   120                 125                 130
   ctt ccc awt tcc aac ccc ctc tgc cca rgg gaa acc cag gaa gga           534
   Leu Pro Xaa Ser Asn Pro Leu Cys Pro Xaa Glu Thr Gln Glu Gly
               135                 140                 145
   taacactgtg ggtgccccca cctgtgcatt gggaccacra cttcaccctc ttggaracaa     594
   taaactctca tgcccccaaa aaaaaaaaa                                        623
<210> 26
   <211> 16
   <212> PRT
   <213> Homo sapiens
<220>
   <221> SIGNAL
   <222> 1..16
   <223> Von Heijne matrix
   score 10.1
   seq LVLTLCTLPLAVA/SA
<400> 26
   Met Glu Arg Leu Val Leu Thr Leu Cys Thr Leu Pro Leu Ala Val Ala
   1               5                   10                  15
<210> 27
   <211> 848
   <212> DNA
   <213> Homo sapiens
<220>
   <221> sig_peptide
   <222> 32..73
   <223> Von Heijne matrix
<400> 27
   aactttgcct tgtgttttcc accctgaaag a atg ttg tgg ctg ctc ttt ttt        52
                                      Met Leu Trp Leu Leu Phe Phe
                                                      -10
   ctg gtg act gcc att cat gct gaa ctc tgt caa cca ggt gca gaa aat      100
   Leu Val Thr Ala Ile His Ala Glu Leu Cys Gln Pro Gly Ala Glu Asn
           -5                  1                5
   gct ttt aaa gtg aga ctt agt atc aga aca gct ctg gga gat aaa gca      148
   Ala Phe Lys Val Arg Leu Ser Ile Arg Thr Ala Leu Gly Asp Lys Ala
   10                  15                  20                  25
   tat gcc tgg gat acc aat gaa gaa tac ctc ttc aaa gcg atg gta gct      196
   Tyr Ala Trp Asp Thr Asn Glu Glu Tyr Leu Phe Lys Ala Met Val Ala
                   30                  35                  40
   ttc tcc atg aga aaa gtt ccc aac aga gaa gca aca gaa att tcc cat      244
   Phe Ser Met Arg Lys Val Pro Asn Arg Glu Ala Thr Glu Ile Ser His
               45                  50                  55
   gtc cta ctt tgc aat gta acc cag agg gta tca ttc tgg ttt gtg gtt      292
   Val Leu Leu Cys Asn Val Thr Gln Arg Val Ser Phe Trp Phe Val Val
           60                  65                  70
   aca gac cct tca aaa aat cac acc ctt cct gct gtt gag gtg caa tca      340
   Thr Asp Pro Ser Lys Asn His Thr Leu Pro Ala Val Glu Val Gln Ser
       75                  80                  85
   gcc ata aga atg aac aag aac cgg atc aac aat gcc ttc ttt cta aat      388
   Ala Ile Arg Met Asn Lys Asn Arg Ile Asn Asn Ala Phe Phe Leu Asn
   90                  95                  100                 105
   gac caa act ctg gaa ttt tta aaa atc cct tcc aca ctt gca cca ccc      436
   Asp Gln Thr Leu Glu Phe Leu Lys Ile Pro Ser Thr Leu Ala Pro Pro
                       110                      115                      120
   atg gac cca tct gtg ccc atc tgg att att ata ttt ggt gtg ata ttt         484
   Met Asp Pro Ser Val Pro Ile Trp Ile Ile Ile Phe Gly Val Ile Phe
                   125                      130                      135
   tgc atc atc ata gtt gca att gca cta ctg att tta tca ggg atc tgg         532
   Cys Ile Ile Ile Val Ala Ile Ala Leu Leu Ile Leu Ser Gly Ile Trp
               140                      145                      150
   caa cgt ada ara aag aac aaa gaa cca tct gaa gtg gat gac gct gaa         580
   Gln Arg Xaa Xaa Lys Asn Lys Glu Pro Ser Glu Val Asp Asp Ala Glu
           155                      160                      165
   rat aak tgt gaa aac atg atc aca att gaa aat ggc atc ccc tct gat         628
   Xaa Xaa Cys Glu Asn Met Ile Thr Ile Glu Asn Gly Ile Pro Ser Asp
   170                      175                      180                      185
   ccc ctg gac atg aag gga ggg cat att aat gat gcc ttc atg aca gag         676
   Pro Leu Asp Met Lys Gly Gly His Ile Asn Asp Ala Phe Met Thr Glu
                       190                      195                      200
   gat gag agg ctc acc cct ctc tgaagggctg ttgttctgct tcctcaaraa            727
   Asp Glu Arg Leu Thr Pro Leu
                   205
   attaaacatt tgtttctgtg tgactgctga gcatcctgaa ataccaagag cagatcatat      787
   wttttgtttc accattcttc ttttgtaata aattttgaat gtgcttgaaa aaaaaaaaaa      847
   c                                                                       848
<210> 28
   <211> 14
   <212> PRT
   <213> Homo sapiens
<220>
   <221> SIGNAL
   <222> 1..14
   <223> Von Heijne matrix
   score 10.7
   seq LWLLFFLVTAIHA/BL
<400> 28
   Met Leu Trp Leu Leu Phe Phe Leu Val Thr Ala Ile His Ala
   1               5                   10
<210> 29
   <211> 25
   <212> DNA
   <213> Artificial sequence
<220>
   <223> Oligonucleotide
<400> 29
   gggaagatgg agatagtatt gcctg 25
<210> 30
   <211> 26
   <212> DNA
   <213> Artificial Sequence
<220>
   <223> Olignucleotide
<400> 30
   ctgccatgta catgatagag agattc 26
<210> 31
   <211> 546
   <212> DNA
   <213> Homo sapiens
<220>
   <221> promoter
   <222> 1..517
<221> transcription start site
   <222> 518
<221> protein bind
   <222> 17..25
   <223> matinspector prediction
   name CMYB_01
   score 0.983
   sequence tgtcagttg
<221> protein bind
   <222> complement(18..27)
   <223> matinspector prediction
   name MYOD_Q6
   score 0.961
   sequence cccaactgac
<221> protein_bind
   <222> complement (75..85)
   <223> matinspector prediction
   name S8_01
   score 0.960
   sequence aatagaattag
<221> protein_bind
   <222> 94..104
   <223> matinspector prediction
   name S8_01
   score 0.966
   sequence aactaaattag
<221> protein_bind
   <222> complement(129..139)
   <223> matinspector prediction
   name DELTAEF1_01
   score 0.960
   sequence gcacacctcag
<221> protein_bind
   <222> complement (155..165)
   <223> matinspector prediction
   name GATA_C
   score 0.964
   sequence agataaatcca
<221> protein_bind
   <222> 170..178
   <223> matinspector prediction
   name CMYB_01
   score 0.958
   sequence cttcagttg
<221> protein bind
   <222> 176..189
   <223> matinspector prediction
   name GATA1_02
   score 0.959
   sequence ttgtagataggaca
<221> protein_bind
   <222> 180..190
   <223> matinspector prediction
   name GATA_C
   score 0.953
   sequence agataggacat
<221> protein bind
   <222> 284..299
   <223> matinspector prediction
   name TAL1ALPHAE47 01 score 0.973
   sequence cataacagatggtaag
<221> protein_bind
   <222> 284..299
   <223> matinspector prediction
   name TAL1BETAE47_01
   score 0.983
   sequence cataacagatggtaag
<221> protein_bind
   <222> 284..299
   <223> matinspector prediction
   name TAL1BETAITF2 01 score 0.978
   sequence cataacagatggtaag
<221> protein_bind
   <222> complement (287..296)
   <223> matinspector prediction
   name MYOD-Q6
   score 0.954
   sequence accatctgtt
<221> protein bind
   <222> complement (302..314)
   <223> matinspector prediction
   name GATA1_04
   score 0.953
   sequence tcaagataaagta
<221> protein_bind
   <222> 393..405
   <223> matinspector prediction
   name IK1_01
   score 0.963
   sequence agttgggaattcc
<221> protein_bind
   <222> 393..404
   <223> matinspector prediction
   name IK2_01
   score 0.985
   sequence agttgggaattc
<221> protein_bind
   <222> 396..405
   <223> matinspector prediction
   name CREL_01
   score 0.962
   sequence tgggaattcc
<221> protein_bind
   <222> 423..436
   <223> matinspector prediction
   name GATA1_02
   score 0.950
   sequence tcagtgatatggca
<221> protein_bind
   <222> complement (478..489)
   <223> matinspector prediction
   name SRY_02
   score 0.951
   sequence caaaacaaaaca
<221> protein_bind
   <222> 486..493
   <223> matinspector prediction
   name E2F_02
   score 0.957
   sequence tttagcgc
<221> protein_bind
   <222> complement (514..521)
   <223> matinspector prediction
   name MZF1_01
   score 0.975
   sequence tgagggga
<400> 31
   tgagtgcagt gttacatgtc agttgggtta agtttgttaa tgtcattcaa atcttctatg 60
   tcttgatttg cctgctaatt ctattatttc tggaactaaa ttagtttgat ggttctatta 120
   gttattgact gaggtgtgct aatctcccat tatgtggatt tatctatttc ttcagttgta 180
   gataggacat tgatagatac ataagtacca ggacaaaagc agggagatct tttttccaaa 240
   atcaggagaa aaaaatgaca tctggaaaac ctatagggaa aggcataaca gatggtaagg 300
   atactttatc ttgagtagga gagccttcct gtggcaacgt ggagaaggga agaggtcgta 360
   gaattgagga gtcagctcag ttagaagcag ggagttggga attccgttca tgtgatttag 420
   catcagtgat atggcaaatg tgggactaag ggtagtgatc agagggttaa aattgtgtgt 480
   tttgttttag cgctgctggg gcatcgcctt gggtcccctc aaacagattc ccatgaatct 540
   cttcat 546
<210> 32
   <211> 23
   <212> DNA
   <213> Artificial Sequence
<220>
   <223> Oligonucleotide
<400> 32
   gtaccaggga ctgtgaccat tgc 23
<210> 33
   <211> 24
   <212> DNA
   <213> Artificial Sequence
<220>
   <223> Oligonucleotide
<400> 33
   ctgtgaccat tgctcccaag agag 24
<210> 34
   <211> 861
   <212> DNA
   <213> Homo sapiens
<220>
   <221> promoter
   <222> 1..806
<221> transcription start site
   <222> 807
<221> protein_bind
   <222> complement (60..70)
   <223> matinspector prediction
   name NFY_Q6
   score 0.956
   sequence ggaccaatcat
<221> protein_bind
   <222> 70..77
   <223> matinspector prediction
   name MZF1_01
   score 0.962
   sequence cctgggga
<221> protein_bind
   <222> 124..132
   <223> matinspector prediction
   name CMYB_01
   score 0.994
   sequence tgaccgttg
<221> protein_bind
   <222> complement (126..134)
   <223> matinspector prediction
   name VMYB_02
   score 0.985
   sequence tccaacggt
<221> protein_bind
   <222> 135..143
   <223> matinspector prediction
   name STAT_01
   score 0.968
   sequence ttcctggaa
<221> protein_bind
   <222> complement (135..143)
   <223> matinspector prediction
   name STAT_01
   score 0.951
   sequence ttccaggaa
<221> protein_bind
   <222> complement (252..259)
   <223> matinspector prediction
   name MZF1_01
   score 0.956
   sequence ttggggga
<221> protein_bind
   <222> 357..368
   <223> matinspector prediction
   name IK2_01
   score 0.965
   sequence gaatgggatttc
<221> protein bind
   <222> 384..391
   <223> matinspector prediction
   name MZF1_01
   score 0.986
   sequence agagggga
<221> protein_bind
   <222> complement (410..421)
   <223> matinspector prediction
   name SRY_02
   score 0.955
   sequence gaaaacaaaaca
<221> protein_bind
   <222> 592..599
   <223> matinspector prediction
   name MZF1_01
   score 0.960
   sequence gaagggga
<221> protein bind
   <222> 618..627
   <223> matinspector prediction
   name MYOD_Q6
   score 0.981
   sequence agcatctgcc
<221> protein_bind
   <222> 632..642
   <223> matinspector prediction
   name DELTAEF1 01
   score 0.958
   sequence tcccaccttcc
<221> protein_bind
   <222> complement(813..823)
   <223> matinspector prediction
   name S8 01
   score 0.992
   sequence gaggcaattat
<221> protein_bind
   <222> complement(824..831)
   <223> matinspector prediction
   name MZF1_01
   score 0.986
   sequence agagggga
<400> 34
   tactataggg cacgcgtggt cgacggccgg gctgttctgg agcagagggc atgtcagtaa    60
   tgattggtcc ctggggaagg tctggctggc tccagcacag tgaggcattt aggtatctct   120
   cggtgaccgt tggattcctg gaagcagtag ctgttctgtt tggatctggt agggacaggg   180
   ctcagagggc taggcacgag ggaaggtcag aggagaaggs aggsarggcc cagtgagarg   240
   ggagcatgcc ttcccccaac cctggcttsc ycttggymam agggcgktty tgggmacttr   300
   aaytcagggc ccaascagaa scacaggccc aktcntggct smaagcacaa tagcctgaat   360
   gggatttcag gttagncagg gtgagagggg aggctctctg gcttagtttt gttttgtttt   420
   ccaaatcaag gtaacttgct cccttctgct acgggccttg gtcttggctt gtcctcaccc   480
   agtcggaact ccctaccact ttcaggagag tggttttagg cccgtggggc tgttctgttc   540
   caagcagtgt gagaacatgg ctggtagagg ctctagctgt gtgcggggcc tgaaggggag   600
   tgggttctcg cccaaagagc atctgcccat ttcccacctt cccttctccc accagaagct   660
   tgcctgagct gtttggacaa aaatccaaac cccacttggc tactctggcc tggcttcagc   720
   ttggaaccca atacctaggc ttacaggcca tcctgagcca ggggcctctg gaaattctct   780
   tcctgatggt cctttaggtt tgggcacaaa atataattgc ctctcccctc tcccattttc   840
   tctcttggga gcaatggtca c                                             861
<210> 35
   <211> 20
   <212> DNA
   <213> Artificial Sequence
<220>
   <223> Oligonucleotide
<400> 35
   ctgggatgga aggcacggta 20
<210> 36
   <211> 20
   <212> DNA
   <213> Artificial Sequence
<220>
   <223> Oligonucleotide
<400> 36
   gagaccacac agctagacaa 20
<210> 37
   <211> 555
   <212> DNA
   <213> Homo sapiens
<220>
   <221> promoter
   <222> 1..500
<221> transcription start site
   <222> 501
<221> protein_bind
   <222> 191..206
   <223> matinspector prediction
   name ARNT_01
   score 0.964
   sequence ggactcacgtgctgct
<221> protein_bind
   <222> 193..204
   <223> matinspector prediction
   name NMYC 01
   score 0.965
   sequence actcacgtgctg
<221> protein_bind
   <222> 193..204
   <223> matinspector prediction
   name USF_01
   score 0.985
   sequence actcacgtgctg
<221> protein_bind
   <222> complement(193..204)
   <223> matinspector prediction
   name USF_01
   score 0.985
   sequence cagcacgtgagt
<221> protein bind
   <222> complement (193..204)
   <223> matinspector prediction
   name NMYC_01
   score 0.956
   sequence cagcacgtgagt
<221> protein bind
   <222> complement (193..204)
   <223> matinspector prediction
   name MYCMAX_02
   score 0.972
   sequence cagcacgtgagt
<221> protein_bind
   <222> 195..202
   <223> matinspector prediction
   name USP_C
   score 0.997
   sequence tcacgtgc
<221> protein_bind
   <222> complement(195..202)
   <223> matinspector prediction
   name USF_C
   score 0.991
   sequence gcacgtga
<221> protein bind
   <222> complement (210..217)
   <223> matinspector prediction
   name MZF1_01
   score 0.968
   sequence catgggga
<221> protein_bind
   <222> 397..410
   <223> matinspector prediction
   name ELK1_02
   score 0.963
   sequence ctctccggaagcct
<221> protein_bind
   <222> 400..409
   <223> matinspector prediction
   name CETS1P54 01
   score 0.974
   sequence tccggaagcc
<221> protein_bind
   <222> complement (460..470)
   <223> matinspector prediction
   name AP1_Q4
   score 0.963
   sequence agtgactgaac
<221> protein_bind
   <222> complement (460..470)
   <223> matinspector prediction
   name AP1FJ_Q2
   score 0.961
   sequence agtgactgaac
<221> protein_bind
   <222> 547..555
   <223> matinspector prediction
   name PADS_C
   score 1.000
   sequence tgtggtctc
<400> 37
   ctatagggca cgcktggtcg acggcccggg ctggtctggt ctgtkgtgga gtcgggttga      60
   aggacagcat ttgtkacatc tggtctactg caccttccct ctgccgtgca cttggccttt     120
   kawaagctca gcaccggtgc ccatcacagg gccggcagca cacacatccc attactcaga     180
   aggaactgac ggactcacgt gctgctccgt ccccatgagc tcagtggacc tgtctatgta     240
   gagcagtcag acagtgcctg ggatagagtg agagttcagc cagtaaatcc aagtgattgt     300
   cattcctgtc tgcattagta actcccaacc tagatgtgaa aacttagttc tttctcatag     360
   gttgctctgc ccatggtccc actgcagacc caggcactct ccggaagcct ggaaatcacc     420
   cgtgtcttct gcctgctccc gctcacatcc cacacttgtg ttcagtcact gagttacaga     480
   ttttgcctcc tcaatttctc ttgtcttagt cccatcctct gttcccctgg ccagtttgtc     540
   tagctgtgtg gtctc                                                      555
<210> 38
   <211> 19
   <212> DNA
   <213> Artificial Sequence
<220>
   <223> oligonucleotide
<400> 38
   ggccatacac ttgagtgac 19
<210> 39
   <211> 19
   <212> DNA
   <213> Artificial Sequence
<220>
   <223> oligonucleotide
<400> 39
   atatagacaa acgcacacc 19
<210> 45
   <211> 2036
   <212> DNA
   <213> Homo sapiens
<220>
   <221> CDS
   <222> 276..1040
<221> sig_peptide
   <222> 276..485
   <223> Von Heijne matrix
   score 3.9
   seq SVIGVMLAPFTAG/LS
<221> polyA_site
   <222> 2024..2036
<400> 45
   gatcctgggt gcagctcatc acaagcgtcg gggtgcagca aaaccatcca ggctggacag     60
   tggctggaca gttccaagaa aagaaacgct tcaccgaaga agtcattgaa tacttccaga    120
   agaaagttag cccagtgcat ctgaaaatcc tgctgactag cgatgaagcc tggaagagat    180
   tcgtgcgtgt ggctggattg cccagggaag aagcagatgc tctctatgaa gctctgaaga    240
   atcttacacc atatgtggct attgaggaca aagac atg cag caa aaa gaa cag        293
                                          Met Gln Gln Lys Glu Gln
                                          -70                 -65
   cag ttt agg gag tgg ttt ttg aaa gag ttt cct caa atc aga tgg aag      341
   Gln Phe Arg Glu Trp Phe Leu Lys Glu Phe Pro Gln Ile Arg Trp Lys
                   -60                 -55                 -50
   att cag gag tcc ata gaa agg ctt cgt gtc att gca aat gag att gaa      389
   Ile Gln Glu Ser Ile Glu Arg Leu Arg Val Ile Ala Asn Glu Ile Glu
               -45                 -40                 -35
   aag gtc cac aga ggc tgc gtc atc gcc aat gtg gtg tct ggc tcc act       437
   Lys Val His Arg Gly Cys Val Ile Ala Asn Val Val Ser Gly Ser Thr
           -30                 -25                 -20
   ggc atc ctg tct gtc att ggc gtt atg ttg gca cca ttt aca gca ggg       485
   Gly Ile Leu Ser Val Ile Gly Val Met Leu Ala Pro Phe Thr Ala Gly
       -15                 -10                  -5
   ctg agc ctg agc att act gca gct ggg gta ggg ctg gga ata gca tct       533
   Leu Ser Leu Ser Ile Thr Ala Ala Gly Val Gly Leu Gly Ile Ala Ser
   1               5                   10                  15
   gcc acg gct ggg atc gcc tcc agc atc gtg gag aac aca tac aca agg       581
   Ala Thr Ala Gly Ile Ala Ser Ser Ile Val Glu Asn Thr Tyr Thr Arg
               20                  25                  30
   tca gca gaa ctc aca gcc agc agg ctg act gca acc agc act gac caa       629
   Ser Ala Glu Leu Thr Ala Ser Arg Leu Thr Ala Thr Ser Thr Asp Gln
           35                  40                  45
   ttg gag gca tta agg gac att ctg cat gac atc aca ccc aat gtg ctt       677
   Leu Glu Ala Leu Arg Asp Ile Leu His Asp Ile Thr Pro Asn Val Leu
       50                  55                  60
   tcc ttt gca ctt gat ttt gac gaa gcc aca aaa atg att gcg aat gat       725
   Ser Phe Ala Leu Asp Phe Asp Glu Ala Thr Lys Met Ile Ala Asn Asp
   65                  70                  75                  80
   gtc cat aca ctc agg aga tct aaa gcc act gtt gga cgc cct ttg att       773
   Val His Thr Leu Arg Arg Ser Lys Ala Thr Val Gly Arg Pro Leu Ile
                   85                  90                  95
   gct tgg cga tat gta cct ata aat gtt gtt gag aca ctg aga aca cgt       821
   Ala Trp Arg Tyr Val Pro Ile Asn Val Val Glu Thr Leu Arg Thr Arg
               100                 105                 110
   ggg gcc ccc acc cgg ata gtg aga aaa gta gcc cgg aac ctg ggc aag       869
   Gly Ala Pro Thr Arg Ile Val Arg Lys Val Ala Arg Asn Leu Gly Lys
           115                 120                 125
   gcc act tca ggt gtc ctc gtt gtg ctg gat gta gtc aac ctt gtg caa       917
   Ala Thr Ser Gly Val Leu Val Val Leu Asp Val Val Asn Leu Val Gln
       130                 135                 140
   gac tca ctg gac ttg cac aag ggg gaa aaa tcc gag tct gct gag ttg       965
   Asp Ser Leu Asp Leu His Lys Gly Glu Lys Ser Glu Ser Ala Glu Leu
   145                 150                 155                 160
   ctg agg cag tgg gct cag gag ctg gag gag aat ctc aat gag ctc acc      1013
   Leu Arg Gln Trp Ala Gln Glu Leu Glu Glu Asn Leu Asn Glu Leu Thr
                   165                 170                 175
   cat atc cat cag agt cta aaa gca ggc taggcccaat tgttgcggga            1060
   His Ile His Gln Ser Leu Lys Ala Gly
               180                 185
   agtcagggac cccaaacgga gggactggct gaagccatgg cagaagaacg tggattgtga   1120
   agatttcatg gacatttatt agttccccaa attaatactt ttataatttc ctatgcctgt   1180
   ctttaccgca atctctaaac acaaattgtg aagatttcat ggacacttat cacttcccca   1240
   atcaataccc ttgtgatttc ttatgcctgt ctttacttta atctcctaat cctgtcagct   1300
   gaggagggtg tatgtcacct caggaccatg tgataattgc gttaactgca caaattgtag   1360
   agcatgtgtg tttgaacaat atgaaatctg ggcaccttga aaaaagaaca ggataacagc   1420
   aatcgttcag gggataagag agataacctt aaactctgac caacagtgag ccgggtggag    1480
   cagagtcata tttcttttct ttcaaaagca aatgggagaa atatcgctga attctttttc    1540
   tcagcaagga acatccctga gaaagagaat gcacccctga gggtgggtct ataaatggcc   1600
   tccttgggtg tggccatctt ctatggtcga gactgtaggg atgaaataaa ccccagtctc   1660
   ccatagtgct cccaggctta ttaggaagag gaaattcccg cctaataaat tttggtcaga   1720
   ccggttgctc tcaaaaccct gtctcctgat aagatgttat caatgacaat ggtgcctgaa  1780
   acctcattag caattttaat ttctccccgg tcctgtggtc ctgtgatctc accctgcctc   1840
   cacttgcctt gtgatattct attaccttgt gaagtaggtg atctttgtga cccacaccct  1900
   attcatacac tccctcccct tttggaagtc cctaataaaa acttgctggt tttgcagctt  1960
   gtgaggcatc acggaaccta ctgatgtgtg atgtctcccc tggacaccta gctttaaaat   2020
   ttcaaaaaaa aaaaaa                                                   2036
<210> 146
   <211> 255
   <212> PRT
   <213> Homo sapiens
<220>
   <221> SIGNAL
   <222> -70..-1
<400> 146
   Met Gln Gln Lys Glu Gln Gln Phe Arg Glu Trp Phe Leu Lys Glu Phe
   -70                 -65                 -60                 -55
   Pro Gln Ile Arg Trp Lys Ile Gln Glu Ser Ile Glu Arg Leu Arg Val
                   -50                 -45                 -40
   Ile Ala Asn Glu Ile Glu Lys Val His Arg Gly Cys Val Ile Ala Asn
               -35                 -30                 -25
   Val Val Ser Gly Ser Thr Gly Ile Leu Ser Val Ile Gly Val Met Leu
           -20                 -15                 -10
   Ala Pro Phe Thr Ala Gly Leu Ser Leu Ser Ile Thr Ala Ala Gly Val
       -5                  1               5                   10
   Gly Leu Gly Ile Ala Ser Ala Thr Ala Gly Ile Ala Ser Ser Ile Val
                   15                  20                  25
   Glu Asn Thr Tyr Thr Arg Ser Ala Glu Leu Thr Ala Ser Arg Leu Thr
               30                  35                  40
   Ala Thr Ser Thr Asp Gln Leu Glu Ala Leu Arg Asp Ile Leu His Asp
           45                  50                  55
   Ile Thr Pro Asn Val Leu Ser Phe Ala Leu Asp Phe Asp Glu Ala Thr
       60                  65                  70
   Lys Met Ile Ala Asn Asp Val His Thr Leu Arg Arg Ser Lys Ala Thr
   75                  80                  85                  90
   Val Gly Arg Pro Leu Ile Ala Trp Arg Tyr Val Pro Ile Asn Val Val
                   95                  100                 105
   Glu Thr Leu Arg Thr Arg Gly Ala Pro Thr Arg Ile Val Arg Lys Val
               110                 115                 120
   Ala Arg Asn Leu Gly Lys Ala Thr Ser Gly Val Leu Val Val Leu Asp
           125                 130                 135
   Val Val Asn Leu Val Gln Asp Ser Leu Asp Leu His Lys Gly Glu Lys
       140                 145                 150
   Ser Glu Ser Ala Glu Leu Leu Arg Gln Trp Ala Gln Glu Leu Glu Glu
   155                 160                 165                 170
   Asn Leu Asn Glu Leu Thr His Ile His Gln Ser Leu Lys Ala Gly
                   175                 180                 185

## Description

The present application, refere to extended cDNAs which were disclosed in several United States Provisional Patent Applications. Table I lists the SEQ ID Nos. of the extended cDNA in the present claims, the SEQ ID Nos. of the identical or nearly identical extended cDNA in the provisional applications, and the identify of the provisional application in which the extended cDNA was disclosed.

### Background of the Invention

The estimated 50.000-100.000 genes scattered along the human chromosomes offer tremondous promise for the understanding, diagnosis, and treatment of human diseases. In addition, probes capable of specifically hybridizing to loci distributed throughout the human genome find applications in the construction of high resolution chromosome maps and in the identification of individuals.

In the past, the characterization of even a single human gene was a painstaking process, requiring years of effort. Recent developments in the areas of cloning vectors. DNA sequencing, and computer technology have merged to greatly accelerate the rate at which human genes can be isolated, sequenced, mapped, and characterized. Cloning vectors such as yeast artificial chromosomes (YACs) and bacterial artificial chromosomes (BACs) are able to accept DNA inserts ranging from 300 to 1000 kilobases (kb) or 100-400 kb in length respectively, thereby facilitating the manipulation and ordering of DNA sequences distributed over great distances on the human chromosomes. Automated DNA sequencing machines permit the rapid sequencing of human genes. Bioinformatics software enables the comparison of nucleic acid and protein sequences, thereby assisting in the characterization of human gene products.

Currently, two different approaches are being pursued for identifying and characterizing the genes distributed along the human genome. In one approach, large fragments of genomic DNA are isolated, cloned, and sequenced. Potential open reading frames in these genomic sequences are identified using bio-informaties software. However, this approach entails sequencing large stretches of human DNA which do not encode proteins in order to find the protein encoding sequences scattered throughout the genome. In addition to requiring extensive sequencing, the bio-informatics software may mischaracterize the genomic sequences obtained. Thus, the software may produce-false positives in which non-coding DNA is mischaracterized as coding DNA or false negatives in which coding DNA is mislabeled as non-coding DNA.

An alternative approach takes a more direct route to identifying and characterizing human genes. In this approach, complementary DNAs (cDNAs) are synthesized from isolated messenger RNAs (mRNAs) which encode human proteins. Using this approach, sequencing is only performed on DNA which is derived from protein coding portions of the genome. Often, only short stretches of the cDNAs are sequenced to obtain sequences called expressed sequence tags (ESTs). The ESTs may then be used to isolate or purify extended cDNAs which include sequences adjacent to the EST sequences. The extended cDNAs may contain all of the sequence of the EST which was used to obtain them or only a portion of the sequence of the EST which was used to obtain them. In addition, the extended cDNAs may contain the full coding sequence of the gene from which the EST was derived or, alternatively, the extended cDNAs may include portions of the coding sequence of the gene from which the EST was derived. It will be appreciated that there may be several extended cDNAs which include the EST sequence as a result of alternate splicing or the activity of alternative promoters.

In the past, the short EST sequences which could be used to isolate or purify extended cDNAs were often obtained from oligo-dT primed cDNA libraries. Accordingly, they mainly corresponded to the 3' untranslated region of the mRNA. In part, the prevalence of EST sequences derived from the 3' end of the mRNA is a result of the fact that typical techniques for obtaining cDNAs, are not well suited for isolating cDNA sequences derived from the 5' ends of mRNAs. (Adams et al., Nature 377:174, 1996, Hillier et al., Genome Res. 6:807-828, 1996).

In addition, in those reported instances where longer cDNA sequences have been obtained, the reported sequences typically correspond to coding sequences and do not include the full 5' untranslated region of the mRNA from which the cDNA is derived. Such incomplete sequences may not include the first exon of the mRNA, particularly in situations where the first exon is short. Furthermore, they may not include some exons, often short ones, which are located upstream of splicing sites. Thus, there is a need to obtain sequences derived from the 5' ends of mRNAs which can be used to obtain extended cDNAs which may include the 5' sequences contained in the 5' ESTs.

While many sequences derived from human chromosomes have practical applications, approaches based on the identification and characterization of those chromosomal sequences which encode a protein product are particularly relevant to diagnostic and therapeutic uses. Of the 50,000-100,000 protein coding genes, those genes encoding proteins which are secreted from the cell in which they are synthesized, as well as the secreted proteins themselves, are particularly valuable as potential therapeutic agents. Such proteins are often involved in cell to cell communication and may be responsible for producing a clinically relevant response in their target cells.

In fact, several secretory proteins, including tissue plasminogen activator, G-CSF, GM-CSF, erythropoietin, human growth hormone, insulin, interferon-α, interferon-β, interferon-γ, and interleukin-2, are currently in clinical use. These proteins are used to treat a wide range of conditions, including acute myocardial infarction, acute ischemic stroke, anemia, diabetes, growth hormone deficiency, hepatitis, kidney carcinoma, chemotherapy induced neutropenia and multiple sclerosis. For these reasons, extended cDNAs encoding secreted proteins or portions thereof represent a particularly valuable source of therapeutic agents. Thus, there is a need for the identification and characterization of secreted proteins and the nucleic acids encoding them.

In addition to being therapeutically useful themselves, secretory proteins include short peptides, called signal peptides, at their amino termini which direct their secretion. These signal peptides are encoded by the signal sequences located at the 5' ends of the coding sequences of genes encoding secreted proteins. Because these signal peptides will direct the extracellular secretion of any protein to which they are operably linked, the signal sequences may be exploited to direct the efficient secretion of any protein by operably linking the signal sequences to a gene encoding the protein for which secretion is desired. This may prove beneficial in gene therapy strategies in which it is desired to deliver a particular gene product to cells other than the cell in which it is produced. Signal sequences encoding signal peptides also find application in simplifying protein purification techniques. In such applications, the extracellular secretion of the desired protein greatly facilitates purification by reducing the number of undesired proteins from which the desired protein must be selected. Thus, there exists a need to identify and characterize the 5' portions of the genes for secretory proteins which encode signal peptides.

Public information on the number of human genes for which the promoters and upstream regulatory regions have been identified and characterized is quite limited. In part this may be due to the difficulty of isolating such regulatory sequences. Upstream regulatory sequences such as transcription factor binding sites are typically too short to be utilized as probes for isolating promoters from human genomic libraries. Recently, some approaches have been developed to isolate human promoters. One of them consists of making a CpG island library (Cross, S.H. et al., Purification of CpG Islands using a Methylated DNA Binding Column, Nature Genetics 6: 236-244 (1994)). The second consists of isolating human genomic DNA sequences containing Spel binding sites by the use of Spel binding protein. (Mortlock et al., Genome Res. 6:327-335. 1996). Both of these approaches have their limits due to a lack of specificity or of comprehensiveness.

5' ESTs and extended cDNAs obtainable therefrom may be used to efficiently identify and isolate upstream regulatory regions which control the location, developmental stage, rate, and quantity of protein synthesis, as well as the stability of the mRNA. (Theil et al., BioFactors 4:87-93, (1993). Once identified and characterized, these regulatory regions may be utilized in gene therapy or protein purification schemes to obtain the desired amount and locations of protein synthesis or to inhibit, reduce, or prevent the synthesis of undesirable gene products.

In addition, ESTs containing the 5' ends of secretory protein genes or extended cDNAs which include sequences adjacent to the sequences of the ESTs may include sequences useful as probes for chromosome mapping and the identification of individuals. Thus, there is a need to identify and characterize the sequences upstream of the 5' coding sequences of genes encoding secretory proteins.

### Summary of the Invention

Described herein are purified, isolated, or recombinant extended cDNAs which encode secreted proteins or fragments thereof. The purified, isolated or recombinant cDNAs may contain the entire open reading frame of their corresponding mRNAs, including a start codon and a stop codon. For example, the extended cDNAs may include nucleic acids encoding the signal peptide as well as the mature protein. Alternatively, the extended cDNAs may contain a fragment of the open reading frame. In some embodiments, the fragment may encode only the sequence of the mature protein. Alternatively, the fragment may encode only a portion of the mature protein. Also described is a nucleic acid which encodes the signal peptide of a secreted protein.

The present extended cDNAs were obtained using ESTs which include sequences derived from the authentic 5' ends of their corresponding mRNAs. As used herein the terms "EST" or "5' EST" refer to the short cDNAs which were used to obtain the extended cDNAs of the present invention. As used herein, the term "extended cDNA" refers to the cDNAs which include sequences adjacent to the 5' EST used to obtain them. The extended cDNAs may contain all or a portion of the sequence of the EST which was used to obtain them. The term "corresponding mRNA" refers to the mRNA which was the template for the cDNA synthesis which produced the 5' EST. As used herein, the term "purified" does not require absolute purity; rather, it is intended as a relative definition. Individual extended cDNA clones isolated from a cDNA library have been conventionally purified to electrophoretic homogeneity. The sequences obtained from these clones could not be obtained directly either from the library or from total human DNA. The extended cDNA clones are not naturally occurring as such, but rather are obtained via manipulation of a partially purified naturally occurring substance (messenger RNA). The conversion of mRNA into a cDNA library involves the creation of a synthetic substance (cDNA) and pure individual cDNA clones can be isolated from the synthetic library by clonal selection. Thus, creating a cDNA library from messenger RNA and subsequently isolating individual clones from that library results in an approximately 10⁴-10⁶ fold purification of the native message. Purification of starting material or natural material to at least one order of magnitude, preferably two or three orders, and more preferably four or five orders of magnitude is expressly contemplated.

As used herein, the term "isolated" requires that the material be removed from its original environment (e.g., the natural environment if it is naturally occurring). For example, a naturally-occurring polynucleotide present in a living animal is not isolated, but the same polynucleotide, separated from some or all of the coexisting materials in the natural system, is isolated.

As used herein, the term "recombinant" means that the extended cDNA is adjacent to "backbone" nucleic acid to which it is not adjacent in its natural environment. Additionally, to be "enriched" the extended cDNAs will represent 5% or more of the number of nucleic acid inserts in a population of nucleic acid backbone molecules. Backbone molecules according to the present invention include nucleic acids such as expression vectors, self-replicating nucleic acids, viruses, integrating nucleic acids, and other vectors or nucleic acids used to maintain or manipulate a nucleic acid insert of interest. Preferably, the enriched extended cDNAs represent 15% or more of the number of nucleic acid inserts in the population of recombinant backbone molecules. More preferably, the enriched extended cDNAs represent 50% or more of the number of nucleic acid inserts in the population of recombinant backbone molecules. In a highly preferred embodiment, the enriched extended cDNAs represent 90% or more of the number of nucleic acid inserts in the population of recombinant backbone molecules. "Stringent", "moderate," and "low" hybridization conditions are as defined in Example 29.

Unless otherwise indicated, a "complementary" sequence is fully complementary. Thus, extended cDNAs encoding secreted polypeptides or fragments thereof which are present in cDNA libraries in which one or more extended cDNAs encoding secreted polypeptides or fragments thereof make up 5% or more of the number of nucleic acid inserts in the backbone molecules are "enriched recombinant extended eDNAs" as defined herein. Likewise, extended cDNAs encoding secreted polypeptides or fragments thereof which are in a population of plasmids in which one or more extended cDNAs of the present invention have been inserted such that they represent 5% or more of the number of inserts in the plasmid backbone are " enriched recombinant extended cDNAs" as defined herein. However, extended cDNAs encoding secreted polypeptides or fragments thereof which are in cDNA libraries in which the extended cDNAs encoding secreted polypeptides or fragments thereof constitute less than 5% of the number of nucleic acid inserts in the population of backbone molecules, such as libraries in which backbone molecules having a cDNA insert encoding a secreted polypeptide are extremely rare, are not "enriched recombinant extended cDNAs."

In particular, described herein are extended cDNAs which were derived from genes encoding secreted proteins. As used herein, a "secreted" protein is one which, when expressed in a suitable host cell, is transported across or through a membrane, including transport as a result of signal peptides in its amino acid sequence. "Secreted" proteins include without limitation proteins secreted wholly (e.g. soluble proteins), or partially (e.g. receptors) from the cell in which they are expressed. "Secreted" proteins also include without limitation proteins which are transported across the membrane of the endoplasmic reticulum.

Extended cDNAs encoding secreted proteins may include nucleic acid sequences, called signal sequences, which encode signal peptides which direct the extracellular secretion of the proteins encoded by the extended cONAs. Generally, the signal peptides are located at the amino termini of secreted proteins.

Secreted proteins are translated by ribosomes associated with the "rough" endoplasmic reticulum. Generally, secreted proteins are co-translationally transferred to the membrane of the endoplasmic reticulum. Association of the ribosome with the endoplasmic reticulum during translation of secreted proteins is mediated by the signal peptide. The signal peptide is typically cleaved following its co-translational entry into the endoplasmic reticulum. After delivery to the endoplasmic reticulum, secreted proteins may proceed through the Golgi apparatus. In the Golgi apparatus, the proteins may undergo post-translational modification before entering secretory vesicles which transport them across the cell membrane.

The extended cDNAs described herein have several important applications. For example, they may be used to express the entire secreted protein which they encode. Alternatively, they may be used to express portions of the secreted protein. The portions may comprise the signal peptides encoded by the extended cDNAs or the mature proteins encoded by the extended cDNAs (i.e. the proteins generated when the signal peptide is cleaved off). The portions may also comprise polypeptides having at least 10 consecutive amino acids encoded by the extended cDNAs. Alternatively, the portions may comprise at least 15 consecutive amino acids encoded by the extended cDNAs.

The portions may comprise at least 25 consecutive amino acids encoded by the extended cDNAs. The portions may comprise at least 40 amino acids encoded by the extended cDNAs.

Antibodies which specifically recognize the entire secreted proteins encoded by the extended cDNAs or fragments thereof having at least 10 consecutive amino acids, at least 15 consecutive amino acids, at least 25 consecutive amino acids, or at least 40 consecutive amino acids may also be obtained as described below. Antibodies which specifically recognize the mature protein generated when the signal peptide is cleaved may also be obtained as described below. Similarly, antibodies which specifically recognize the signal peptides encoded by the extended cDNAs may also be obtained.

The extended cDNAs may include the signal sequence. The extended cDNAs may include the full coding sequence for the mature protein (i.e. the protein generated when the signal polypeptide is cleaved off). In addition, the extended cDNAs may include regulatory regions upstream of the translation start site or downstream of the stop codon which control the amount, location, or developmental stage of gene expression. As discussed above, secreted proteins are therapeutically important. Thus, the proteins expressed from the cDNAs may be useful in treating or controlling a variety of human conditions. The extended cDNAs may also be used to obtain the corresponding genomic DNA. The term "corresponding genomic DNA" refers to the genomic DNA which encodes mRNA which includes the sequence of one of the strands of the extended cDNA in which thymidine residues in the sequence of the extended cDNA are replaced by uracil residues in the mRNA.

The extended cDNAs or genomic DNAs obtained therefrom may be used in forensic procedures to identify individuals or in diagnostic procedures to identify individuals having genetic diseases resulting from abnormal expression of the genes corresponding to the extended cDNAs. In addition, the present DNAs are useful for constructing a high resolution map of the human chromosomes.

Also described are secretion vectors capable of directing the secretion of a protein of interest. Such vectors may be used in gene therapy strategies in which it is desired to produce a gene product in-one cell which is to be delivered to another location in the body. Secretion vectors may also facilitate the purification of desired proteins.

Also described are expression vectors capable of directing the expression of an inserted gene in a desired spatial or temporal manner or at a desired level. Such vectors may include sequences upstream of the extended cDNAs such as promoters or upstream regulatory sequences.

In addition, the present DNAs may also be used for gene therapy to control or treat genetic diseases. Signal peptides may also be fused to heterologous proteins to direct their extracellular secretion.

Described herein is a purified or isolated nucleic acid comprising the sequence of SEQ ID NO: 45 or a sequence complementary thereto. In one aspect, the nucleic acid is recombinant.

Also described is a purified or isolated nucleic acid comprising at least 10 consecutive bases of the sequence of SEQ ID NO: 45 or a sequence complementary thereto. In one aspect, the nucleic acid comprises at least 15, 25, 30, 40, 50, 75, or 100 consecutive bases of the sequence of SEQ ID NO: 45 or a sequence complementary thereto. The nucleic acid may be a recombinant nucleic acid.

Also described is a purified or isolated nucleic acid of at least 15 bases capable of hybridizing under stringent conditions to the sequence of SEQ ID NO: 45 or a sequence complementary to the sequence of SEQ ID NO: 45. In one aspect, the nucleic acid is recombinant.

Also described is a purified or isolated nucleic acid comprising the full coding sequence of SEQ ID NO: 45, wherein the full coding sequence optionally comprises the sequence encoding signal peptide as well as the sequence encoding mature protein. In one aspect, the nucleic acid is recombinant.

Also described is a purified or isolated nucleic acid comprising the nucleotides of SEQ ID NO: 45 which encode a mature protein. In one aspect, the nucleic acid is recombinant.

Also described is a purified or isolated nucleic acid comprising the nucleotides of SEQ ID NO: 45 which encode the signal peptide. In one aspect, the nucleic acid is recombinant.

Also described is a purified or isolated nucleic acid encoding a polypeptide having the sequence of SEQ ID NO: 146.

Also described is a purified or isolated nucleic acid encoding a polypeptide having the sequence of a mature protein included in the sequence of SEQ ID NO: 146.

Also described is a purified or isolated nucleic acid encoding a polypeptide having the sequence of a signal peptide included in the sequence of SEQ ID NO: 146.

Also described is a purified or isolated protein comprising the sequence of SEQ ID NO: 146.

Also described is a purified or isolated polypeptide comprising at least 10 consecutive amino acids of the sequence of SEQ ID NO: 146. In one aspect, the purified or isolated polypeptide comprises at least 15, 20, 25, 35, 50, 75, 100, 150 or 200 consecutive amino acids of the sequence of SEQ ID NO: 146. In still another aspect, the purified or isolated polypeptide comprises at least 25 consecutive amino acids of the sequence of SEQ ID NO: 146.

Also described is an isolated or purified polypeptide comprising a signal peptide of the polypeptide of SEQ ID NO: 146.

Also described is an isolated or purified polypeptide comprising a mature protein of the polypeptide of SEQ ID NO: 146.

Also described is a method of making a protein comprising the sequence of SEQ ID NO: 146, comprising the steps of obtaining a cDNA comprising the sequence of SEQ ID NO: 45, inserting the cDNA in an expression vector such that the cDNA is operably linked to a promoter, and introducing the expression vector into a host cell whereby the host cell produces the protein encoded by said cDNA. In one aspect, the method further comprises the step of isolating the protein.

Also described is a protein obtainable by the method described in the preceding paragraph.

Also described is a method of making a protein comprising the amino acid sequence of the mature protein contained in the sequence of SEQ ID NO: 146, comprising the steps of obtaining a cDNA comprising the nucleotide sequence of SEQ ID NO: 45 which encode for the mature protein, inserting the cDNA in an expression vector such that the cDNA is operably linked to a promoter, and introducing the expression vector into a host cell whereby the host cell produces the mature protein encoded by the cDNA. In one aspect, the method further comprises the step of isolating the protein.

Also described is a mature protein obtainable by the method described in the preceding paragraph.

Also described is a host cell containing the purified or isolated nucleic acids comprising the sequence of SEQ ID NO: 45 or a sequence complementary thereto described herein.

Also described is a host cell containing a purified or isolated nucleic acid comprising the full coding sequence of SEQ ID NO: 45, wherein the full coding sequence comprises the sequence encoding signal peptide and the sequence encoding mature protein described herein.

Also described is a host cell containing a purified or isolated nucleic acid comprising the nucleotides of SEQ ID NO: 45 which encode a mature protein which are described herein.

Also described is a host cell containing the purified or isolated nucleic acid comprising the nucleotides of SEQ ID NO: 45 which encode the signal peptide which are described herein.

Also described is a purified or isolated antibody capable of specifically binding to a protein having the sequence of SEQ ID NO: 146. In one aspect, the antibody is capable of binding to a polypeptide comprising at least 10 consecutive amino acids of the sequence of SEQ ID NO: 146.

Also described is an array of cDNAs or fragments thereof of at least 15 nucleotides in length which includes at least one of the sequences of SEQ ID NOs: 40-140 and 242-377, or one of the sequences complementary to the sequences of SEQ ID NOs: 40-140 and 242-377, or a fragment thereof of at least 15 consecutive nucleotides. In one aspect, the array includes at least two of the sequences of SEQ ID NOs: 40-140 and 242-377, the sequences complementary to the sequences of SEQ ID NOs: 40-140 and 242-377, or fragments thereof of at least 15 consecutive nucleotides. In another aspect, the array includes at least five of the sequences of SEQ ID NOs: 40-140 and 242-377, the sequences complementary to the sequences of SEQ ID NOs: 40-140 and 242-377, or fragments thereof of at least 15 consecutive nucleotides.

Also described are purified polynucleotides comprising an insert from a clone deposited in a deposit having an accession number listed in Table VI or a fragment thereof comprising a contiguous span of at least 8, 10, 12, 15, 20, 25, 40, 60, 100, or 200 nucleotides of said insert. Also described are purified polypeptides which comprise, consist of, or consist essentially of an amino acid sequence encoded by the insert from a clone deposited in a deposit having an accession number listed in Table VI, as well as polypeptides which comprise a fragment of said amino acid sequence consisting of a signal peptide, a mature protein, or a contiguous span of at least 5, 8, 10, 12, 15, 20, 25, 40, 60, 100, or 200 amino acids encoded by said insert.

Accordingly, in one aspect the invention provides a purified or isolated polypeptide comprising a sequence having at least 80% identity to the polypeptide of SEQ ID NO: 146. In a further aspect, the invention provides a purified or isolated nucleic acid encoding a polypeptide comprising or consisting of SEQ ID NO: 146. The invention further provides an expression vector comprising the nucleic acid and a host cell comprising the recombinant nucleic acid. Also provided is a method of making the polypeptide of the invention comprising the steps of: (a) obtaining a cDNA encoding said polypeptide; (b) inserting said cDNA in an expression vector such that said cDNA is operably linked to a promoter; and (c) introducing said expression vector into a host cell whereby said host cell produces said polypeptide. The invention further provides a purified or isolated antibody capable of specifically binding to the polypeptide of the invention.

EMBL Database Accession No. Z95114 provides a human DNA sequence from clone 212A2. Duchateau et al., 1997, Journal of Biological Chemistry, 272: 25576-25582, is concerned with Apolipoprotein L, a new human high density lipoprotein apolipoprotein expressed by the pancreas.

### Brief Description of the Drawings

Figure 1 is a summary of a procedure for obtaining cDNAs which have been selected to include the 5'ends of the mRNAs from which they are derived.
Figure 2 is an analysis of the 43 amino terminal amino acids of all human SwissProt proteins to determine the frequency of false positives and false negatives using the techniques for signal peptide identification described herein.
Figure 3 shows the distribution of von Heijne scores for 5' ESTs in each of the categories described herein and the probability that these 5' ESTs encode a signal peptide.
Figure 4 shows the distribution of 5' ESTs in each category and the number of 5' ESTs in each category having a given minimum von Heijne's score.
Figure 5 shows the tissues from which the mRNAs corresponding to the 5'ESTs in each of the categories described herein were obtained.
Figure 6 illustrates a method for obtaining extended cDNAs.
Figure 7 is a map of pED6dpc2. pED6dpc2 is derived from pED6dpc1 by insertion of a new polylinker to facilitate cDNA cloning. SSt cDNAs are cloned between EcoRI and Notl. PED vectors are described in Kaufman et al. (1991). NAR 19: 4485-4490.
Figure 8 provides a schematic description of the promoters isolated and the way they are assembled with the corresponding 5' tags.
Figure 9 describes the transcription factor binding sites present in each of these promoters.

### Detailed Description of The Preferred Embodiment

### 1. Obtaining 5' ESTs

The present extended cDNAs were obtained using 5' ESTs which were isolated as described below.

### A. Chemical Methods for Obtaining mRNAs having intact 5' Ends

In order to obtain the 5' ESTs used to obtain the extended cDNAs described herein, mRNAs having intact 5' ends must be obtained. Currently, there are two approaches for obtaining such mRNAs. One of these approaches is a chemical modification method involving derivatization of the 5' ends of the mRNAs and selection of the derivatized mRNAs. The 5' ends of eucaryotic mRNAs possess a structure referred to as a "cap" which comprises a guanosine methylated at the 7 position. The cap is joined to the first transcribed base of the mRNA by a 5', 5'-triphosphate bond. In some instances, the 5' guanosine is methylated in both the 2 and 7 positions. Rarely, the 5' guanosine is trimethylated at the 2. 7 and 7 positions. In the chemical method for obtaining mRNAs having intact 5' ends, the 5' cap is specifically derivatized and coupled to a reactive group on an immobilizing substrate. This specific derivatization is based on the fact that only the ribose linked to the methylated guanosine at the 5' end of the mRNA and the ribose linked to the base at the 3' terminus of the mRNA, possess 2', 3'-cis diols. Optionally, where the 3' terminal ribose has a 2', 3'-cis dial, the 2', 3'-cis diol at the 3' end may be chemically modified, substituted, converted, or eliminated, leaving only the ribose linked to the methylated guanosine at the 5' end of the mRNA with a 2', 3'-cis diol. A variety of techniques are available for eliminating the 2', 3'-cis diol on the 3' terminal ribose. For example, controlled alkaline hydrolysis may be used to generate mRNA fragments in which the 3' terminal ribose is a 3'-phosphate, 2'-phosphate or (2'. 3')-cyclophosphate. Thereafter, the fragment which includes the original 3' ribose may be eliminated from the mixture through chromatography on an oligo-dt column. Alternatively, a base which lacks the 2', 3'-cis diol may be added to the 3' end of the mRNA using an RNA ligase such as T4 RNA ligase. Example 1 below describes a method for ligation of pCp to the 3' end of messenger RNA.

### EXAMPLE 1

### Ligation of the Nucleoside Diphosphate pCp to the 3' End of Messenger RNA

1 µg of RNA was incubated in a final reaction medium of 10 µl in the presence of 5 U of T₄ phage RNA ligase in the buffer provided by the manufacturer (Gibco - BRL). 40 U of the RNase inhibitor RNasin (Promega) and, 2 µl of ³²pCp (Amersham #PB 10208).

The incubation was performed at 37°C for 2 hours or overnight at 7-8°C.

Following modification or elimination of the 2', 3'-cis diol at the 3' ribose, the 2', 3'-cis diol present at the 5' end of the mRNA may be oxidized using reagents such as NaBH₄, NaBH₃CN, or sodium periodate, thereby converting the 2', 3'-cis diol to a dialdehyde. Example 2 describes the oxidation of the 2', 3'-cis diol at the 5' end of the mRNA with sodium periodate.

### EXAMPLE 2

### Oxidation of 2'. 3'-cis diol at the 5' End of the mRNA

0.1 OD unit of either a capped oligoribonucleotide of 47 nucleotides (including the cap) or an uncapped oligoribonucleotide of 46 nucleotides were treated as follows. The oligoribonucleotides were produced by in vitro transcription using the transcription kit "AmpliScribe T7" (Epicentre Technologies). As indicated below, the DNA template for the RNA transcript contained a single cytosine. To synthesize the uncapped RNA, all four NTPs were included in the in vitro transcription reaction. To obtain the capped RNA, GTP was replaced by an analogue of the cap, m7G(5')ppp(5')G. This compound, recognized by polymerase, was incorporated into the 5' end of the nascent transcript during the step of initiation of transcription but was not capable of incorporation during the extension step. Consequently, the resulting RNA contained a cap at its 5' end. The sequences of the oligoribonucleotides produced by the in vitro transcription reaction were:
+ Cap:
   5'm7GpppGCAUCCUACUCCCAUCCAAUUCCACCCUAACUCCUCCCAUCUCCAC-3' (SED ID NO:1)
-Cap:
   5'-pppGCAUCCUACUCCCAUCCAAUUCCACCCUAACUCCUCCCAUCUCCAC-3' (SEQ ID NO:2)

The oligoribonucleotides were dissolved in 9 µl of acetate buffer (0.1 M sodium acetate, pH 5.2) and 3 µl of freshly prepared 0.1 M sodium periodate solution. The mixture was incubated for 1 hour in the dark at 4°C or room temperature. Thereafter, the reaction was stopped by adding 4 µl of 10% ethylene glycol. The product was ethanol precipitated, resuspended in 10µl or more of water or appropriate buffer and dialyzed against water.

The resulting aldehyde groups may then be coupled to molecules having a reactive amine group, such as hydrazine, carbazide, thiocarbazide or semicarbazide groups, in order to facilitate enrichment of the 5' ends of the mRNAs. Molecules having reactive amine groups which are suitable for use in selecting mRNAs having intact 5' ends include avidin, proteins, antibodies, vitamins, ligands capable of specifically binding to receptor molecules, or oligonucleotides. Example 3 below describes the coupling of the resulting dialdehyde to biotin.

### EXAMPLE 3

### Coupling of the Dialdehyde with Biotin

The oxidation product obtained in Example 2 was dissolved in 50 µl of sodium acetate at a pH of between 5 and 5.2 and 50 µl of freshly prepared 0.02 M solution of biotin hydrazide in a methoxyethanol/water mixture (1:1) of formula:

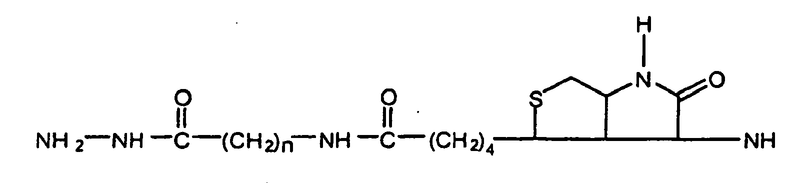

In the compound used in these experiments, n=5. However, it will be appreciated that other commercially available hydrazides may also be used, such as molecules of the formula above in which n varies from 0 to 5.

The mixture was then incubated for 2 hours at 37°C. Following the incubation, the mixture was precipitated with ethanol and dialyzed against distilled water.

Example 4 demonstrates the specificity of the biotinylation reaction.

### EXAMPLE 4

### Specificity of Biotinylation

The specificity of the biotinylation for capped mRNAs was evaluated by gel electrophoresis of the following samples:
Sample 1. The 46 nucleotide uncapped in vitro transcript prepared as in Example 2 and labeled with ³²pCp as described in Example 1.
Sample 2. The 46 nucleotide uncapped in vitro transcript prepared as in Example 2, labeled with ³²pCp as described in Example 1, treated with the oxidation reaction of Example 2, and subjected to the biotinylation conditions of Example 3.
Sample 3. The 47 nucleotide capped in vitro transcript prepared as in Example 2 and labeled with ³²pCp as described in Example 1.
Sample 4. The 47 nucleotide capped in vitro transcript prepared as in Example 2, labeled with ³²pCp as described in Example 1, treated with the oxidation reaction of Example 2, and subjected to the biotinylation conditions of Example 3.
Samples 1 and 2 had indentical migration rates, demonstrating that the uncapped RNAs were not oxidized and biotinylated. Sample 3 migrated more slowly than Samples 1 and 2, while Sample 4 exhibited the slowest migration. The difference in migration of the RNAs in Samples 3 and 4 demonstrates that the capped RNAs were specifically biotinylated.

In some cases, mRNAs having intact 5' ends may be enriched by binding the molecule containing a reactive amine group to a suitable solid phase substrate such as the inside of the vessel containing the mRNAs, magnetic beads, chromatography matrices, or nylon or nitrocellulose membranes. For example, where the molecule having a reactive amine group is biotin, the solid phase substrate may be coupled to avidin or streptavidin. Alternatively, where the molecule having the reactive amine group is an antibody or receptor ligand, the solid phase substrate may be coupled to the cognate antigen or receptor. Finally, where the molecule having a reactive amine group comprises an oligonucleotide, the solid phase substrate may comprise a complementary oligonucleotide.

The mRNAs having intact 5' ends may be released from the solid phase following the enrichment procedure. For example, where the dialdehyde is coupled to biotin hydrazide and the solid phase comprises streptavidin, the mRNAs may be released from the solid phase by simply heating to 95 degrees Celsius in 2% SOS. In some methods, the molecule having a reactive amine group may also be cleaved from the mRNAs having intact 5' ends following enrichment. Example 5 describes the capture of biotinylated mRNAs with streptavidin coated beads and the release of the biotinylated mRNAs from the beads following enrichment.

### EXAMPLE 5

### Capture and Release of Biotinylated mRNAs Using Strepatividin Coated Beads

The streptavidin-coated magnetic beads were prepared according to the manufacturer's instructions (CPG Inc., USA). The biotinylated mRNAs were added to a hybridization buffer (1.5 M NaCl, pH 5 - 6). After incubating for 30 minutes, the unbound and nonbiotinylated material was removed. The beads were washed several times in water with 1% SDS. The beads obtained were incubated for 15 minutes at 95°C in water containing 2% SDS.

Example 6 demonstrates the efficiency with which biotinylated mRNAs were recovered from the streptavidin coated beads.

### EXAMPLE 6

### Efficiency of Recovery of Biotinylated mRNAs

The efficiency of the recovery procedure was evaluated as follows. RNAs were labeled with ³²pCp, oxidized, biotinylated and bound to streptavidin coated beads as described above. Subsequently, the bound RNAs were incubated for 5, 15 or 30 minutes at 95°C in the presence of 2% SDS.

The products of the reaction were analyzed by electrophoresis on 12% polyacrylamide gels under denaturing conditions (7 M urea). The gels were subjected to autoradiography. During this manipulation, the hydrazone bonds were not reduced.

Increasing amounts of nucleic acids were recovered as incubation times in 2% SDS increased, demonstrating that biotinylated mRNAs were efficiently recovered.

In an alternative method for obtaining mRNAs having intact 5' ends, an oligonucleotide which has been derivatized to contain a reactive amine group is specifically coupled to mRNAs having an intact cap. Preferably, the 3' end of the mRNA is blocked prior to the step in which the aldehyde groups are joined to the derivatized oligonucleotide, as described above, so as to prevent the derivatized oligonucleotide from being joined to the 3' end of the mRNA. For example, pCp may be attached to the 3' end of the mRNA using T4 RNA ligase. However, as discussed above, blocking the 3' end of the mRNA is an optional step. Derivatized oligonucleotides may be prepared as described below in Example 7.

### EXAMPLE 7

### Derivatization of the Oligonucleotide

An oligonucleotide phosphorylated at its 3' end was converted to a 3' hydrazide in 3' by treatment with an aqueous solution of hydrazine or of dihydrazide of the formula H₂N(R1)NH₂ at about 1 to 3 M, and at pH 4.5, in the presence of a carbodiimide type agent soluble in water such as 1-ethyl-3-(3-dimethytaminopropyl)carbodiimide at a final concentration of 0.3 M at a temperature of 8°C overnight.

The derivatized oligonucleotide was then separated from the other agents and products using a standard technique for isolating oligonucleotides.

As discussed above, the mRNAs to be enriched may be treated to eliminate the 3' OH groups which may be present thereon. This may be accomplished by enzymatic ligation of sequences lacking a 3' OH, such as pCp, as described above in Example 1. Alternatively, the 3' OH groups may be eliminated by alkaline hydrolysis as described in Example 8 below.

### EXAMPLE 8

### Alkaline Hydrolysis of mANA

The mRNAs may be treated with alkaline hydrolysis as follows. In a total volume of 100µl of O.1N sodium hydroxide, 1.5µg mRNA is incubated for 40 to 60 minutes at 4°C. The solution is neutralized with acetic acid and precipitated with ethanol.

Following the optional elimination of the 3' OH groups, the diol groups at the 5' ends of the mRNAs are oxidized as described below in Example 9.

### EXAMPLE 9

### Oxidation of Diols

Up to 1 00 unit of RNA was dissolved in 9 µl of buffer (0.1 M sodium acetate, pH 6-7 or water) and 3 µl of freshly prepared 0.1 M sodium periodate solution. The reaction was incubated for 1 h in the dark at 4°C or room temperature. Following the incubation, the reaction was stopped by adding 4 µl of 10% ethylene glycol. Thereafter the mixture was incubated at room temperature for 15 minutes. After ethanol precipitation, the product was resuspended in 10µl or more of water or appropriate buffer and dialyzed against water.

Following oxidation of the diol groups at the 5' ends of the mRNAs, the derivatized oligonucleotide was joined to the resulting aldehydes as described in Example 10.

### EXAMPLE 10

### Reaction of Aldehydes with Derivatized Oligonucleotides

The oxidized mRNA was dissolved in an acidic medium such as 50 µl of sodium acetate pH 4-6, 50 µl of a solution of the derivatized oligonucleotide was added such that an mRNA:derivatized oligonucleotide ratio of 1:20 was obtained and mixture was reduced with a borohydride. The mixture was allowed to incubate for 2 h at 37°C or overnight (14 h) at 10°C. The mixture was ethanol precipitated, resuspended in 10µl or more of water or appropriate buffer and dialyzed against distilled water. If desired, the resulting product may be analyzed using acrylamide gel electrophoresis, HPLC analysis, or other conventional techniques.

Following the attachment of the derivatized oligonucleotide to the mRNAs, a reverse transcription reaction may be performed as described in Example 11 below.

### EXAMPLE 11

### Reverse Transcription of mRNAs

An oligodeoxyribonucleotide was derivatized as follows. 3 00 units of an oligodeoxyribonucleotide of sequence ATCAAGAATTCGCACGAGACCATTA (SEQ 10 NO:3) having 5'-OH and 3'-P ends were dissolved in 70 µl of a 1.5 M hydroxybenzotriazole solution, pH 5.3, prepared in dimethylformamide/water (75:25) containing 2 µg of 1-ethyl-3-(3-dimethylaminopropyl)carbodiimide. The mixture was incubated for 2 h 30 min at 22°C. The mixture was then precipitated twice in LiClO₄/acetone. The pellet was resuspended in 200 µl of 0.25 M hydrazine and incubated at 8°C from 3 to 14 h. Following the hydrazine reaction, the mixture was precipitated twice in LiClO₄/acetone.

The messenger RNAs to be reverse transcribed were extracted from blocks of placenta having sides of 2 cm which had been stored at -80°C. The mRNA was extracted using conventional acidic phenol techniques. Oligo-dT chromatography was used to purify the mRNAs. The integrity of the mRNAs was checked by Northern-blotting.

The diol groups on 7 µg of the placental mRNAs were oxidized as described above in Example 9. The derivatized oligonucleotide was joined to the mRNAs as described in Example 10 above except that the precipitation step was replaced by an exclusion chromatography step to remove derivatized oligodeoxyribonucleotides which were not joined to mRNAs. Exclusion chromatography was performed as follows:

10 ml of AcA34 (BioSepra#230151) gel were equilibrated in 50 ml of a solution of 10 mM Tris pH 8.0, 300 mM NaCl, 1 mM EDTA, and 0.05% SDS. The mixture was allowed to sediment. The supernatant was eliminated and the gel was resuspended in 50 ml of buffer. This procedure was repeated 2 or 3 times.

A glass bead (diameter 3 mm) was introduced into a 2 ml disposable pipette (length 25 cm). The pipette was filled with the gel suspension until the height of the gel stabilized at 1 cm from the top of the pipette. The column was then equilibrated with 20 ml of equilibration buffer (10 mM Tris HCl pH 7.4, 20 mM NaCl).

10 µl of the mRNA which had been reacted with the derivatized oligonucleotide were mixed in 39 µl of 10 mM urea and 2 µl of blue-glycerol buffer, which had been prepared by dissolving 5 mg of bromophenol blue in 60% glycerol (v/v), and passing the mixture through a filter with a filter of diameter 0.45 µm.

The column was loaded. As soon as the sample had penetrated, equilibration buffer was added. 100 µl fractions were collected. Derivatized oligonucleotide which had not been attached to mRNA appeared in fraction 16 and later fractions. Fractions 3 to 15 were combined and precipitated with ethanol.

The mRNAs which had been reacted with the derivatized oligonucleotide were spotted on a nylon membrane and hybridized to a radioactive probe using conventional techniques. The radioactive probe used in these hybridizations was an oligodeoxyribonucleotide of sequence TAATGGTCTCGTGCGAATTCTTGAT (SEQ ID NO:4) which was anticomplemantary to the derivatized oligonucleotide and was labeled at its 5' end with ³²P. 1/10h of the mRNAs which had been reacted with the derivatized oligonucleotide was spotted in two spots on the membrane and the membrane was visualized by autoradiography after hybridization of the probe. A signal was observed, indicating that the derivatized oligonucleotide had been joined to the mRNA.

The remaining 9/10 of the mRNAs which had been reacted with the derivatized oligonucleotide was reverse transcribed as follows. A reverse transcription reaction was carried out with reverse transcriptase following the manufacturer's instructions. To prime the reaction, 50 pmol of nonamers with random sequence were used.

A portion of the resulting cDNA was spotted on a positively charged nylon membrane using conventional methods. The cDNAs were spotted on the membrane after the cDNA:RNA heteroduplexes had been subjected to an alkaline hydrolysis in order to eliminate the RNAs. An oligonucleotide having a sequence identical to that of the derivatized oligonucleotide was labeled at its 5' end with ³²P and hybridized to the cDNA blots using conventional techniques. Single-stranded cDNAs resulting from the reverse transcription reaction were spotted on the membrane. As controls, the blot contained 1 pmol, 100 fmol, 50 fmol, 10 fmol and 1 fmol respectively of a control oligodeoxyribonucleotide of sequence identical to that of the derivatized oligonucleotide. The signal observed in the spots containing the cDNA indicated that approximately 15 fmol of the derivatized oligonucleotide had been reverse transcribed.

These results demonstrate that the reverse transcription can be performed through the cap and, in particular, that reverse transcriptase crosses the 5'-P-P-P-5' bond of the cap of eukaryotic messenger RNAs.

The single stranded cDNAs obtained after the above first strand synthesis were used as template for PCR reactions. Two types of reactions were carried out. First, specific amplification of the mRNAs for the alpha globin, dehydrogenase, pp15 and elongation factor E4 were carried out using the following pairs of oligodeoxyribonucleotide primers.
alpha-globin
   GLO-S: CCG ACA AGA CCA ACG TCA AGG CCG C (SEQ ID NO:5)
   GLO-As: TCA CCA GCA GGC AGT GGC TTA GGA G 3' (SEQ ID NO:6)
dehydrogenase
   3 DH-S: AGT GAT TCC TGC TAC TTT GGA TGG C (SEQ ID NO:7)
   3 DH-As: GCT TGG TCT TGT TCT GGA GTT TAG A (SEQ ID NO:8)
pp15
   PP15-S: TCC AGA ATG GGA GAC AAG CCA ATT T (SEQ ID NO:9)
   PP15-As: AGG GAG GAG GAA ACA GCG TGA GTC C (SEQ ID NO:10)
Elongation factor E4
   EFA1-S: ATG GGA AAG GAA AAG ACT CAT ATC A (SEQ ID NO:11)
   EF1A-As: AGC AGC AAC AAT CAG GAC AGC ACA G (SEQ ID NO:12)

Non specific amplifications were also carried out with the antisense (_As) oligodeoxyribonucleotides of the pairs described above and a primer chosen from the sequence of the derivatized oligodeoxyribonucleotide (ATCAAGAATTCGCACGAGACCATTA) (SEQ ID NO:13).

A 1.5% agarose gel containing the following samples corresponding to the PCR products of reverse transcription was stained with ethidium bromide. (1/20th of the products of reverse transcription were used for each PCR reaction).

Sample 1: The products of a PCR reaction using the globin primers of SEQ ID NOs 5 and 6 in the presence of cDNA.

Sample 2: The products of a PCR reaction using the globin primers of SEQ ID NOs 5 and 6 in the absence of added cONA.

Sample 3: The products of a PCR reaction using the dehydrogenase primers of SEQ ID NOs 7 and 8 in the presence of cDNA.

Sample 4: The products of a PCR reaction using the dehydrogenase primers of SEQ ID NOs 7 and 8 in the absence of added cONA.

Sample 5: The products of a PCR reaction using the pp15 primers of SEQ ID NOs 9 and 10 in the presence of cDNA.

Sample 6: The products of a PCR reaction using the pp15 primers of SEQ ID NOs 9 and 10 in the absence of added cONA.

Sample 7: The products of a PCR reaction using the EIE4 primers of SEQ ID NOs 11 and 12 in the presence of added cDNA.

Sample 8: The products of a PCR reaction using the EIE4 primers of SEQ ID NOs 11 and 12 in the absence of added cDNA.

In Samples 1, 3. 5 and 7, a band of the size expected for the PCR product was observed, indicating the presence of the corresponding sequence in the cONA population.

PCR reactions were also carried out with the antisense oligonucleotides of the globin and dehydrogenase primers (SEQ ID NOs 6 and 8) and an oligonucleotide whose sequence corresponds to that of the derivatized oligonucleotide. The presence of PCR products of the expected size in the samples corresponding to samples 1 and 3 above indicated that the derivatized oligonucleotide had been incorporated.

The above examples summarize the chemical procedure for enriching mRNAs for those having intact 5' ends. Further detail regarding the chemical approaches for obtaining mRNAs having intact 5' ends are disclosed in International Application No. W096134981, published November 7, 1996.

Strategies based on the above chemical modifications to the 5' cap structure may be utilized to generate cDNAs which have been selected to include the 5' ends of the mRNAs from which they are derived. In one version of such procedures, the 5' ends of the mRNAs are modified as described above. Thereafter, a reverse transcription reaction is conducted to extend a primer complementary to the mRNA to the 5' end of the mRNA. Single stranded RNAs are eliminated to obtain a population of cONA/mRNA heteroduplexes in which the mRNA includes an intact 5' end. The resulting heteroduplexes may be captured on a solid phase coated with a molecule capable of interacting with the molecule used to derivatize the 5' end of the mRNA. Thereafter, the strands of the heteroduplexes are separated to recover single stranded first cONA strands which include the 5' end of the mRNA. Second strand cDNA synthesis may then proceed using conventional techniques. For example, the procedures disclosed in WO 96134981 or in Carninci, P. et al. High-Efficiency Full-Length cDNA Cloning by Biotinylated CAP Trapper. Genomics 37:327-336 (1996) may be employed to select cONAs which include the sequence derived from the 5' end of the coding sequence of the mRNA.

Following ligation of the oligonucleotide tag to the 5' cap of the mRNA, a reverse transcription reaction is conducted to extend a primer complementary to the mRNA to the 5' end of the mRNA. Following elimination of the RNA component of the resulting heteroduplex using standard techniques, second strand cDNA synthesis is conducted with a primer complementary to the oligonucleotide tag.

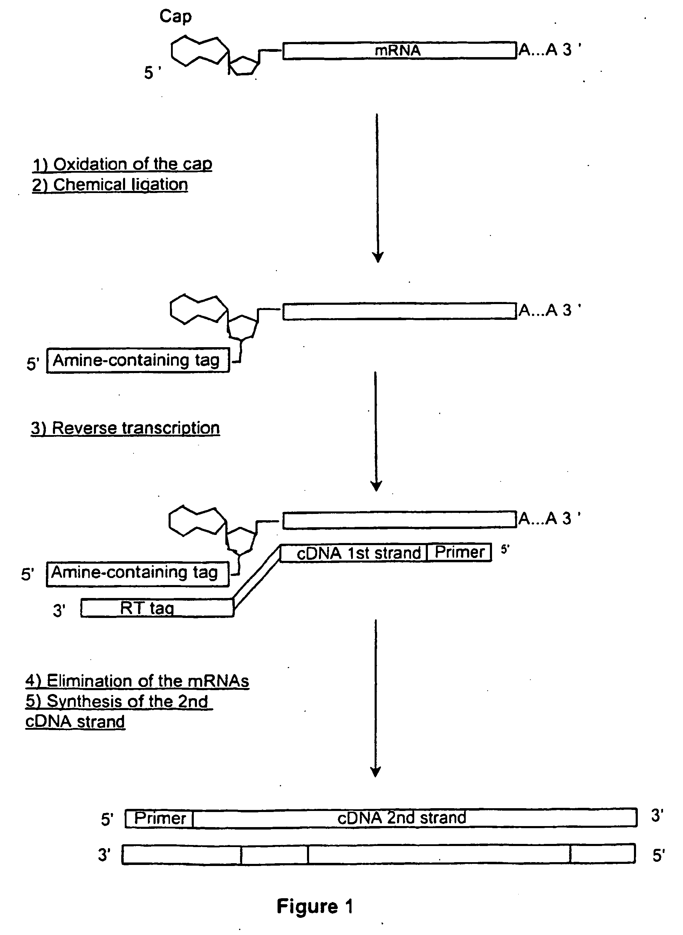

Figure 1 summarizes the above procedures for obtaining cDNAs which have been selected to include the 5' ends of the mRNAs from which they are derived.

### B. Enzymatic Methods for Obtaining mRNAs having Intact 5' Ends

Other techniques for selecting cDNAs extending to the 5' end of the mRNA from which they are derived are fully enzymatic. Some versions of these techniques are disclosed in Dumas Milne Edwards J.B. (Doctoral Thesis of Paris VI University, Le clonage des ADNc complets: difficultes et perspectives nouvelles. Apports pour l'etude de la regulation de l'expression de la tryptophane hydroxylase de rat, 20 Dec. 1993), EPO 625572 and Kato et al. Construction of a Human Full-Length cONA Bank. Gene 150:243-250 (1994).

Briefly, in such approaches, isolated mRNA is treated with alkaline phosphatase to remove the phosphate groups present on the 5' ends of uncapped incomplete mRNAs. Following this procedure, the cap present on full length mRNAs is enzymatically removed with a decapping enzyme such as T4 polynucleotide kinase or tobacco acid pyrophosphatase. An oligonucleotide, which may be either a DNA oligonucleotide or a DNA-RNA hybrid oligonucleotide having RNA at its 3' end, is then ligated to the phosphate present at the 5' end of the decapped mRNA using T4 RNA ligase. The oligonucleotide may include a restriction site to facilitate cloning of the cONAs following their synthesis. Example 12 below describes one enzymatic method based on the doctoral thesis of Dumas.

### EXAMPLE 12

### Enzymatic Approach for Obtaining 5' ESTs

Twenty micrograms of PolyA+ RNA were dephosphorylated using Calf Intestinal Phosphatase (Biolabs). After a phenol chloroform extraction, the cap structure of mRNA was hydrolysed using the Tobacco Acid Pyrophosphatase (purified as described by Shinshi et al., Biochemistry 15: 2185-2190, 1976) and a hemi 5'DNA/RNA-3' oligonucleotide having an unphosphorylated 5' end, a stretch of adenosine ribophosphate at the 3' end, and an EcoRI site near the 5' end was ligated to the 5'P ends of mRNA using the T4 RNA ligase (Biolabs). Oligonucleotides suitable for use in this procedure are preferably 30-50 bases in length. Oligonucleotides having an unphosphorylated 5' end may be synthesized by adding a fluorochrome at the 5' end. The inclusion of a stretch of adenosine ribophosphates at the 3' end of the oligonucleotide increases ligation efficiency. It will be appreciated that the oligonucleotide may contain cloning sites other than EcoRI.

Following ligation of the oligonucleotide to the phosphate present at the 5' end of the decapped mRNA, first and second strand cDNA synthesis may be carried out using conventional methods or those specified in EPO 625,572 and Kato et al. Construction of a Human Full-Length cONA Bank. Gene 150:243-250 (1994), and Dumas Milne Edwards, *supra*. The resulting cDNA may then be ligated into vectors such as those disclosed in Kato et al. Construction of a Human Full-Length cDNA Bank. Gene 150:243-250 (1994) or other nucleic acid vectors known to those skilled in the art using techniques such as those described in Sambrook et al., Molecular Cloning: A Laboratory Manual 2d Ed., Cold Spring Harbor Laboratory Press, 1989.

### II. Characterization of 5' ESTs

The above chemical and enzymatic approaches for enriching mRNAs having intact 5' ends were employed to obtain 5' ESTs. First, mRNAs were prepared as described in Example 13 below.

### EXAMPLE 13

### Preparation of mRNA

Total human RNAs or PolyA+ RNAs derived from 29 different tissues were respectively purchased from LABIMO and CLONTECH and used to generate 44 cDNA libraries as described below. The purchased RNA had been isolated from cells or tissues using acid guanidium thiocyanate-phenol-chloroform extraction (Chomczyniski, P and Sacchi, N., Analytical Biochemistry 162:156-159, 1987). PolyA+ RNA was isolated from total RNA (LABIMO) by two passes of oligodT chromatography, as described by Aviv and Leder (Aviv, H. and Leder, P., Proc. Natl. Acad. Sci. USA 69:1408-1412, 1972) in order to eliminate ribosomal RNA.

The quality and the integrity of the poly A+ were checked. Northern blots hybridized with a globin probe were used to confirm that the mRNAs were not degraded. Contamination of the PolyA + mRNAs by ribosomal sequences was checked using RNAs blots and a probe derived from the sequence of the 28S RNA. Preparations of mRNAs with less than 5% of ribosomal RNAs were used in library construction. To avoid constructing libraries with RNAs contaminated by exogenous sequences (prokaryotic or fungal), the presence of bacterial 16S ribosomal sequences or of two highly expressed mRNAs was examined using PCR.

Following preparation of the mRNAs, the above described chemical and/or the enzymatic procedures for enriching mRNAs having intact 5' ends discussed above were employed to obtain 5' ESTs from various tissues. In both approaches an oligonucleotide tag was attached to the cap at the 5' ends of the mRNAs. The oligonucleotide tag had an EcoRI site therein to facilitate later cloning procedures.

Following attachment of the oligonucleotide tag to the mRNA by either the chemical or enzymatic methods, the integrity of the mRNA was examined by performing a Northern blot with 200-500ng of mRNA using a probe complementary to the oligonucleotide tag.

### EXAMPLE 14

### cDNA Synthesis Using mRNA Templates Having Intact 5' Ends

For the mRNAs joined to oligonucleotide tags using both the chemical and enzymatic methods, first strand cDNA synthesis was performed using reverse transcriptase with random nonamers as primers. In order to protect internal EcoRI sites in the cDNA from digestion at later steps in the procedure, methylated dCTP was used for first strand synthesis. After removal of RNA by an alkaline hydrolysis, the first strand of cONA was precipitated using isopropanol in order to eliminate residual primers.

For both the chemical and the enzymatic methods, the second strand of the cDNA was synthesized with a Klenow fragment using a primer corresponding to the 5'end of the ligated oligonucleotide described in Example 12. Preferably, the primer is 20-25 bases in length. Methylated dCTP was also used for second strand synthesis in order to protect internal EcoRI sites in the cDNA from digestion during the cloning process.

Following cDNA synthesis, the cDNAs were cloned into pBlueScript as described in Example 15 below.

### EXAMPLE 15

### Insertion of cDNAs into BlueScriDt

Following second strand synthesis, the ends of the cDNA were blunted with T4 DNA polymerase (Biolabs) and the cDNA was digested with EcoRI. Since methylated dCTP was used during cDNA synthesis, the EcoRI site present in the tag was the only site which was hemi-methylated. Consequently, only the EcoRI site in the oligonucleotide tag was susceptible to EcoRI digestion. The cONA was then size fractionated using exclusion chromatography (AcA, Biosepra). Fractions corresponding to cDNAs of more than 150 bp were pooled and ethanol precipitated. The cDNA was directionally cloned into the Smal and EcoRI ends of the phagemid pBlueScript vector (Stratagene). The ligation mixture was electroporated into bacteria and propagated under appropriate antibiotic selection. Clones containing the oligonucleotide tag attached were selected as described in Example 16 below.

### EXAMPLE 16

### Selection of Clones Having the Oligonucleotide Tag Attached Thereto

The plasmid ONAs containing 5' EST libraries made as described above were purified (Qiagen). A positive selection of the tagged clones was performed as follows. Briefly, in this selection procedure, the plasmid DNA was converted to single stranded DNA using gene II endonuclease of the phage F1 in combination with an exonuclease (Chang et al., Gene 127:95-8, 1993) such as exonuclease III or T7 gene 6 exonuclease. The resulting single stranded DNA was then purified using paramagnetic beads as described by Fry et al, Biotechniques, 13: 124-131, 1992. In this procedure, the single stranded DNA was hybridized with a biotinylated oligonucleotide having a sequence corresponding to the 3' end of the oligonucleotide described in Example 13. Preferably, the primer has a length of 20-25 bases. Clones including a sequence complementary to the biotinylated oligonucleotide were captured by incubation with streptavidin coated magnetic beads followed by magnetic selection. After capture of the positive clones, the plasmid DNA was released from the magnetic beads and converted into double stranded DNA using a DNA polymerase such as the ThermoSequenase obtained from Amersham Pharmacia Biotech. Alternatively, protocols such as the Gene Trapper kit (Gibco BRL) may be used. The double stranded DNA was then electroporated into bacteria. The percentage of positive clones having the 5' tag oligonucleotide was estimated to typically rank between 90 and 98% using dot blot analysis.

Following electroporation, the libraries were ordered in 384 microtiter plates (MTP). A copy of the MTP was stored for future needs. Then the libraries were transferred into 96 MTP and sequenced as described below.

### EXAMPLE 17

### Sequencing of Inserts in Selected Clones

Plasmid inserts were first amplified by PCR on PE 9600 thermocyclers (Perkin-Elmer), using standard SETA-A and SETA-B primers (Genset SA), AmpliTaqGold (Perkin-Elmer), dNTPs (Boehringer), buffer and cycling conditions as recommended by the Perkin-Elmer Corporation.

PCR products were then sequenced using automatic ABI Prism 377 sequencers (Perkin Elmer, Applied Biosystems Division, Foster City, CA). Sequencing reactions were performed using PE 9600 thermocyclers (Perkin Elmer) with standard dye-primer chemistry and ThermoSequenase (Amersham Life Science). The primers used were either T7 or 21M13 (available from Genset SA) as appropriate. The primers were labeled with the JOE, FAM, ROX and TAMRA dyes. The dNTPs and ddNTPs used in the sequencing reactions were purchased from Boehringer. Sequencing buffer, reagent concentrations and cycling conditions were as recommended by Amersham.

Following the sequencing reaction, the samples were precipitated with EtOH, resuspended in formamide loading buffer, and loaded on a standard 4% acrylamide gel. Electrophoresis was performed for 2.5 hours at 3000V on an ABI 377 sequencer, and the sequence data were collected and analyzed using the ABI Prism DNA Sequencing Analysis Software, version 2.1.2.

The sequence data from the 44 cDNA libraries made as described above were transferred to a proprietary database, where quality control and validation steps were performed. A proprietary base-caller ("Trace"), working using a Unix system automatically flagged suspect peaks, taking into account the shape of the peaks, the inter-peak resolution, and the noise level. The proprietary base-caller also performed an automatic trimming. Any stretch of 25 or fewer bases having more than 4 suspect peaks was considered unreliable and was discarded. Sequences corresponding to cloning vector or ligation oligonucleotides were automatically removed from the EST sequences. However, the resulting EST sequences may contain 1 to 5 bases belonging to the above mentioned sequences at their 5' end. If needed, these can easily be removed on a case by case basis.

Thereafter, the sequences were transferred to the proprietary NETGENE™ Database for further analysis as described below.

Following sequencing as described above, the sequences of the 5' ESTs were entered in a proprietary database called NETGENE™ for storage and manipulation. It will be appreciated by those skilled in the art that the data could be stored and manipulated on any medium which can be read and accessed by a computer. Computer readable media include magnetically readable media, optically readable media, or electronically readable media. For example, the computer readable media may be a hard disc, a floppy disc, a magnetic tape, CD-ROM, RAM, or ROM as well as other types of other media known to those skilled in the art.

In addition, the sequence data may be stored and manipulated in a variety of data processor programs in a variety of formats. For example, the sequence data may be stored as text in a word processing file, such as MicrosoftWORD or WORDPERFECT or as an ASCII file in a variety of database programs familiar to those of skill in the art, such as DB2, SYBASE, or ORACLE.

The computer readable media on which the sequence information is stored may be in a personal computer, a network, a server or other computer systems known to those skilled in the art. The computer or other system preferably includes the storage media described above, and a processor for accessing and manipulating the sequence data.

Once the sequence data has been stored it may be manipulated and searched to locate those stored sequences which contain a desired nucleic acid sequence or which encode a protein having a particular functional domain. For example, the stored sequence information may be compared to other known sequences to identify homologies, motifs implicated in biological function, or structural motifs.

Programs which may be used to search or compare the stored sequences include the MacPattern (EMBL), BLAST, and BLAST2 program series (NCBI), basic local alignment search tool programs for nucleotide (BLASTN) and peptide (BLASTX) comparisons (Altschul et al, J. Mol. Biol. 215: 403 (1990)) and FASTA (Pearson and Lipman, Proc. Natl. Acad. Sci. USA, 85: 2444 (1988)). The BLAST programs then extend the alignments on the basis of defined match and mismatch criteria.

Motifs which may be detected using the above programs include sequences encoding leucine zippers, helix-turn-helix motifs, glycosylation sites, ubiquitination sites, alpha helices, and beta sheets, signal sequences encoding signal peptides which direct the secretion of the encoded proteins, sequences implicated in transcription regulation such as homeoboxes, acidic stretches, enzymatic active sites, substrate binding sites, and enzymatic cleavage sites.

Before searching the cDNAs in the NETGENE™ database for sequence motifs of interest, cDNAs derived from mRNAs which were not of interest were identified and eliminated from further consideration as described in Example 18 below.

### EXAMPLE 18

### Elimination of Undesired Sequences from Further Consideration

5' ESTs in the NETGENE™ database which were derived from undesired sequences such as transfer RNAs, ribosomal RNAs, mitochondrial RNAs, procaryotic RNAs, fungal RNAs, Alu sequences, LI sequences, or repeat sequences were identified using the FASTA and BLASTN programs with the parameters listed in Table II.

To eliminate 5' ESTs encoding tRNAs from further consideration, the 5' EST sequences were compared to the sequences of 1190 known tRNAs obtained from EMBL release 38, of which 100 were human. The comparison was performed using FASTA on both strands of the 5' ESTs. Sequences having more than 80% homology over more than 60 nucleotides were identified as tRNA. Of the 144,341 sequences screened, 26 were identified as tRNAs and eliminated from further consideration.

To eliminate 5' ESTs encoding rRNAs from further consideration, the 5' EST sequences were compared to the sequences of 2497 known rRNAs obtained from EMBL release 38, of which 73 were human. The comparison was performed using BLASTN on both strands of the 5' ESTs with the parameter S-108. Sequences having more than 80% homology over stretches longer than 40 nucleotides were identified as rRNAs. Of the 144,341 sequences screened, 3,312 were identified as rRNAs and eliminated from further consideration.

To eliminate 5' ESTs encoding mtRNAs from further consideration, the 5' EST sequences were compared to the sequences of the two known mitochondrial genomes for which the entire genomic sequences are available and all sequences transcribed from these mitochondrial genomes including tRNAs, rRNAs, and mRNAs for a total of 38 sequences. The comparison was performed using BLASTN on both strands of the 5' ESTs with the parameter S = 108. Sequences having more than 80% homology over stretches longer than 40 nucleotides were identified as mtRNAs. Of the 144,341 sequences screened, 6,110 were identified as mtRNAs and eliminated from further consideration.

Sequences which might have resulted from exogenous contaminants were eliminated from further consideration by comparing the 5' EST sequences to release 46 of the EMBL bacterial and fungal divisions using BLASTN with the parameter S-144. All sequences having more than 90% homology over at least 40 nucleotides were identified as exogenous contaminants. Of the 42 cDNA libraries examined, the average percentages of procaryotic and fungal sequences contained therein were 0.2% and 0.5% respectively. Among these sequences, only one could be identified as a sequence specific to fungi. The others were either fungal or procaryotic sequences having homologies with vertebrate sequences or including repeat sequences which had not been masked during the electronic comparison.

In addition, the 5' ESTs were compared to 6093 Alu sequences and 1115 L1 sequences to mask 5' ESTs containing such repeat sequences from further consideration. 5' ESTs including THE and MER repeats, SSTR sequences or satellite, micro-satellite, or telomeric repeats were also eliminated from further consideration. On average, 11.5% of the sequences in the libraries contained repeat sequences. Of this 11.5%, 7% contained Alu repeals, 3.3% contained L1 repeats and the remaining 1.2% were derived from the other types of repetitive sequences which were screened. These percentages are consistent with those found in cDNA libraries prepared by other groups. For example, the cDNA libraries of Adams et al. contained between 0% and 7.4% Alu repeats depending on the source of the RNA which was used to prepare the cDNA library (Adams et al., Nature 377:174, 1996).

The sequences of those 5' ESTs remaining after the elimination of undesirable sequences were compared with the sequences of known human mRNAs to determine the accuracy of the sequencing procedures described above.

### EXAMPLE 19

### Measurement of Sequencing Accuracy by Comparison to Known Sequences

To further determine the accuracy of the sequencing procedure described above, the sequences of 5' ESTs derived from known sequences were identified and compared to the known sequences. First, a FASTA analysis with overhangs shorter than 5 bp on both ends was conducted on the 5' ESTs to identify those matching an entry in the public human mRNA database. The 6655 5' ESTs which matched a known human mRNA were then realigned with their cognate mRNA and dynamic programming was used to include substitutions, insertions, and deletions in the list of "errors" which would be recognized. Errors occurring in the last 10 bases of the 5' EST sequences were ignored to avoid the inclusion of spurious cloning sites in the analysis of sequencing accuracy.

This analysis revealed that the sequences incorporated in the NETGENE™ database had an accuracy of more than 99.5%.

To determine the efficiency with which the above selection procedures select cDNAs which include the 5' ends of their corresponding mRNAs, the following analysis was performed.

### EXAMPLE 20

### Determination of Efficiency of 5' EST Selection

To determine the efficiency at which the above selection procedures isolated 5' ESTs which included sequences close to the 5' end of the mRNAs from which they were derived, the sequences of the ends of the 5' ESTs which were derived from the elongation factor 1 subunit a and ferritin heavy chain genes were compared to the known cDNA sequences for these genes. Since the transcription start sites for the elongation factor 1 subunit α and ferritin heavy chain are well characterized, they may be used to determine the percentage of 5' ESTs derived from these genes which included the authentic transcription start sites.

For both genes, more than 95% of the cDNAs included sequences close to or upstream of the 5' end of the corresponding mRNAs.

To extend the analysis of the reliability of the procedures for isolating 5' ESTs from ESTs in the NETGENE™ database, a similar analysis was conducted using a database composed of human mRNA sequences extracted from GenBank database release 97 for comparison. For those 5' ESTs derived from mRNAs included in the GeneBank database, more than 85% had their 5' ends close to the 5' ends of the known sequence. As some of the mRNA sequences available in the GenBank database are deduced from genomic sequences, a 5' end matching with these sequences will be counted as an internal match. Thus, the method used here underestimates the yield of ESTs including the authentic 5' ends of their corresponding mRNAs.

The EST libraries made above included multiple 5' ESTs derived from the same mRNA. The sequences of such 5' ESTs were compared to one another and the longest 5' ESTs for each mRNA were identified. Overlapping cDNAs were assembled into continuous sequences (contigs). The resulting continuous sequences were then compared to public databases to gauge their similarity to known sequences, as described in Example 21 below.

### EXAMPLE 21

### Clustering of the 5' ESTs and Calculation of Novelty Indices for cDNA Libraries

For each sequenced EST library, the sequences were clustered by the 5' end. Each sequence in the library was compared to the others with BLASTN2 (direct strand, parameters S = 107). ESTs with High Scoring Segment Pairs (HSPs) at least 25 bp long, having 95% identical bases and beginning closer than 10 bp from each EST 5' end were grouped. The longest sequence found in the cluster was used as representative of the cluster. A global clustering between libraries was then performed leading to the definition of super-contigs.

To assess the yield of new sequences within the EST libraries, a novelty rate (NR) was defined as: NR = 100 X (Number of new unique sequences found in the library/Total number of sequences from the library). Typically, novelty rating range between 10% and 41 % depending on the tissue from which the EST library was obtained. For most of the libraries, the random sequencing of 5' EST libraries was pursued until the novelty rate reached 20%.

Following characterization as described above, the collection of 5' ESTs in NETGENE™ was screened to identify those 5' ESTs bearing potential signal sequences as described in Example 22 below.

### EXAMPLE 22

### Identification of Potential Signal Sequences in 5' ESTs

The 5' ESTs in the NETGENE™ database were screened to identify those having an uninterrupted open reading frame (ORF) longer than 45 nucleotides beginning with an ATG codon and extending to the end of the EST. Approximately half of the cDNA sequences in NETGENE™ contained such an ORF. The ORFs of these 5' ESTs were searched to identify potential signal motifs using slight modifications of the procedures disclosed in Von Heijne, G. A New Method for Predicting Signal Sequence Cleavage Sites. Nucleic Acids Res. 14:4683-4690 (1986). Those 5' EST sequences encoding a 15 amino acid long stretch with a score of at least 3.5 in the Von Heijne signal peptide identification matrix were considered to possess a signal sequence. Those 5' ESTs which matched a known human mRNA or EST sequence and had a 5' end more than 20 nucleotides downstream of the known 5' end were excluded from further analysis. The remaining cDNAs having signal sequences therein were included in a database called SIGNALTAG™.

To confirm the accuracy of the above method for identifying signal sequences, the analysis of Example 23 was performed.

### EXAMPLE 23

### Confirmation of Accuracy of Identification of Potential Signal Sequences in 5' ESTs

The accuracy of the above procedure for identifying signal sequences encoding signal peptides was evaluated by applying the method to the 43 amino terminal amino acids of all human SwissProt proteins. The computed Von Heijne score for each protein was compared with the known characterization of the protein as being a secreted protein or a non-secreted protein. In this manner, the number of non-secreted proteins having a score higher than 3.5 (false positives) and the number of secreted proteins having a score lower than 3.5 (false negatives) could be calculated.

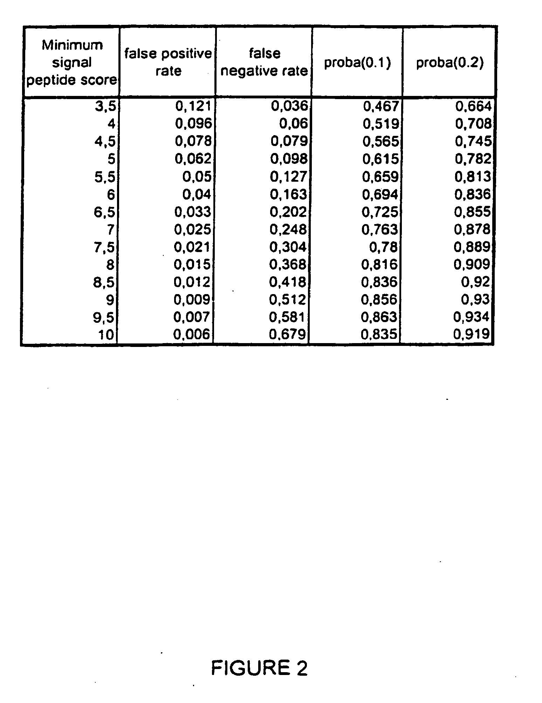

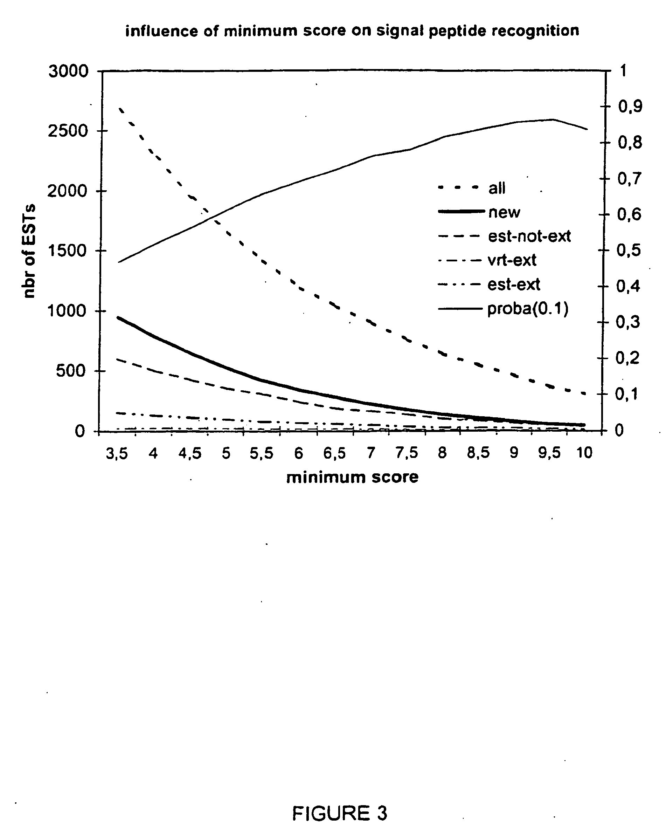

Using the results of the above analysis, the probability that a peptide encoded by the 5' region of the mRNA is in fact a genuine signal peptide based on its Von Heijne's score was calculated based on either the assumption that 10% of human proteins are secreted or the assumption that 20% of human proteins are secreted. The results of this analysis are shown in Figures 2 and 3.

Using the above method of identifying secretory proteins, 5' ESTs for human glucagon, gamma interferon induced monokine precursor, secreted cyclophilin-like protein, human pleiotropin, and human biotinidase precursor all of which are polypeptides which are known to be secreted, were obtained. Thus, the above method successfully identified those 5' ESTs which encode a signal peptide.

To confirm that the signal peptide encoded by the 5' ESTs actually functions as a signal peptide, the signal sequences from the 5' ESTs may be cloned into a vector designed for the identification of signal peptides. Some signal peptide identification vectors are designed to confer the ability to grow in selective medium on host cells which have a signal sequence operably inserted into the vector. For example, to confirm that a 5' EST encodes a genuine signal peptide, the signal sequence of the 5' EST may be inserted upstream and in frame with a non secreted form of the yeast invertase gene in signal peptide selection vectors such as those described in U.S. Patent No. 5,536,637. Growth of host cells containing signal sequence selection vectors having the signal sequence from the 5' EST inserted therein confirms that the 5' EST encodes a genuine signal peptide.

Alternatively, the presence of a signal peptide may be confirmed by cloning the extended cDNAs obtained using the ESTs into expression vectors such as pXT1 (as described below), or by constructing promoter-signal sequence reporter gene vectors which encode fusion proteins between the signal peptide and an assayable reporter protein. After introduction of these vectors into a suitable host cell, such as COS cells or NIH 3T3 cells, the growth medium may be harvested and analyzed for the presence of the secreted protein. The medium from these cells is compared to the medium from cells containing vectors lacking the signal sequence or extended cDNA insert to identify vectors which encode a functional signal peptide or an authentic secreted protein.

Those 5' ESTs which encoded a signal peptide, as determined by the method of Example 22 above, were further grouped into four categories based on their homology to known sequences. The categorization of the 5' ESTs is described in Example 24 below.

### EXAMPLE 24

### Categorization of 5' ESTs Encoding a Signal Peptide

Those 5' ESTs having a sequence not matching any known vertebrate sequence not any publicly available EST sequence were designated "new." Of the sequences in the SIGNALTAG™ database, 947 of the 5' ESTs having a Von Heijne's score of at least 3.5 fell into this category.

Those 5' ESTs having a sequence not matching any vertebrate sequence but matching a publicly known EST were designated "EST-ext", provided that the known EST sequence was extended by at least 40 nucleotides in the 5' direction. Of the sequences in the SIGNALTAG™ database, 150 of the 5' ESTs having a Von Heijne's score of at least 3.5 fell into this category.

Those ESTs not matching any vertebrate sequence but matching a publicly known EST without extending the known EST by at least 40 nucleotides in the 5' direction were designated "EST." Of the sequences in the SIGNALTAG™ database, 599 of the 5' ESTs having a Von Heijne's score of at least 3.5 fell into this category.

Those 5' ESTs matching a human mRNA sequence but extending the known sequence by at least 40 nucleotides in the 5' direction were designated "VERT-ext." Of the sequences in the SIGNALTAG™ database, 23 of the 5' ESTs having a Von Heijne's score of at least 3.5 fell into this category, Included in this category was a 5' EST which extended the known sequence of the human translocase mRNA by more than 200 bases in the 5' direction. A 5' EST which extended the sequence of a human tumor suppressor gene in the 5' direction was also identified.

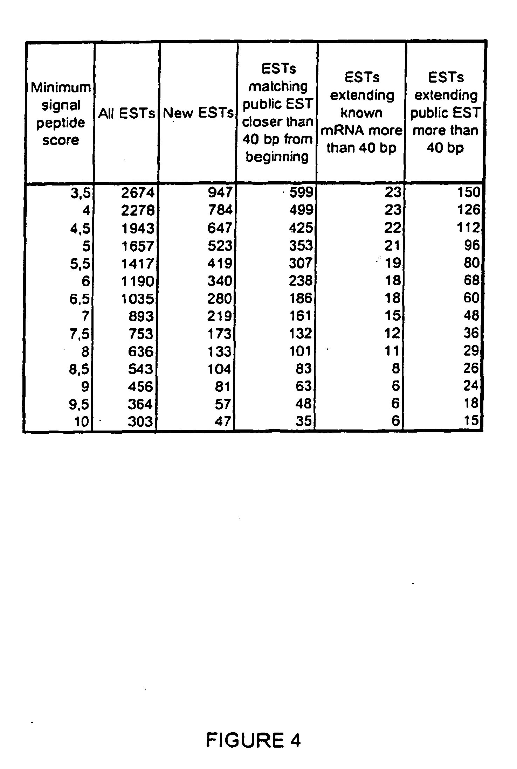

Figure 4 shows the distribution of 5' ESTs in each category and the number of 5' ESTs in each category having a given minimum von Heijne's score.

Each of the 5' ESTs was categorized based on the tissue from which its corresponding mRNA was obtained, as described below in Example 25.

### EXAMPLE 25

### Categorization of Expression Patterns

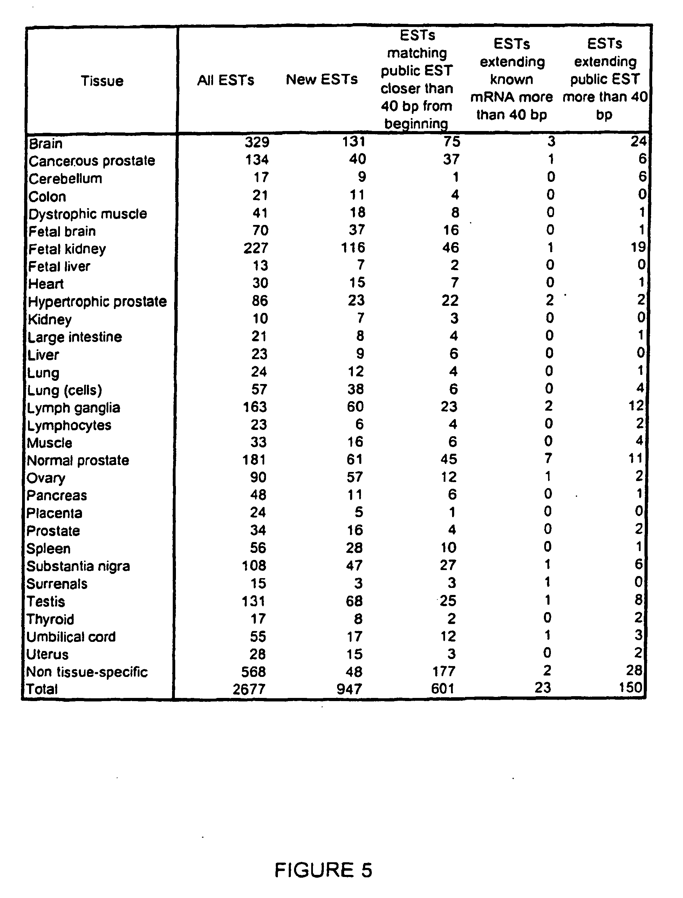

Figure 5 shows the tissues from which the mRNAs corresponding to the 5' ESTs in each of the above described categories were obtained.

In addition to categorizing the 5' ESTs by the tissue from which the cDNA library in which they were first identified was obtained, the spatial and temporal expression patterns of the mRNAs corresponding to the 5' ESTs, as well as their expression levels, may be determined as described in Example 26 below. Characterization of the spatial and temporal expression patterns and expression levels of these mRNAs is useful for constructing expression vectors capable of producing a desired level of gene product in a desired spatial or temporal manner, as will be discussed in more detail below.

In addition, 5' ESTs whose corresponding mRNAs are associated with disease states may also be identified. For example, a particular disease may result from lack of expression, over expression, or under expression of an mRNA corresponding to a 5' EST. By comparing mRNA expression patterns and quantities in samples taken from healthy individuals with those from individuals suffering from a particular disease. 5' ESTs responsible for the disease may be identified.

It will be appreciated that the results of the above characterization procedures for 5' ESTs also apply to extended cDNAs (obtainable as described below) which contain sequences adjacent to the 5' ESTs. It will also be appreciated that if it is desired to defer characterization until extended cDNAs have been obtained rather than characterizing the ESTs themselves, the above characterization procedures can be applied to characterize the extended cONAs after their isolation.

### EXAMPLE 26

### Evaluation of Expression levels and Patterns of mRNAs Corresponding to 5' ESTs or Extended cONAs

Expression levels and patterns of mRNAs corresponding to 5' ESTs or extended cDNAs (obtainable as described below) may be analyzed by solution hybridization with long probes as described in International Patent Application No. WO 97/05277. Briefly, a 5' EST, extended cDNA, or fragment thereof corresponding to the gene encoding the mRNA to be characterized is inserted at a cloning site immediately downstream of a bacteriophage (T3, T7 or SP6) RNA polymerase promoter to produce antisense RNA. Preferably, the 5' EST or extended cDNA has 100 or more nucleotides. The plasmid is linearized and transcribed in the presence of ribonucleotides comprising modified ribonucleotides (i.e. biotin-UTP and DIG-UTP). An excess of this doubly labeled RNA is hybridized in solution with mRNA isolated from cells or tissues of interest. The hybridizations are performed under standard stringent conditions (40-50°C for 16 hours in an 80% formamide, 0.4 M NaCl buffer, pH 7-8). The unhybridized probe is removed by digestion with ribonucleases specific for single-stranded RNA (i.e. RNases CL3, T1, Phy M, U2 or A). The presence of the biotin-UTP modification enables capture of the hybrid on a microtitration plate coated with streptavidin. The presence of the DIG modification enables the hybrid to be detected and quantified by ELISA using an anti-DIG antibody coupled to alkaline phosphatase.

The 5' ESTs, extended cDNAs, or fragments thereof may also be tagged with nucleotide sequences for the serial analysis of gene expression (SAGE) as disclosed in UK Patent Application No. 2 305 241 A. In this method, cDNAs are prepared from a cell, tissue, organism or other source of nucleic acid for which it is desired to determine gene expression patterns. The resulting cDNAs are separated into two pools. The cDNAs in each pool are cleaved with a first restriction endonuclease, called an "anchoring enzyme," having a recognition site which is likely to be present at least once in most cDNAs. The fragments which contain the 5' or 3' most region of the cleaved cDNA are isolated by binding to a capture medium such as streptavidin coated beads. A first oligonucleotide linker having a first sequence for hybridization of an amplification primer and an internal restriction site for a "tagging endonuclease" is ligated to the digested cDNAs in the first pool. Digestion with the second endonuclease produces short "tag" fragments from the cDNAs.

A second oligonucleotide having a second sequence for hybridization of an amplification primer and an internal restriction site is ligated to the digested cDNAs in the second pool. The cDNA fragments in the second pool are also digested with the "tagging endonuclease" to generate short "tag" fragments derived from the cDNAs in the second pool. The "tags" resulting from digestion of the first and second pools with the anchoring enzyme and the tagging endonuclease are ligated to one another to produce "ditags." In some embodiments, the ditags are concatamerized to produce ligation products containing from 2 to 200 ditags. The tag sequences are then determined and compared to the sequences of the 5' ESTs or extended cDNAs to determine which 5' ESTs or extended cDNAs are expressed in the cell, tissue, organism, or other source of nucleic acids from which the tags were derived. In this way, the expression pattern of the 5' ESTs or extended cDNAs in the cell, tissue, organism, or other source of nucleic acids is obtained.

Quantitative analysis of gene expression may also be performed using arrays. As used herein, the term array means a one dimensional, two dimensional, or multidimensional arrangement of full length cDNAs (i.e. extended cDNAs which include the coding sequence for the signal peptide, the coding sequence for the mature protein, and a stop codon), extended cDNAs, 5' ESTs or fragments of the full length cDNAs, extended cDNAs, or 5' ESTs of sufficient length to permit specific detection of gene expression. Preferably, the fragments are at least 15 nucleotides in length. More preferably, the fragments are at least 100 nucleotides in length. More preferably, the fragments are more than 100 nucleotides in length. In some embodiments the fragments may be more than 500 nucleotides in length.

For example, quantitative analysis of gene expression may be performed with full length cDNAs, extended cDNAs, 5' ESTs, or fragments thereof in a complementary DNA microarray as described by Schena et al. (*Science* **270**:467-470, 1995; *Proc. Natl. Acad. Sci. U.S.A*. **93**:10614-10619, 1996). Full length cDNAs, extended cDNAs, 5' ESTs or fragments thereof are amplified by PCR and arrayed from 96-well microtiter plates onto silylated microscope slides using high-speed robotics. Printed arrays are incubated in a humid chamber to allow rehydration of the array elements and rinsed, once in 0.2% SDS for 1 min, twice in water for 1 min and once for 5 min in sodium borohydride solution. The arrays are submerged in water for 2 min at 95°C, transferred into 0.2% SDS for 1 min, rinsed twice with water, air dried and stored in the dark at 25°C.

Cell or tissue mRNA is isolated or commercially obtained and probes are prepared by a single round of reverse transcription. Probes are hybridized to 1 cm² microarrays under a 14 x 14 mm glass coverslip for 6-12 hours at 60°C. Arrays are washed for 5 min at 25°C in low stringency wash buffer (1 x x SSC/0.2% SDS), then for 10 min at room temperature in high stringency wash buffer (0.1 x SSC/0.2% SDS). Arrays are scanned in 0.1 x SSC using a fluorescence laser scanning device fitted with a custom filter set. Accurate differential expression measurements are obtained by taking the average of the ratios of two independent hybridizations.

Quantitative analysis of the expression of genes may also be performed with full length cDNAs, extended cDNAs, 5' ESTs, or fragments thereof in complementary DNA arrays as described by Pietu et al. (Genome Research 6:492-503, 1996). The full length cDNAs, extended cDNAs, 5' ESTs or fragments thereof are PCR amplified and spotted on membranes. Then, mRNAs originating from various tissues or cells are labeled with radioactive nucleotides. After hybridization and washing in controlled conditions, the hybridized mRNAs are detected by phospho-imaging or autoradiography. Duplicate experiments are performed and a quantitative analysis of differentially expressed mRNAs is then performed.

Alternatively, expression analysis of the 5' ESTs or extended cDNAs can be done through high density nucleotide arrays as described by Lockhart et al. (Nature Biotechnology 14: 1675-1680, 1996) and Sosnowsky et al (Proc. Natl. Acad. Sci. 94:1119-1123, 19971. Oligonucleotides of 15-50 nucleotides corresponding to sequences of the 5' ESTs or extended cDNAs are synthesized directly on the chip (Lockhart et al.. *supra*) or synthesized and then addressed to the chip (Sosnowski et al., *supra*). Preferably, the oligonucleotides are about 20 nucleotides in length.

cDNA probes labeled with an appropriate compound, such as biotin, digoxigenin or fluorescent dye, are synthesized from the appropriate mRNA population and then randomly fragmented to an average size of 50 to 100 nucleotides. The said probes are then hybridized to the chip. After washing as described in Lockhart et al., *supra* and application of different electric fields (Sosnowsky et al., Proc. Natl. Acad. Sci. 94:119-1123), the dyes or labeling compounds are detected and quantified. Duplicate hybridizations are performed. Comparative analysis of the intensity of the signal originating from cDNA probes on the same target oligonucleotide in different cDNA samples indicates a differential expression of the mRNA corresponding to the 5' EST or extended cDNA from which the oligonucleotide sequence has been designed.

### III. Use of 5' ESTs to Clone Extended cDNAs and to Clone the Corresponding Genomic DNAs

Once 5' ESTs which include the 5' end of the corresponding mRNAs have been selected using the procedures described above, they can be utilized to isolate extended cDNAs which contain sequences adjacent to the 5' ESTs. The extended cDNAs may include the entire coding sequence of the protein encoded by the corresponding mRNA, including the authentic translation start site, the signal sequence, and the sequence encoding the mature protein remaining after cleavage of the signal peptide. Such extended cDNAs are referred to herein as "full length cDNAs." Alternatively, the extended cDNAs may include only the sequence encoding the mature protein remaining after cleavage of the signal peptide, or only the sequence encoding the signal peptide.

Example 27 below describes a general method for obtaining extended cDNAs. Example 28 below describes the cloning and sequencing of several extended cDNAs, including extended cDNAs which include the entire coding sequence and authentic 5' end of the corresponding mRNA for several secreted proteins.

The methods of Examples 27, 28, and 29 can also be used to obtain extended cDNAs which encode less than the entire coding sequence of the secreted proteins encoded by the genes corresponding to the 5' ESTs. In some aspects, the extended cDNAs isolated using these methods encode at least 10 amino acids of the protein encoded by the sequence of SEO ID NO: 45. In further aspects, the extended cDNAs encode at least 20 amino acids of the protein encoded by the sequence of SEO ID NOs: 45. In further aspects, the extended cDNAs encode at least 30 amino amino acids of the sequence of SEQ ID NOs: 45 In a preferred embodiment, the extended cDNAs encode a full length protein sequence, which includes the protein coding sequence of SEQ ID NO: 45.

### EXAMPLE 27

### General Method for Using 5' ESTs to Clone and Sequence Extended cDNAs

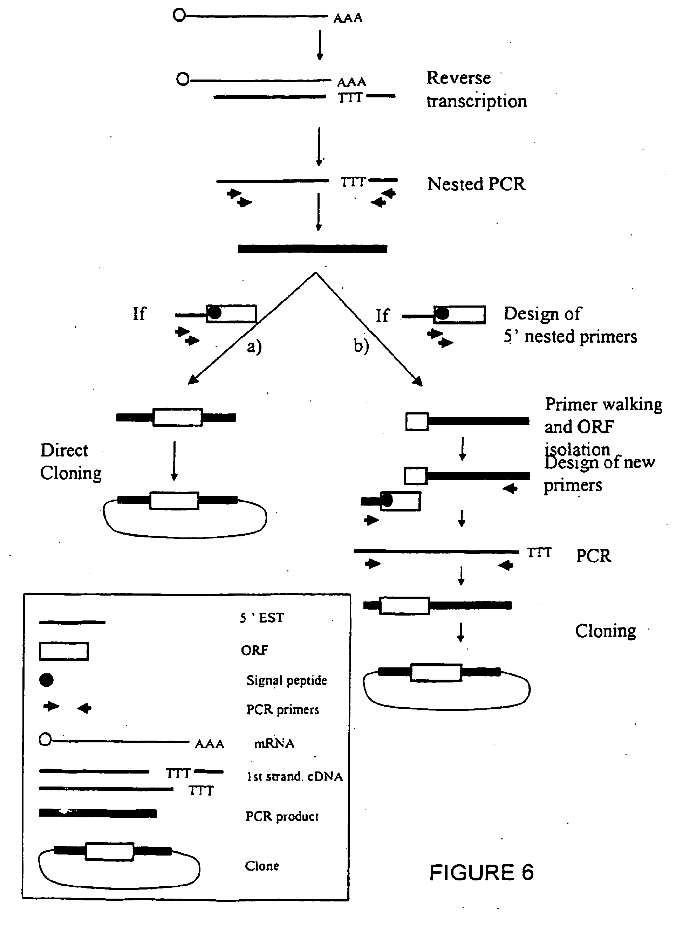

The following general method has been used to quickly and efficiently isolate extended cDNAs including sequence adjacent to the sequences of the 5' ESTs used to obtain them. This method may be applied to obtain extended cDNAs for any 5' EST in the NETGENE™ database, including those 5' ESTs encoding secreted proteins. The method is summarized in Figure 6.

### 1. Obtaining Extended cDNAs

### a) First strand synthesis

The method takes advantage of the known 5' sequence of the mRNA. A reverse transcription reaction is conducted on purified mRNA with a poly 14dT primer containing a 49 nucleotide sequence at its 5' end allowing the addition of a known sequence at the end of the cDNA which corresponds to the 3' end of the mRNA. For example, the primer may have the following sequence: 5'-ATC GTT GAG ACT CGT ACC AGC AGA GTC ACG AGA GAG ACT ACA CGG TAC TGG TTT TTT TTT TTT TTVN -3' (SEQ ID NO:14). Those skilled in the art will appreciate that other sequences may also be added to the poly dT sequence and used to prime the first strand synthesis. Using this primer and a reverse transcriptase such as the Superscript II (Gibco BRL) or Rnase H Minus M-MLV (Promega) enzyme, a reverse transcript anchored at the 3' polyA site of the RNAs is generated.

After removal of the mRNA hybridized to the first cDNA strand by alkaline hydrolysis, the products of the alkaline hydrolysis and the residual poly dT primer are eliminated with an exclusion column such as an AcA34 (Biosepra) matrix as explained in Example 11.

### b) Second strand synthesis

A pair of nested primers on each end is designed based on the known 5' sequence from the 5' EST and the known 3' end added by the poly dT primer used in the first strand synthesis. Software used to design primers are either based on GC content and melting temperatures of oligonucleotides, such as OSP (Illier and Green, PCA Meth. Appl. 1:124.128. 1991), or based on the octamer frequency disparity method (Griffais et al., Nucleic Acids Res. 19: 3887-3891, 1991 such as PC-Rare (http://bioinformatics.weizmann.ac.il/soflware/PC.Rare/doc/manuel.html).

Preferably, the nested primers at the 5' end are separated from one another by four to nine bases. The 5' primer sequences may be selected to have melting temperatures and specificities suitable for use in PCR.

Preferably, the nested primers at the 3' end are separated from one another by four to nine bases. For example, the nested 3' primers may have the following sequences: (5'- CCA GCA GAG TCA CGA GAG AGA CTA CAC GG -3'(SEQ ID NO:15), and 5'- CAC GAG AGA GAC TAC ACG GTA CTG G -3' (SEO ID NO:16). These primers were selected because they have melting temperatures and specificities compatible with their use in PCR. However, those skilled in the art will appreciate that other sequences may also be used as primers.

The first PCR run of 25 cycles is performed using the Advantage Tth Polymerase Mix (Clontech) and the outer primer from each of the nested pairs. A second 20 cycle PCR using the same enzyme and the inner primer from each of the nested pairs is then performed on 1/2500 of the first PCR product. Thereafter, the primers and nucleotides are removed.

### 2. Sequencing of Full Length Extended cDNAs or Fragments Thereof

Due to the lack of position constraints on the design of 5' nested primers compatible for PCR use using the OSP software, amplicons of two types are obtained. Preferably, the second 5' primer is located upstream of the translation initiation codon thus yielding a nested PCR product containing the whole coding sequence. Such a full length extended cDNA undergoes a direct cloning procedure as described in section a below. However, in some cases, the second 5' primer is located downstream of the translation initiation codon, thereby yielding a PCR product containing only part of the ORF. Such incomplete PCR products are submitted to a modified procedure described in section b below.

### a) Nested PCR products containing complete ORFs

When the resulting nested PCR product contains the complete coding sequence, as predicted from the 5'EST sequence, it is cloned in an appropriate vector such as pED6dpc2, as described in section 3.

### b) Nested PCR products containing incomplete ORFs

When the amplicon does not contain the complete coding sequence, intermediate steps are necessary to obtain both the complete coding sequence and a PCR product containing the full coding sequence. The complete coding sequence can be assembled from several partial sequences determined directly from different PCR products as described in the following section.

Once the full coding sequence has been completely determined, new primers compatible for PCR use are designed to obtain amplicons containing the whole coding region. However, in such cases, 3' primers compatible for PCR use are located inside the 3' UTR of the corresponding mRNA, thus yielding amplicons which lack part of this region, i.e. the polyA tract and sometimes the polyadenylation signal, as illustrated in figure 6. Such full length extended cDNAs are then cloned into an appropriate vector as described in section 3.

### c) Sequencing extended cDNAs

Sequencing of extended cDNAs is performed using a Die Terminator approach with the AmpliTaq DNA polymerase FS kit available from Perkin Elmer.

In order to sequence PCR fragments, primer walking is performed using software such as OSP to choose primers and automated computer software such as ASMG (Sutton et al., Genome Science Technol. 1: 9.19, 1995) to construct contigs of walking sequences including the initial 5' tag using minimum overlaps of 32 nucleotides. Preferably, primer walking is performed until the sequences of full length cDNAs are obtained.

Completion of the sequencing of a given extended cDNA fragment is assessed as follows. Since sequences located after a polyA tract are difficult to determine precisely in the case of uncloned products, sequencing and primer walking processes for PCR products are interrupted when a polyA tract is identified in extended cDNAs obtained as described in case b. The sequence length is compared to the size of the nested PCR product obtained as described above. Due to the limited accuracy of the determination of the PC R product size by gel electrophoresis, a sequence is considered complete if the size of the obtained sequence is at least 70 % the size of the first nested PCR product. If the length of the sequence determined from the computer analysis is not at least 70% of the length of the nested PCR product, these PCR products are cloned and the sequence of the insertion is determined. When Northern blot data are available, the size of the mRNA detected for a given PCR product is used to finally assess that the sequence is complete. Sequences which do not fulfill the above criteria are discarded and will undergo a new isolation procedure.

Sequence data of all extended cDNAs are then transferred to a proprietary database, where quality controls and validation steps are carried out as described in example 15.

### 3. Cloning of Full Length Extended cDNAs

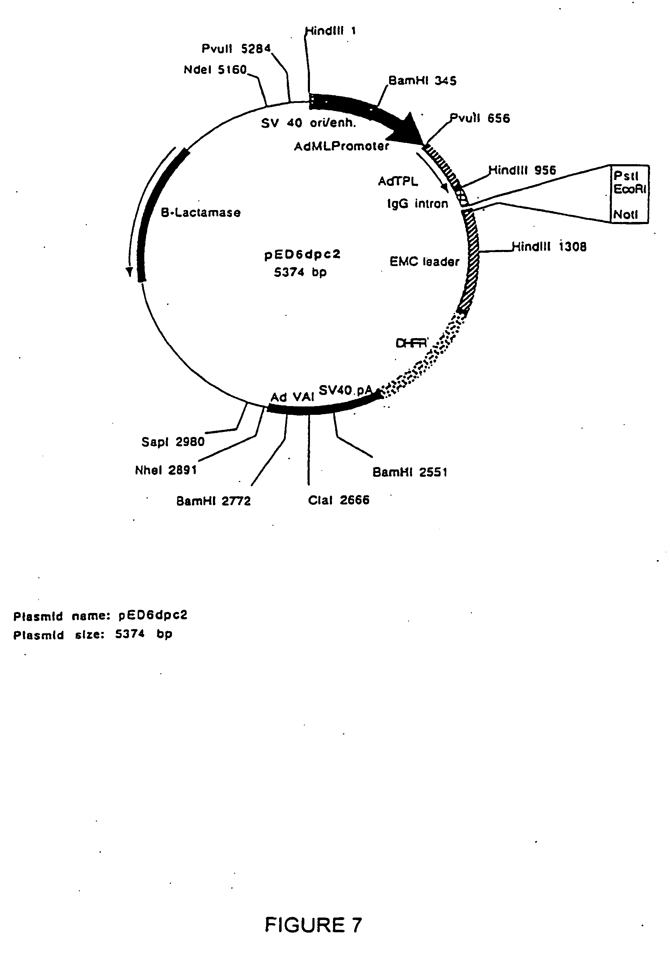

The PCR product containing the full coding sequence is then cloned in an appropriate vector. For example, the extended cDNAs can be cloned into the expression vector pED6dpc2 (DiscoverEase, Genetics Institute, Cambridge, MA) as follows. The structure of pED6dpc2 is shown in Figure 7. pED6dpc2 vector DNA is prepared with blunt ends by performing an EcoRI digestion followed by a fill in reaction. The blunt ended vector is dephosphorylated. After removal of PCR primers and ethanol precipitation, the PCR product containing the full coding sequence or the extended cDNA obtained as described above is phosphorylated with a kinase subsequently removed by phenol-Sevag extraction and precipitation. The double stranded extended cDNA is then ligated to the vector and the resulting expression plasmid introduced into appropriate host cells.

Since the PCR products obtained as described above are blunt ended molecules that can be cloned in either direction, the orientation of several clones for each PCR product is determined. Then, 4 to 10 clones are ordered in microtiter plates and subjected to a PCR reaction using a first primer located in the vector close to the cloning site and a second primer located in the portion of the extended cDNA corresponding to the 3' end of the mRNA. This second primer may be the antisense primer used in anchored PCR in the case of direct cloning (case a) or the antisense primer located inside the 3'UTR in the case of indirect cloning (case b). Clones in which the start codon of the extended cDNA is operably linked to the promoter in the vector so as to permit expression of the protein encoded by the extended cDNA are conserved and sequenced. In addition to the ends of cONA inserts, approximately 50 bp of vector DNA on each side of the cDNA insert are also sequenced.

The cloned PCR products are then entirely sequenced according to the aforementioned procedure. In this case, contig assembly of long fragments is then performed on walking sequences that have already contigated for uncloned PCR products during primer walking. Sequencing of cloned amplicons is complete when the resulting contigs include the whole coding region as well as overlapping sequences with vector DNA on both ends.

### 4. Computer Analysis of Full Length Extended cDNA

Sequences of all full length extended cDNAs are then submitted to further analysis as described below and using the parameters found in Table II with the following modifications. For screening of miscellaneous subdivisions of Genbank. FASTA was used instead of BLASTN and 15 nucleotide of homology was the limit instead of 17. For Alu detection. BLASTN was used with the following parameters: S-72; identity-70%; and length = 40 nucleotides. Polyadenylation signal and polyA tail which were not search for the 5' ESTs were searched. For polyadenylation signal detection the signal (AATAAA) was searched with one permissible mismatch in the last ten nucleotides preceding the 5' end of the polyA. For the polyA, a stretch of 8 amino acids in the last 20 nucleotides of the sequence was searched with BLAST2N in the sense strand with the following parameters (W-6, S-10, E-1000, and identity-90%). Finally, patented sequences and ORF homologies were searched using, respectively, BLASTN and 8LASTP on GenSEQ (Derwent's database of patented nucleotide sequences) and SWISSPROT for ORFs with the following parameters (W = 8 and B = 10). Before examining the extended full length cDNAs for sequences of interest, extended cDNAs which are not of interest are searched as follows.

### a) Elimination of undesired sequences

Although 5'ESTs were checked to remove contaminant sequences as described in Example 18, a last verification was carried out to identify extended cONAs sequences derived from undesired sequences such as vector RNAs, transfer RNAs, ribosomal rRNAs, mitochondrial RNAs, prokaryotic RNAs and fungal RNAs using the FASTA and BLASTN programs on both strands of extended cDNAs as described below.

To identify the extended cDNAs encoding vector RNAs, extended cDNAs are compared to the known sequences of vector RNA using the FASTA program. Sequences of extended cDNAs with more than 90% homology over stretches of 15 nucleotides are identified as vector RNA.

To identify the extended cDNAs encoding tRNAs, extended cDNA sequences were compared to the sequences of 1190 known tRNAs obtained from EMBL release 38, of which 100 were human. Sequences of extended cDNAs having more than 80% homology over 60 nucleotides using FASTA were identified as tRNA.

To identify the extended cDNAs encoding rRNAs, extended cDNA sequences were compared to the sequences of 2497 known rRNAs obtained from EMBL release 38, of which 73 were human. Sequences of extended cDNAs having more than 80% homology over stretches longer than 40 nucleotides using BLASTN were identified as rRNAs..

To identify the extended cDNAs encoding mtRNAs, extended cDNA sequences were compared to the sequences of the two known mitochondrial genomes for which the entire genomic sequences are available and all sequences transcribed from these mitochondrial genomes including tRNAs, rRNAs, and mRNAs for a total of 38 sequences. Sequences of extended cDNAs having more than 80% homology over stretches longer than 40 nucleotides using BLASTN were identified as mtRNAs.

Sequences which might have resulted from other exogenous contaminants were identified by comparing extended cDNA sequences to release 105 of Genbank bacterial and fungal divisions. Sequences of extended cDNAs having more than 90% homology over 40 nucleotides using BLASTN were identified as exogenous prokaryotic or fungal contaminants.

In addition, extended cDNAs were searched for different repeat sequences, including Alu sequences, L1 sequences, THE and MER repeats, SSTR sequences or satellite, micro-satellite, or telomeric repeats. Sequences of extended cDNAs with more than 70% homology over 40 nucleotide stretches using BLASTN were identified as repeat sequences and masked in further identification procedures. In addition, clones showing extensive homology to repeats, i.e., matches of either more than 50 nucleotides if the homology was at least 75% or more than 40 nucleotides if the homology was at least 85% or more than 30 nucleotides if the homology was at least 90%, were flagged.

### b) Identification of structural features

Structural features, e.g. polyA tail and polyadenylation signal, of the sequences of full length extended cDNAs are subsequently determined as follows.

A polyA tail is defined as a homopolymeric stretch of at least 11 A with at most one alternative base within it. The polyA tail search is restricted to the last 20 nt of the sequence and limited to stretches of 11 consecutive A's because sequencing reactions are often not readable after such a polyA stretch. Stretches with 100% homology over 6 nucleotides are identified as polyA tails.

To search for a polyadenylation signal, the polyA tail is clipped from the full-length sequence. The 50 bp preceding the polyA tail are searched for the canonic polyadenylation AAUAAA signal allowing one mismatch to account for possible sequencing errors and known variation in the canonical sequence of the polyadenylation signal.

### c) Identification of functional features

Functional features, e.g. ORFs and signal sequences, of the sequences of full length extended cDNAs were subsequently determined as follows.

The 3 upper strand frames of extended cDNAs are searched for ORFs defined as the maximum length fragments beginning with a translation initiation codon and ending with a stop codon. ORFs encoding at least 20 amino acids are preferred.

Each found ORF is then scanned for the presence of a signal peptide in the first 50 amino-acids or, where appropriate, within shorter regions down to 20 amino acids or less in the ORF, using the matrix method of von Heijne (Nuc. Acids Res. 14: 4683-4690 (1986)) and the modification described in Example 22.

### d) Homology to either nucleotidic or proteic sequences

Sequences of full length extended cDNAs are then compared to known sequences on a nucleotidic or proteic basis.

Sequences of full length extended cDNAs are compared to the following known nucleic acid sequences: vertebrate sequences (Genbank), EST sequences (Genbank), patented sequences (Geneseqn) and recently identified sequences (Genbank daily releases) available at the time of filing for the priority documents. Full length cDNA sequences are also compared to the sequences of a private database (Genset internal sequences) in order to find sequences that have already been identified by applicants. Sequences of full length extended cONAs with more than 90% homology over 30 nucleotides using either BLASTN or BLAST2N as indicated in Table III are identified as sequences that have already been described. Matching vertebrate sequences are subsequently examined using FASTA; full length extended cONAs with more than 70% homology over 30 nucleotides are identified as sequences that have already been described.

ORFs encoded by full length extended cDNAs as defined in section c) are subsequently compared to known amino acid sequences found in Swissprot release CHP, PIR release PIR# and Genpept release GPEPT public databases using BLASTP with the parameter W-8 and allowing a maximum of 10 matches. Sequences of full length extended cDNAs showing extensive homology to known protein sequences are recognized as already identified proteins.

In addition, the three-frame conceptual translation products of the top strand of full length extended cDNAs are compared to publicly known amino acid sequences of Swissprot using BLASTX with the parameter E-0.001. Sequences of full length extended cDNAs with more than 70% homology over 30 amino acid stretches are detected as already identified proteins.

### 5. Selection of Cloned Full Length Sequences of the Present Invention

Cloned full length extended cDNA sequences that have already been characterized by the aforementioned computer analysis are then submitted to an automatic procedure in order to preselect full length extended cDNAs containing sequences of interest.

### a) Automatic sequence preselection

All complete cloned full length extended cDNAs clipped for vector on both ends are considered. First, a negative selection is operated in order to eliminate unwanted cloned sequences resulting from either contaminants or PCR artifacts as follows. Sequences matching contaminant sequences such as vector RNA, tRNA, mtRNA, rRNA sequences are discarded as well as those encoding ORF sequences exhibiting extensive homology to repeats as defined in section 4 a). Sequences obtained by direct cloning using nested primers on 5' and 3' tags (section 1. case a) but lacking polyA tail are discarded. Only ORFs containing a signal peptide and ending either before the polyA tail (case a) or before the end of the cloned 3'UTR (case b) are kept. Then, ORFs containing unlikely mature proteins such as mature proteins which size is less than 20 amino acids or less than 25% of the immature protein size are eliminated.

In the selection of the OFR, priority was given to the ORF and the frame corresponding to the polypeptides described in SignalTag Patents (United States Patent Application Serial Nos: 08/905,223; 081905,135; 08/905,051; 08/905,144; 08/905,279; 08/904,468; 08/905,134; and 08/905,133). If the ORF was not found among the OFRs described in the SignalTag Patents, the ORF encoding the signal peptide with the highest score according to Von Heijne method as defined in Example 22 was chosen. If the scores were identical, then the longest ORF was chosen.

Sequences of full length extended cDNA clones are then compared pairwise with BLAST after masking of the repeat sequences. Sequences containing at least 90% homology over 30 nucleotides are clustered in the same class. Each cluster is then subjected to a cluster analysis that detects sequences resulting from internal priming or from alternative splicing, identical sequences or sequences with several frameshifts. This automatic analysis serves as a basis for manual selection of the sequences.

### b) Manual sequence selection

Manual selection is carried out using automatically generated reports for each sequenced full length extended cDNA clone. During this manual procedures, a selection is operated between clones belonging to the same class as follows. ORF sequences encoded by clones belonging to the same class are aligned and compared. If the homology between nucleotidic sequences of clones belonging to the same class is more than 90% over 30 nucleotide stretches or if the homology between amino acid sequences of clones belonging to the same class is more than 80% over 20 amino acid stretches, than the clones are considered as being identical. The chosen ORF is the best one according to the criteria mentioned below. If the nucleotide and amino acid homologies are less than 90% and 80% respectively, the clones are said to encode distinct proteins which can be both selected if they contain sequences of interest. _

Selection of full length extended cDNA clones encoding sequences of interest is performed using the following criteria. Structural parameters (initial tag, polyadenylation site and signal) are first checked. Then, homologies with known nucleic acids and proteins are examined in order to determine whether the clone sequence match a known nucleic/proteic sequence and, in the latter case, its covering rate and the date at which the sequence became public. If there is no extensive match with sequences other than ESTs or genomic DNA, or if the clone sequence brings substantial new information, such as encoding a protein resulting from alternative slicing of an mRNA coding for an already known protein, the sequence is kept. Examples of such cloned full length extended cDNAs containing sequences of interest are described in Example 28. Sequences resulting from chimera or double inserts as assessed by homology to other sequences are discarded during this procedure.

### EXAMPLE 28

### Cloning and Sequencing of Extended cDNAs

The procedure described in Example 27 above was used to obtain the extended cDNAs described herein. Using this approach, the full length cDNA of SEQ ID NO:17 was obtained. This cDNA falls into the "EST-ext" category described above and encodes the signal peptide MKKVLLLITAILAVAVG (SEQ ID NO: 18) having a von Heijne score of 8.2.

The full length cDNA of SEQ ID NO:19 was also obtained using this procedure. This cDNA falls into the "EST-ext" category described above and encodes the signal peptide MWWFQQGLSFLPSALVIWTSA (SEQ ID NO:20) having a von Heijne score of 5.5.

Another full length cDNA obtained using the procedure described above has the sequence of SEO ID NO:21. This cDNA, falls into the "EST-ext" category described above and encodes the signal peptide MVLTTLPSANSANSPVNMPTTGPNSLSYASSALSPCLT (SEQ ID NO:22) having a von Heijne score of 5.9.

The above procedure was also used to obtain a full length cDNA having the sequence of SEQ ID NO:23. This cDNA falls into the "EST-ext" category described above and encodes the signal peptide ILSTVALTFAXA (SEQ ID NO:24) having a von Heijne score of 5.5.

The full length cDNA of SEQ ID NO:25 was also obtained using this procedure. This cDNA falls into the "new" category described above and encodes a signal peptide LVLTLCTLPLAVA (SEQ ID NO:26) having a von Heijne score of 10.1.

The full length cDNA of SEQ ID NO:27 was also obtained using this procedure. This cDNA falls into the "new" category described above and encodes a signal peptide LWLLFFLVTAIHA (SEQ ID NO:28) having a von Heijne score of 10.7.

The above procedures were also used to obtain the extended cDNAs of the present invention. 5' ESTs expressed in a variety of tissues were obtained as described above.

5' ESTs obtained as described above were used to obtain extended cDNAs having the sequences of SEQ ID NOs: 40-140 and 242-377. Table IV provides the sequence identification numbers of the extended cDNAs of SEQ ID NO: 45, the location of the full coding sequence in SEQ ID NO: 45 (i.e. the nucleotides encoding both the signal peptide and the mature protein, listed under the heading FCS location in Table IV), the location of the nucleotides in SEQ ID NO: 45 which encode the signal peptides (listed under the heading SigPep Location in Table IV), the location of the nucleotides in SEQ ID NO: 45 which encode the mature proteins generated by cleavage of the signal peptide (listed under the heading Mature Polypeptide Location in Table IV), the location in SEQ ID NO 45 of stop codons (listed under the heading Stop Codon Location in Table IV), the location in SEQ ID NO: 45 of polyA signals (listed under the heading Poly A Signal Location in Table IV) and the location of polyA sites (listed under the heading Poly A Site Location in Table IV).

The polypeptides encoded by the extended cDNAs were screened for the presence of known structural or functional motifs or for the presence of signatures, small amino acid sequences which are well conserved amongst the members of a protein family. The conserved regions have been used to derive consensus patterns or matrices included in the PROSITE data bank, in particular in the file prosite-dat (Release 13.0 of November 1995. located at http://expasy.hcuge.ch/sprot/prosite.html. Prosite_convert and prosite scan programs (http://ulrec3.unil.ch/ftpserveur/prosite scan) were used to find signatures on the extended cDNAs.

For each pattern obtained with the prosite convert program from the prosite.dat file, the accuracy of the detection on a new protein sequence has been tested by evaluating the frequency of irrelevant hits on the population of human secreted proteins included in the data bank SWISSPROT. The ratio between the number of hits on shuffled proteins (with a window size of 20 amino acids) and the number of hits on native (unshuffled) proteins was used as an index. Every pattern for which the ration was greater than 20% (one hit on shuffled proteins for 5 hits on native proteins) was skipped during the search with prosite scan. The program used to shuffle protein sequences (db shuffled) and the program used to determine the statistics for each pattern in the protein data banks (prosite statistics) are available on the ftp site http://utrec3.unil.ch//ftpserveur/prosite scan.

Table V lists the sequence identification number of the polypeptides of SEQ ID NO 146, the location of the amino acid residues of SEQ ID NO 146 in the full length polypeptide (second column), the location of the amino acid residues of SEQ ID NO 146 in the signal peptides (third column), and the location of the amino acid residues of SEQ ID NO 146 in the mature polypeptide created by cleaving the signal peptide from the full length polypeptide (fourth column).

The nucleotide sequences of the sequences of SEQ ID NO: 45 and the amino acid sequence encoded by SEQ 10 NOs: 45 (i.e. amino acid sequence of SEQ ID NOs: 146) are provided in the appended sequence listing. In some instances, the sequences are preliminary and may include some incorrect or ambiguous sequences or amino acids. The sequences of SEQ ID NO: 45 can readily be screened for any errors therein and any sequence ambiguities can be resolved by resequencing a fragment containing such errors or ambiguities on both strands. Nucleic acid fragments for resolving sequencing errors or ambiguities may be obtained from the deposited clones or can be isolated using the techniques described herein. Resolution of any such ambiguities or errors may be facilitated by using primers which hybridize to sequences located close to the ambiguous or erroneous sequences. For example, the primers may hybridize to sequences within 50-75 bases of the ambiguity or error. Upon resolution of an error or ambiguity, the corresponding corrections can be made in the protein sequences encoded by the DNA containing the error or ambiguity. For example, in the sequences described herein ambiguities in the sequence of SEQ ID NO: 131 were resolved. The amino acid sequence of the protein encoded by a particular clone can also be determined by expression of the clone in a suitable host cell, collecting the protein, and determining its sequence.

For each amino acid sequence. Applicants have identified what they have determined to be the reading frame best identifiable with sequence information available at the time of filing. Some of the amino acid sequences may contain "Xaa" designators. These "Xaa" designators indicate either (1) a residue which cannot be identified because of nucleotide sequence ambiguity or (2) a stop codon in the determined sequence where Applicants believe one should not exist (if the sequence were determined more accurately).

Cells containing the extended cDNAs (SEQ ID NOs: 40-140 and 242-377) described herein in the vector pED6dpc2. are maintained in permanent deposit by the inventors at Genset. S.A., 24 Rue Royale, 75008 Paris, France.

Pools of cells containing the extended cDNAs (SEO ID NOs: 40-40 and 242-377), from which cells containing a particular polynucleotide are obtainable, were deposited with the American Type Culture Colleclion, 10801 University Blvd., Manassas, VA 20110-2209 or the European Collection of Cell Cultures, Vaccine Research and Production Laboratory, Public Health Laboratory Service. Centre for Applied Microbiology and Research, Porton Down, Salisbury. Wiltshire SP4 OJG. United Kingdom. Each extended cDNA clone has been transfected into separate bacterial cells (E-coli) for this composite deposit. Table VI lists the deposit number of the clone containing the extended cDNAs of

SEQ ID NO: 45. Table VII provides the internal designation number assigned to SEQ ID NO 45 and 146 and indicates whether the sequence is a nucleic acid sequence or a protein sequence.

Each extended cDNA can be removed from the pED6dpc2 vector in which it was deposited by performing a Notl. PstI double digestion to produce the appropriate fragment for each clone. The proteins encoded by the extended cDNAs may also be expressed from.the promoter in pED6dpc2.

Bacterial cells containing a particular clone can be obtained from the composite deposit as follows:

An oligonucleotide probe or probes should be designed to the sequence that is known for that particular clone. This sequence can be derived from the sequences provided herein, or from a combination of those sequences. The design of the oligonucleotide probe should preferably follow these parameters:
(a) It should be designed to an area of the sequence which has the fewest ambiguous bases ("N's"), if any;
(b) Preferably, the probe is designed IL have a Tₘ of approx. 80°C (assuming 2 degrees for each A or T and 4 degrees for each G or C). However, probes having melting temperatures between 40 °C and 80 °C may also be used provided that specificity is not lost.

The oligonucleotide should preferably be labeled with (-[³²P]ATP (specific activity 6000 Ci/mmole) and T4 polynucleotide kinase using commonly employed techniques for labeling oligonucleotides. Other labeling techniques can also be used. Unincorporated label should preferably be removed by gel filtration chromatography or other established methods. The amount of radioactivity incorporated into the probe should be quantified by measurement in a scintillation counter. Preferably, specific activity of the resulting probe should be approximately 4X10⁶ dpm/pmole.

The bacterial culture containing the pool of full-length clones should preferably be thawed and 100 µl of the stock used to inoculate a sterile culture flask containing 25 ml of sterile L-broth containing ampicillin at 100 ug/ml. The culture should preferably be grown to saturation at 37°C, and the saturated culture should preferably be diluted in fresh L-broth. Aliquots of these dilutions should preferably be plated to determine the dilution and volume which will yield approximately 5000 distinct and well-separated colonies on solid bacteriological media containing L-broth containing ampicillin at 100 µg/ml and agar at 1.5% in a 150 mm petri dish when grown overnight at 37°C. Other known methods of obtaining distinct, well-separated colonies can also be employed.

Standard colony hybridization procedures should then be used to transfer the colonies to nitrocellulose filters and lyse, denature and bake them.

The filter is then preferably incubated at 65°C for 1 hour with gentle agitation in 6X SSC (20X stock is 175.3 g NaCl/liter, 88.2 g Na citrate/liter, adjusted to pH 7.0 with NaOH) containing 0.5% SDS, 100 pg/ml of yeast RNA, and 10 mM EDTA (approximately 10 ml per 150 mm lilter). Preferably, the probe is then added to the hybridization mix at a concentration greater than or equal to 1X10⁵ dpm/mL. The filter is then preferably incubated at 65°C with gentle agitation overnight. The filter is then preferably washed in 500 mL of 2X SSC/0.1% SDS at room temperature with gentle shaking for 15 minutes. A third wash with 0.1X SSC/0.5% SDS at 65°C for 30 minutes to 1 hour is optional. The filter is then preferably dried and subjected to autoradiography for sufficient time to visualize the positives on the X-ray film. Other known hybridization methods can also be employed.

The positive colonies are picked, grown in culture, and plasmid DNA isolated using standard procedures. The clones can then be verified by restriction analysis, hybridization analysis, or DNA sequencing.

The plasmid DNA obtained using these procedures may then be manipulated using standard cloning techniques familiar to those skilled in the art. Alternatively, a PCR can be done with primers designed at both ends of the extended cDNA insertion. For example, a PCR reaction may be conducted using a primer having the sequence GGCCATACACTTGAGTGAC (SEQ ID NO:38) and a primer having the sequence ATATAGACAAACGCACACC (SEQ. ID. NO:39). The PCR product which corresponds to the extended cDNA can then be manipulated using standard cloning techniques familiar to those skilled in the art.

In addition to PCR based methods for obtaining extended cDNAs, traditional hybridization based methods may also be employed. These methods may also be used to obtain the genomic DNAs which encode the mRNAs from which the 5' ESTs were derived, mRNAs corresponding to the extended cDNAs, or nucleic acids which are homologous to extended cDNAs or 5' ESTs. Example 29 below provides an example of such methods.

### EXAMPLE 29

### Methods for Obtaining Extended cDNAs or Nucleic Acids Homologous to Extended cDNAs or 5' ESTs

A full length cDNA library can be made using the strategies described in Examples 13, 14, 15, and 16 above by replacing the random nonamer used in Example 14 with an oligo-dT primer. For instance, the oligonucleotide of SEQ ID NO:14 may be used.

Alternatively, a cDNA library or genomic DNA library may be obtained from a commercial source or made using techniques familiar to those skilled in the art. The library includes cDNAs which are derived from the mRNA corresponding to a 5' EST or which have homology to an extended cDNA or 5' EST. The cDNA library or genomic DNA library is hybridized to a detectable probe comprising at least 10 consecutive nucleotides from the 5' EST or extended cDNA using conventional techniques. Preferably, the probe comprises at least 12, 15, or 17 consecutive nucleotides from the 5' EST or extended cDNA. More preferably, the probe comprises at least 20-30 consecutive nucleotides from the 5' EST or extended cDNA. In some embodiments, the probe comprises at least 30 nucleotides from the 5' EST or extended cDNA. In other embodiments, the probe comprises at least 40, at least 50, at least 75, at least 100, at least 150, or at least 200 consecutive nucleotides from the 5' EST or extended cDNA.

Techniques for identifying cDNA clones in a cDNA library which hybridize to a given probe sequence are disclosed in Sambrook et al., Molecular Cloning: A Laboratory Manual 2d Ed., Cold Spring Harbor Laboratory Press, 1989. The same techniques may be used to isolate genomic DNAs.

Briefly, cDNA or genomic DNA clones which hybridize to the detectable probe are identified and isolated for further manipulation as follows. A probe comprising at least 10 consecutive nucleotides from the 5' EST or extended cDNA is labeled with a detectable label such as a radioisotope or a fluorescent molecule. Preferably, the probe comprises at least 12, 15, or 17 consecutive nucleotides from the 5' EST or extended cDNA. More preferably, the probe comprises 20-30 consecutive nucleotides from the 5' EST or extended cDNA. In some embodiments, the probe comprises more than 30 nucleotides from the 5' EST or extended cDNA. In some embodiments, the probe comprises at least 40, at least 50, at least 75, at least 100, at least 150, or at least 200 consecutive nucleotides from the 5' EST or extended cDNA.

Techniques for labeling the probe are well known and include phosphorylation with polynucleotide kinase, nick translation, in vitro transcription, and non-radioactive techniques. The cDNAs or genomic DNAs in the library are transferred to a nitrocellulose or nylon filter and denatured. After incubation of the filter with a blocking solution, the filter is contacted with the labeled probe and incubated for a sufficient amount of time for the probe to hybridize to cDNAs or genomic DNAs containing a sequence capable of hybridizing to the probe.

By varying the stringency of the hybridization conditions used to identify extended cDNAs or genomic DNAs which hybridize to the detectable probe, extended cDNAS having different levels of homology to the probe can be identified and isolated. To identify extended cDNAs or genomic DNAs having a high degree of homology to the probe sequence, the melting temperature of the probe may be calculated using the following formulas:
For probes between 14 and 70 nucleotides in length the melting temperature (Tm) is calculated using the formula: Tm=81.5+16.6(log [Na+])+0.41(fraction G+C)-(600/N) where N is the length of the probe.
If the hybridization is carried out in a solution containing formamide, the melting temperature may be calculated using the equation Tm=81.5+16.6(log [Na+])+0.41(fraction G+C)-(0.63% formamide)-(600/N) where N is the length of the probe.

Prehybridization may be carried out in 6X SSC, 5X Denhardt's reagent, 0.5% SDS, 100µg denatured fragmented salmon sperm DNA or 6X SSC, 5X Denhardt's reagent, 0.5% SDS, 100µg denatured fragmented salmon sperm DNA, 50% formamide. The formulas for SSC and Denhardt's solutions are listed in Sambrook et al., supra.

Hybridization is conducted by adding the detectable probe to the prehybridization solutions listed above. Where the probe comprises double stranded DNA, it is denatured before addition to the hybridization solution. The filter is contacted with the hybridization solution for a sufficient period of time to allow the probe to hybridize to extended cDNAs or genomic DNAs containing sequences complementary thereto or homologous thereto. For probes over 200 nucleotides in length, the hybridization may be carried out at 15-25°C below the Tm. For shorter probes, such as oligonucleotide probes, the hybridization may be conducted at 15-25°C below the Tm. Preferably, for hybridizations in 6X SSC, the hybridization is conducted at approximately 68°C. Preferably, for hybridizations in 50% formamide containing solutions, the hybridization is conducted at approximately 42°C.

All of the foregoing hybridizations would be considered to be under "stringent" conditions. Following hybridization, the filter is washed in 2X SSC, 0.1 % SDS at room temperature for 15 minutes. The filter is then washed with 0.1X SSC, 0.5% SDS at room temperature for 30 minutes to 1 hour. Thereafter, the solution is washed at the hybridization temperature in 0.1X SSC, 0.5% SDS. A final wash is conducted in 0.1X SSC at room temperature.

Extended cDNAs, nucleic acids homologous to extended cDNAs or 5' ESTs, or genomic DNAs which have hybridized to the probe are identified by autoradiography or other conventional techniques.

The above procedure may be modified to identify extended cDNAs, nucleic acids homologous to extended cDNAs, or genomic DNAs having decreasing levels of homology to the probe sequence. For example, to obtain extended cDNAs, nucleic acids homologous to extended cDNAs, or genomic DNAs of decreasing homology to the detectable probe, less stringent conditions may be used. For example, the hybridization temperature may be decreased in increments of 5°C from 68°C to 42°C in a hybridization buffer having a Na+ concentration of approximately 1 M. Following hybridization, the filter may be washed with 2X SSC, 0.5% SDS at the temperature of hybridization. These conditions are considered to be "moderate" conditions above 50°C and "low" conditions below 50°C.

Alternatively, the hybridization may be carried out in buffers, such as 6X SSC, containing formamide at a temperature of 42°C. In this case, the concentration of formamide in the hybridization buffer may be reduced in 5% increments from 50% to 0% to identify clones having decreasing levels of homology to the probe. Following hybridization, the filter may be washed with 6X SSC, 0.5% SDS at 50°C. These conditions are considered to be "moderate" conditions above 25% formamide and "low" conditions below 25% formamide.

Extended cDNAs, nucleic acids homologous to extended cDNAs, or genomic DNAs which have hybridized to the probe are identified by autoradiography.

If it is desired to obtain nucleic acids homologous to extended cDNAs, such as allelic variants thereof or nucleic acids encoding proteins related to the proteins encoded by the extended cDNAs, the level of homology between the hybridized nucleic acid and the extended cDNA or 5' EST used as the probe may readily be determined. To determine the level of homology between the hybridized nucleic acid and the extended cDNA or 5'EST from which the probe was derived, the nucleotide sequences of the hybridized nucleic acid and the extended cDNA or 5'EST from which the probe was derived are compared. For example, using the above methods, nucleic acids having at least 95% nucleic acid homology to the extended cDNA or 5'EST from which the probe was derived may be obtained and identified. Similarly, by using progressively less stringent hybridization conditions one can obtain and identify nucleic acids having at least 90%, at least 85%, at least 80% or at least 75% homology to the extended cDNA or 5'EST from which the probe was derived. The level of homology between the hybridized nucleic acid and the extended cDNA or 5' EST used as the probe may be further determined using BLAST2N; parameters may be adapted depending on the sequence length and degree of homology studied. In such comparisons, the default parameters or the parameters listed in Tables II and III may be used.

To determine whether a clone encodes a protein having a given amount of homology to the protein encoded by the extended cDNA or 5' EST, the amino acid sequence encoded by the extended cDNA or 5' EST is compared to the amino acid sequence encoded by the hybridizing nucleic acid. Homology is determined to exist when an amino acid sequence in the extended cDNA or 5' EST is closely related to an amino acid sequence in the hybridizing nucleic acid. A sequence is closely related when it is identical to that of the extended cDNA or 5' EST or when it contains one or more amino acid substitutions therein in which amino acids having similar characteristics have been substituted for one another. Using the above methods, one can obtain nucleic acids encoding proteins having at least 95%, at least 90%, at least 85%, at least 80% or at least 75% homology to the proteins encoded by the extended cDNA or 5'EST from which the probe was derived. Using the above methods and algorithms such as FASTA with parameters depending on the sequence length and degree of homology studied the level of homology may be determined. In determining the level of homology using FASTA, the default parameters or the parameters listed in Tables II or III may be used.

Alternatively, extended cDNAs may be prepared by obtaining mRNA from the tissue, cell, or organism of interest using mRNA preparation procedures utilizing poly A selection procedures or other techniques known to those skilled in the art. A first primer capable of hybridizing to the poly A tail of the mRNA is hybridized to the mRNA and a reverse transcription reaction is performed to generate a first cDNA strand.

The first cDNA strand is hybridized to a second primer containing at least 10 consecutive nucleotides of the sequences of the 5' EST for which an extended cDNA is desired. Preferably, the primer comprises at least 12, 15, or 17 consecutive nucleotides from the sequences of the 5' EST. More preferably, the primer comprises 20-30 consecutive nucleotides from the sequences of the 5' EST. In some embodiments, the primer comprises more than 30 nucleotides from the sequences of the 5' EST. If it is desired to obtain extended cDNAs containing the full protein coding sequence, including the authentic translation initiation site, the second primer used contains sequences located upstream of the translation initiation site. The second primer is extended to generate a second cDNA strand complementary to the first cDNA strand. Alternatively, RTPCR may be performed as described above using primers from both ends of the cDNA to be obtained.

Extended cDNAs containing 5' fragments of the mRNA may be prepared by contacting an mRNA comprising the sequence of the 5' EST for which an extended cDNA is desired with a primer comprising at least 10 consecutive nucleotides of the sequences complementary to the 5' EST, hybridizing the primer to the mRNAs, and reverse transcribing the hybridized primer to make a first cDNA strand from the mRNAs. Preferably, the primer comprises at least 12, 15, or 17 consecutive nucleotides from the 5' EST. More preferably, the primer comprises 20-30 consecutive nucleotides from the 5' EST.

Thereafter, a second cDNA strand complementary to the first cDNA strand is synthesized. The second cDNA strand may be made by hybridizing a primer complementary to sequences in the first cDNA strand to the first cDNA strand and extending the primer to generate the second cDNA strand.

The double stranded extended cDNAs made using the methods described above are isolated and cloned. The extended cDNAs may be cloned into vectors such as plasmids or viral vectors capable of replicating in an appropriate host cell. For example, the host cell may be a bacterial, mammalian, avian, or insect cell.

Techniques for isolating mRNA, reverse transcribing a primer hybridized to mRNA to generate a first cDNA strand, extending a primer to make a second cDNA strand complementary to the first cDNA strand, isolating the double stranded cDNA and cloning the double stranded cDNA are well known to those skilled in the art and are described in Current Protocols in Molecular Biology, John Wiley 503 Sons, Inc. 1997 and Sambrook et al. Molecular Cloning: A Laboratory Manual, Second Edition, Cold Spring Harbor Laboratory Press, 1989.

Alternatively, kits for obtaining full length cDNAs, such as the GeneTrapper (Cat. No. 10356-020, Gibco, BRL), may be used for obtaining full length cDNAs or extended cDNAs. In this approach, full length or extended cDNAs are prepared from mRNA and cloned into double stranded phagemids. The cDNA library in the double stranded phagemids is then rendered single stranded by treatment with an endonuclease, such as the Gene II product of the phage F1, and Exonuclease III as described in the manual accompanying the GeneTrapper kit. A biotinylated oligonucleotide comprising the sequence of a 5' EST, or a fragment containing at least 10 nucleotides thereof, is hybridized to the single stranded phagemids. Preferably, the fragment comprises at least 12, 15, or 17 consecutive nucleotides from the 5' EST. More preferably, the fragment comprises 20-30 consecutive nucleotides from the 5' EST. In some procedures, the fragment may comprise more than 30 consecutive nucleotides from the 5' EST. For example, the fragment may comprises at least 40, at least 50, at least 75, at least 100, at least 150, or at least 200 consecutive nucleotides from the 5' EST.

Hybrids between the biotinylated oligonucleotide and phagemids having inserts containing the 5' EST sequence are isolated by incubating the hybrids with streptavidin coated paramagnetic beads and retrieving the beads with a magnet. Thereafter, the resulting phagemids containing the 5' EST sequence are released from the beads and converted into double stranded DNA using a primer specific for the 5' EST sequence. The resulting double stranded DNA is transformed into bacteria. Extended cDNAs containing the 5' EST sequence are identified by colony PCR or colony hybridization.

A plurality of extended cDNAs containing full length protein coding sequences or sequences encoding only the mature protein remaining after the signal peptide is cleaved may be provided as cDNA libraries for subsequent evaluation of the encoded proteins or use in diagnostic assays as described below.

### IV. Expression of Proteins Encoded by Extended cDNAs Isolated Using 5' ESTs

Extended cDNAs containing the full protein coding sequences of their corresponding mRNAs or portions thereof, such as cDNAs encoding the mature protein, may be used to express the secreted proteins or portions thereof which they encode as described in Example 30 below. If desired, the extended cDNAs may contain the sequences encoding the signal peptide to facilitate secretion of the expressed protein. It will be appreciated that a plurality of extended cDNAs containing the full protein coding sequences or portions thereof may be simultaneously cloned into expression vectors to create an expression library for analysis of the encoded proteins as described below.

### EXAMPLE 30

### Expression of the Proteins Encoded by Extended cDNAs or Portions Thereof

To express the proteins encoded by the extended cDNAs or portions thereof, nucleic acids containing the coding sequence for the proteins or portions thereof to be expressed are obtained as described in Examples 27-29 and cloned into a suitable expression vector. If desired, the nucleic acids may contain the sequences encoding the signal peptide to facilitate secretion of the expressed protein. For example, the nucleic acid may comprise the sequence of SEQ ID NO 45 listed in Table IV and in the accompanying sequence listing. Alternatively, the nucleic acid may comprise those nucleotides which make up the full coding sequence of the sequence of SEQ ID NO: 45 as defined in Table IV above.

It will be appreciated that should the extent of the full coding sequence (i.e. the sequence encoding the signal peptide and the mature protein resulting Irom cleavage of the signal peptide) differ from that listed in Table IV as a result of a sequencing error, reverse transcription or amplification error, mRNA splicing, post-translational modification of the encoded protein, enzymatic cleavage of the encoded protein, or other biological factors, one skilled in the art would be readily able to identify the extent of the full coding sequence in the sequence of SEQ ID NO. 45. For example, the sequence of SEQ ID NO: 115 represents an alternatively spliced transcript of a previously identified mRNA. Accordingly, the scope of any claims herein relating to nucleic acids containing the full coding sequence of SEQ ID NO. 45 is not to be construed as excluding any readily identifiable variations from or equivalents to the full coding sequences listed in Table IV Similarly, should the extent of the full length polypeptides differ from those indicated in Table V as a result of any of the preceding factors, the scope of claims relating to polypeptides comprising the amino acid sequence of the full length polypeptide is not to be construed as excluding any readily identifiable variations from or equivalents to the sequences listed in Table V.

Alternatively, the nucleic acid used to express the protein or portion thereof may comprise those nucleotides which encode the mature protein (i.e. the protein created by cleaving the signal peptide off) encoded by

SEQ ID NO: 45 as defined in Table IV above. It will be appreciated that should the extent of the sequence encoding the mature protein differ from that listed in Table IV as a result of a sequencing error, reverse transcription or amplification error, mRNA splicing, post-translational modification of the encoded protein, enzymatic cleavage of the encoded protein, or other biological factors, one skilled in the art would be readily able to identify the extent of the sequence encoding the mature protein in the sequence of SEQ ID NO. 45. Accordingly, the scope of any claims herein relating to nucleic acids containing the sequence encoding the mature protein encoded by SEQ ID No. 45 is not to be construed as excluding any readily identifiable variations from or equivalents to the sequences listed in Table IV. Thus, claims relating to nucleic acids containing the sequence encoding the mature protein encompass equivalents to the sequences listed in Table IV. such as sequences encoding biologically active proteins resulting from post-translational modification, enzymatic cleavage, or other readily identifiable variations from or equivalents to the secreted protein in addition to cleavage of the signal peptide. Similarly, should the extent of the mature polypeptide differ from that indicated in Table V as a result of any of the preceding factors, the scope of claims relating to polypeptides comprising the sequence of a mature protein included in the sequence of SEQ ID NOs. 146 is not to be construed as excluding any readily identifiable variations from or equivalents to the sequences listed in Table V. Thus, claims relating to polypeptides comprising the sequence of the mature protein encompass equivalents to the sequences listed in Table IV, such as biologically active proteins resulting from post-translational modification, enzymatic cleavage, or other readily identifiable variations from or equivalents to the secreted proteins in addition to cleavage of the signal peptide. It will also be appreciated that should the biologically active form of the polypeptide included in the sequence of SEQ ID NOs. 146 or the nucleic acid encoding the biologically active form of the polypeptide differ from those identified as the mature polypeptide in Table V or the nucleotides encoding the mature polypeptide in Table IV as a result of a sequencing error, reverse transcription or amplification error, mRNA splicing, post-translational modification of the encoded protein, enzymatic cleavage of the encoded protein, or other biological factors, one skilled in the art would be readily able to identify the amino acids in the biologically active form of the polypeptides and the nucleic acids encoding the biologically active form of the polypeptides. In such instances, the claims relating to polypetides comprising the mature protein included in SEQ ID NO. 146 or nucleic acids comprising the nucleotides of SEQ ID NO. 45 encoding the mature protein shall not be construed to exclude any readily identifiable variations from the sequences listed in Table IV and Table V.

In some embodiments, the nucleic acid used to express the protein or portion thereof may comprise those nucleotides which encode the signal peptide encoded by SEQ 10 NOs: 45 as defined in Table IV above.

It will be appreciated that should the extent of the sequence encoding the signal peptide differ from that listed in Table IV as a result of a sequencing error, reverse transcription or amplification error, mRNA splicing, post-translational modification of the encoded protein, enzymatic cleavage of the encoded protein, or other biological factors, one skilled in the art would be readily able to identify the extent of the sequence encoding the signal peptide in the sequence of SEQ ID NO 45. Accordingly, the scope of any claims herein relating to nucleic acids containing the sequence encoding the signal peptide encoded by SEQ ID No. 45 is not to be construed as excluding any readily identifiable variations from the sequences listed in Table IV. Similarly, should the extent of the signal peptide differ from those indicated in Table V as a result of any of the preceding factors, the scope of claims relating to polypeptides comprising the sequence of a signal peptide included in the sequence of SEQ ID NOs. 146 is not to be construed as excluding any readily identifiable variations from the sequences listed in Table V.

Alternatively, the nucleic acid may encode a polypeptide comprising at least 10 consecutive amino acids of the sequence of SEQ ID NOs: 146. The nucleic acid may encode a polypeptide comprising at least 15 consecutive amino acids of the sequence of SEQ ID NOs: 146. The nucleic acid may encode a polypeptide comprising at least 25 consecutive amino acids of the sequence of SEQ ID NOs: 146. The nucleic acid may encode a polypeptide comprising at least 60, at least 75, at least 100 or more than 100 consecutive amino acids of the sequence of SEQ ID Nos: 146.

The nucleic acids inserted into the expression vectors may also contain sequences upstream of the sequences encoding the signal peptide, such as sequences which regulate expression levels or sequences which confer tissue specific expression.

The nucleic acid encoding the protein or polypeptide to be expressed is operably linked to a promoter in an expression vector using conventional cloning technology. The expression vector may be any of the mammalian, yeast, insect or bacterial expression systems known in the art. Commercially available vectors and expression systems are available from a variety of suppliers including Genetics Institute (Cambridge, MA), Stratagene (La Jolla, California), Promega (Madison, Wisconsin), and Invitrogen (San Diego, California). If desired, to enhance expression and facilitate proper protein folding, the codon context and codon pairing of the sequence may be optimized for the particular expression organism in which the expression vector is introduced, as explained by Hatfield, et al., U.S. Patent No. 5,082,767.

The following is provided as one exemplary method to express the proteins encoded ty the extended cDNAs corresponding to the 5' ESTs or the nucleic acids described above. First, the methionine initiation codon for the gene and the poly A signal of the gene are identified. If the nucleic acid encoding the polypeptide to be expressed lacks a methionine to serve as the initiation site, an initiating methionine can be introduced next to the first codon of the nucleic acid using conventional techniques. Similarly, if the extended cDNA lacks a poly A signal, this sequence can be added to the construct by, for example, splicing out the Poly A signal from pSG5 (Stratagene) using BglI and Sall restriction endonuclease enzymes and incorporating it into the mammalian expression vector pXT1 (Stratagene). pXT1 contains the LTRs and a portion of the gag gene from Moloney Murine Leukemia Virus. The position of the LTRs in the construct allow efficient stable transfection. The vector includes the Herpes Simplex Thymidine Kinase promoter and the selectable neomycin gene. The extended cDNA or portion thereof encoding the polypeptide to be expressed is obtained by PCR from the bacterial vector using oligonucleotide primers complementary to the extended cDNA or portion thereof and containing restriction endonuclease sequences for Pst I incorporated into the 5'primer and BgIII at the 5' end of the corresponding cDNA 3' primer, taking care to ensure that the extended cDNA is positioned in frame with the poly A signal. The purified fragment obtained from the resulting PCR reaction is digested with PstI, blunt ended with an exonuclease, digested with Bgl II, purified and ligated to pXT1, now containing a poly A signal and digested with BgIII.

The ligated product is transfected into mouse NIH 3T3 cells using Lipofectin (Life Technologies, Inc., Grand Island, New York) under conditions outlined in the product specification. Positive transfectants are selected after growing the transfected cells in 600ug/ml G418 (Sigma, St. Louis, Missouri). Preferably the expressed protein is released into the culture medium, thereby facilitating purification.

Alternatively, the extended cDNAs may be cloned into pED6dpc2 as described above. The resulting pED6dpc2 constructs may be transfected into a suitable host cell, such as COS 1 cells. Methotrexate resistant cells are selected and expanded. Preferably, the protein expressed from the extended cDNA is released into the culture medium thereby facilitating purification.

Proteins in the culture medium are separated by gel electrophoresis. If desired, the proteins may be ammonium sulfate precipitated or separated based on size or charge prior to electrophoresis.

As a control, the expression vector lacking a cDNA insert is introduced into host cells or organisms and the proteins in the medium are harvested. The secreted proteins present in the medium are detected using techniques such as Coomassie or silver staining or using antibodies against the protein encoded by the extended cDNA. Coomassie and silver staining techniques are familiar to those skilled in the art.

Antibodies capable of specifically recognizing the protein of interest may be generated using synthetic 15-mer peptides having a sequence encoded by the appropriate 5' EST, extended cDNA, or portion thereof. The synthetic peptides are injected into mice to generate antibody to the polypeptide encoded by the 5' EST, extended cDNA, or portion thereof.

Secreted proteins from the host cells or organisms containing an expression vector which contains the extended cDNA derived from a 5' EST or a portion thereof are compared to those from the control cells or organism. The presence of a band in the medium from the cells containing the expression vector which is absent in the medium from the control cells indicates that the extended cDNA encodes a secreted protein. Generally, the band corresponding to the protein encoded by the extended cDNA will have a mobility near that expected based on the number of amino acids in the open reading frame of the extended cDNA. However, the band may have a mobility different than that expected as a result of modifications such as glycosylation, ubiquitination, or enzymatic cleavage.

Alternatively, if the protein expressed from the above expression vectors does not contain sequences directing its secretion, the proteins expressed from host cells containing an expression vector containing an insert encoding a secreted protein or portion thereof can be compared to the proteins expressed in host cells containing the expression vector without an insert. The presence of a band in samples from cells containing the expression vector with an insert which is absent in samples from cells containing the expression vector without an insert indicates that the desired protein or portion thereof is being expressed. Generally, the band will have the mobility expected for the secreted protein or portion thereof. However, the band may have a mobility different than that expected as a result of modifications such as glycosylation, ubiquitination, or enzymatic cleavage.

The protein encoded by the extended cDNA may be purified using standard immunochromatography techniques. In such procedures, a solution containing the secreted protein, such as the culture medium or a cell extract, is applied to a column having antibodies against the secreted protein attached to the chromatography matrix. The secreted protein is allowed to bind the immunochromatography column. Thereafter, the column is washed to remove non-specifically bound proteins. The specifically bound secreted protein is then released from the column and recovered using standard techniques.

If antibody production is not possible, the extended cDNA sequence or portion thereof may be incorporated into expression vectors designed for use in purification schemes employing chimeric polypeptides. In such strategies the coding sequence of the extended cDNA or portion thereof is inserted in frame with the gene encoding the other half of the chimera. The other half of the chimera may be β-globin or a nickel binding polypeptide encoding sequence. A chromatography matrix having antibody to β-globin or nickel attached thereto is then used to purify the chimeric protein. Protease cleavage sites may be engineered between the β-globin gene or the nickel binding polypeptide and the extended cDNA or portion thereof. Thus, the two polypeptides of the chimera may be separated from one another by protease digestion.

One useful expression vector for generating β-globin chimerics is pSG5 (Stratagene), which encodes rabbit β-globin. Intron II of the rabbit β-globin gene facilitates splicing of the expressed transcript, and the polyadenylation signal incorporated into the construct increases the level of expression. These techniques as described are well known to those skilled in the art of molecular biology. Standard methods are published in methods texts such as Davis et al., (Basic Methods in Molecular Biology, L.G. Davis, M.D. Dibner, and J.F. Battey, ed., Elsevier Press, NY, 1986) and many of the methods are available from Stratagene, Life Technologies, Inc., or Promega. Polypeptide may additionally be produced from the construct using in vitro translation systems such as the In vitro Express^{™} Translation Kit (Stratagene).

Following expression and purification of the secreted proteins encoded by the 5' ESTs, extended cDNAs, or fragments thereof, the purified proteins may be tested for the ability to bind to the surface of various cell types as described in Example 31 below. It will be appreciated that a plurality of proteins expressed from these cDNAs may be included in a panel of proteins to be simultaneously evaluated for the activities specifically described below, as well as other biological roles for which assays for determining activity are available.

### EXAMPLE 31

### Analysis of Secreted Proteins to Determine Whether they Bind to the Cell Surface

The proteins encoded by the 5' ESTs, extended cDNAs, or fragments thereof are cloned into expression vectors such as those described in Example 30. The proteins are purified by size, charge, immunochromatography or other techniques familiar to those skilled in the art. Following purification, the proteins are labeled using techniques known to those skilled in the art. The labeled proteins are incubated with cells or cell lines derived from a variety of organs or tissues to allow the proteins to bind to any receptor present on the cell surface. Following the incubation, the cells are washed to remove non-specifically bound protein. The labeled proteins are detected by autoradiography. Alternatively, unlabeled proteins may be incubated with the cells and detected with antibodies having a detectable label, such as a fluorescent molecule, attached thereto.

Specificity of cell surface binding may be analyzed by conducting a competition analysis in which various amounts of unlabeled protein are incubated along with the labeled protein. The amount of labeled protein bound to the cell surface decreases as the amount of competitive unlabeled protein increases. As a control, various amounts of an unlabeled protein unrelated to the labeled protein is included in some binding reactions. The amount of labeled protein bound to the cell surface does not decrease in binding reactions containing increasing amounts of unrelated unlabeled protein, indicating that the protein encoded by the cDNA binds specifically to the cell surface.

As discussed above, secreted proteins have been shown to have a number of important physiological effects and, consequently, represent a valuable therapeutic resource. The secreted proteins encoded by the extended cDNAs or portions thereof made according to Examples 27-29 may be evaluated to determine their physiological activities as described below.

### EXAMPLE 32

### Assaying the Proteins Expressed from Extended cDNAs or Portions Thereof for Cytokine, Cell Proliferation or Cell Differentiation Activity

As discussed above, secreted proteins may act as cytokines or may affect cellular proliferation or differentiation. Many protein factors discovered to date, including all known cytokines, have exhibited activity in one or more factor dependent cell proliferation assays, and hence the assays serve as a convenient confirmation of cytokine activity. The activity of a protein described herein is evidenced by any one of a number of routine factor dependent cell proliferation assays for cell lines including, without limitation, 32D, DA2, DA1G, T10, B9, B9/11, 8aF3, MC9/G, M+ (preB M+), 2E8, RB5, DA1, 123, T1165, HT2, CTLL2, TF-1, Mo7c and CMK. The proteins encoded by the above extended cDNAs or portions thereof may be evaluated for their ability to regulate T cell or thymocyte proliferation in assays such as those described above or in the following references: Current Protocols in Immunology, Ed. by J.E. Coligan et al., Greene Publishing Associates and Wiley-Interscience; Takai et at. J. Immunol, 137:3494-3500, 1986. Bertagnolli et al. J. Immunol. 145:1706-1712, 1990. Bertagnolli et al., Cellular Immunology 133:327-341, 1991. Bertagnolli, et al. J. Immunol. 149:3778-3783, 1992; Bowman et al., J. Immunol. 152:1756-1761, 1994.

In addition, numerous assays for cytokine production and/or the proliferation of spleen cells, lymph node cells and thymocytes are known. These include the techniques disclosed in Current Protocols in Immunology. J.E. Coligan et al. Eds., Vol 1 pp. 3.12.1-3.12.14 John Wiley and Sons, Toronto. 1994; and Schreiber, R.D. Current Protocols in Immunology., supra Vol 1 pp. 6.8.1-6.8.8, John Wiley and Sons, Toronto. 1994.

The proteins encoded by the cDNAs may also be assayed for the ability to regulate the proliferation and differentiation of hematopoietic or lymphopoietic cells. Many assays for such activity are familiar to those skilled in the art, including the assays in the following references: Bottomly, K., Davis, LS. and Lipsky, P.E., Measurement of Human and Murine Interleukin 2 and Interleukin 4, Current Protocols in Immunology., J.E. Coligan et al. Eds. Vol 1 pp. 6.3.1-6.3.12, John Wiley and Sons, Toronto. 1991; deVries et al., J. Exp. Med. 173:1205-1211, 1991: Moreau et al., Nature 36:690-692, 1988; Greenberger et al., Proc. Natl. Acad. Sci. U.S.A. 80:2931-2938, 1983; Nordan, R., Measurement of Mouse and Human Interleukin 6 Current Protocols in Immunology. J.E. Coligan et al. Eds. Vol 1 pp. 6.6.1-6.6.5, John Wiley and Sons, Toronto. 1991; Smith et al., Proc. Natl. Acad. Sci. U.S.A. 83:1857-1861, 1986; Bennett, F., Giannotti, J., Clark, S.C. and Turner, K.J., Measurement of Human Interleukin 11 Current Protocols in Immunology. J.E. Coligan et al. Eds. Vol 1 pp. 6.15.1 John Wiley and Sons. Toronto. 1991; Ciarletta, A., Giannotti, J., Clark. S.C. and Turner, K.J., Measurement of Mouse and Human Interleukin 9 Current Protocols in Immunology. J.E. Coligan et al., Eds. Vol 1 pp. 6.13.1, John Wiley and Sons, Toronto. 1991.

The proteins encoded by the cDNAs may also be assayed for their ability to regulate T-cell responses to antigens. Many assays for such activity are familiar to those skilled in the art, including the assays described in the following references: Chapter 3 (In Vitro Assays for Mouse lymphocyte Function), Chapter 6 (Cytokines and Their Cellular Receptors) and Chapter 7, (Immunologic Studies in Humans) in Current Protocols in Immunology, J.E. Coligan et al. Eds. Greene Publishing Associates and Wiley-Interscience; Weinberger et al., Proc. Natl. Acad. Sci. USA 77:6091-6095, 1980; Weinberger et al., Eur. J. Immun. 11:405-411, 1981; Takai et al., J. Immunol. 137:3494-3500, 1986; Takai et al., J. Immunol. 140:508-512, 1988.

Those proteins which exhibit cytokine, cell proliferation, or cell differentiation activity may then be formulated as pharmaceuticals and used to treat clinical conditions in which induction of cell proliferation or differentiation is beneficial. Alternatively, as described in more detail below, genes encoding these proteins or nucleic acids regulating the expression of these proteins may be introduced into appropriate host cells to increase or decrease the expression of the proteins as desired.

### EXAMPLE 33

### Assaying the Proteins Expressed from Extended cDNAs or Portions Thereof for Activity as Immune System Regulators

The proteins encoded by the cDNAs may also be evaluated for their effects as immune regulators. For example, the proteins may be evaluated for their activity to influence thymocyte or splenocyte cytotoxicity. Numerous assays for such activity are familiar to those skilled in the art including the assays described in the following references: Chapter 3 (In Vitro Assays for Mouse Lymphocyte Function 3.1-3.19) and Chapter 7 (Immunologic studies in Humans) in Current Protocols in Immunology, J.E. Coligan et al. Eds, Greene Publishing Associates and Wiley-Interscience; Herrmann et al., Proc. Natl. Acad. Sci. USA 78:2488-2492, 1981; Herrmann et al., J. Immunol. 128:1968-1974, 1982; Handa et al., J. Immunol. 135:1564-1572, 1985; Takai et al., J. Immunol. 137:3494-3500, 1986; Takai et al., J. Immunol. 140:508-512, 1988; Herrmann et al., Proc. Natl. Acad. Sci. USA 78:2488-2492, 1981; Herrmann et al., J. Immunol. 128:1968-1974, 1982; Handa et al., J. Immunol. 135:1564-1572, 1985; Takai et al., J. Immunol. 137:3494-3500, 1986; Bowman et al., J. Virology 61:1992-1998; Takai et al., J. Immunol. 140:508-512, 1988; Bertagnolli et al., Cellular Immunology 133:327-341, 1991; Brown et al., J. Immunol. 153:3079-3092, 1994.

The proteins encoded by the cDNAs may also be evaluated for their effects on T-cell dependent immunoglobulin responses and isotype switching. Numerous assays for such activity are familiar to those skilled in the art, including the assays disclosed in the following references: Maliszewski, J. Immunol. 144:3028-3033, 1990; Mond, J.J. and Brunswick, M Assays for B Cell Function: In vitro Antibody Production, Vol 1 pp. 3.8.1-3.8.16 in Current Protocols in Immunology. J.E. Coligan et al Eds., John Wiley and Sons, Toronto. 1994.

The proteins encoded by the cDNAs may also be evaluated for their effect on immune effector cells, including their effect on Th1 cells and cytotoxic lymphocytes. Numerous assays for such activity are familiar to those skilled in the art, including the assays disclosed in the following references: Chapter 3 (In Vitro Assays for Mouse Lymphocyte function 3.1-3.19) and Chapter 7 (Immunologic Studies in Humans) in Current Protocols in Immunology, J.E. Coligan et al. Eds., Greene Publishing Associates and Wiley-Interscience; Takai et al., J. Immunol. 137:3494-3500, 1986; Takai et al.; J. Immunol. 140:508-512, 1988: Bertagnolli et al., J. Immunol. 149:3778-3783, 1992.

The proteins encoded by the cDNAs may also be evaluated for their effect on dendritic cell mediated activation of naive T-cells. Numerous assays for such activity are familiar to those skilled in the art, including the assays disclosed in the following references: Guery et al., J. Immunol, 134:536-544, 1995; Inaba et al., Journal of Experimental Medicine 173:549-559, 1991; Macatonia et al., Journal of Immunology 154:5071-5079, 1995; Porgador et al., Journal of Experimental Medicine 182:255-260, 1995; Nair et al., Journal of Virology 67:4062-4069, 1993; Huang et al., Science 264:961-965, 1994; Macatonia et al., Journal of Experimental Medicine 169:1255-1264, 1989; Bhardwaj et al., Journal of Clinical Investigation 94:797-807, 1994; and Inaba et al., Journal of Experimental Medicine 172:631-640, 1990.

The proteins encoded by the cDNAs may also be evaluated for their influence on the lifetime of lymphocytes. Numerous assays for such activity are familiar to those skilled in the art, including the assays disclosed in the following references: Darzynkiewicz et al., Cytometry 13:795-808, 1992; Gorczyca et al., Leukemia 7:659-670, 1993; Gorczyca et al., Cancer Research 53:1945-1951, 1993; Itoh et al., Cell 66:233-243, 1991; Zacharchuk, Journal of Immunology 145:4037-4045, 1990; Zamai et al., Cytometry 14:891-897, 1993; Gorczyca et al., International Journal of Oncology 1:639-648, 1992.

Assays for proteins that influence early steps of T-cell commitment and development include, without limitation, those described in: Antica et al., Blood 84:111-117, 1994; Fine et al.. Cellular immunology 155: 111-122, 1994; Galy et al., Blood 85:2770-2778, 1995; Toki et al., Proc. Nat. Acad Sci. USA 88:7548-7551, 1991.

Those proteins which exhibit activity as immune system regulators activity may then be formulated as pharmaceuticals and used to treat clinical conditions in which regulation of immune activity is beneficial. For example, the protein may be useful in the treatment of various immune deficiencies and disorders (including severe combined immunodeficiency (SCID)), e.g., in regulating (up or down) growth and proliferation of T and/or B lymphocytes, as well as effecting the cytolytic activity of NK cells and other cell populations. These immune deficiencies may be genetic or be caused by viral (e.g., HIV) as well as bacterial or fungal infections, or may result from autoimmune disorders. More specifically, infectious diseases caused by viral, bacterial, fungal or other infection may be treatable using a protein of the present invention, including infections by HIV, hepatitis viruses, herpesviruses, mycobacteria, Leishmania spp., malaria spp. and various fungal infections such as candidiasis. Of course, in this regard, a protein may also be useful where a boost to the immune system generally may be desirable, i.e., in the treatment of cancer.

Autoimmune disorders which may be treated using a protein include, for example, connective tissue disease, multiple sclerosis, systemic lupus erythematosus, rheumatoid arthritis, autoimmune pulmonary inflammation, Guillain-Barre syndrome, autoimmune thyroiditis, insulin dependent diabetes mellitis, myasthenia gravis, graft-versus-host disease and autoimmune inflammatory eye disease. Such a protein of the present invention may also to be useful in the treatment of allergic reactions and conditions, such as asthma (particularly allergic asthma) or other respiratory problems. Other conditions, in which immune suppression is desired (including, for example, organ transplantation), may also be treatable using a protein.

Using the proteins it may also be possible to regulate immune responses, in a number of ways. Down regulation may be in the form of inhibiting or blocking an immune response already in progress or may involve preventing the induction of an immune response. The functions of activated T-cells may be inhibited by suppressing T cell responses or by inducing specific tolerance in T cells, or both. Immunosuppression of T cell responses is generally an active, non-antigen-specific, process which requires continuous exposure of the T cells to the suppressive agent. Tolerance, which involves inducing non-responsiveness or anergy in T cells, is distinguishable from immunosuppression in that it is generally antigen-specific and persists after exposure to the tolerizing agent has ceased. Operationally, tolerance can be demonstrated by the lack of a T cell response upon reexposure to specific antigen in the absence of the tolerizing agent.

Down regulating of preventing one or more antigen functions (including without limitation 8 lymphocyte antigen functions (such as, for example, 87)), e.g., preventing high level lymphokine synthesis by activated T cells, will be useful in situations of tissue, skin and organ transplantation and in graft-versus-host disease (GVHD). For example, blockage of T cell function should result in reduced tissue destruction in tissue transplantation. Typically, in tissue transplants, rejection of the transplant is initiated through its recognition as foreign by T cells, followed by an immune reaction that destroys the transplant. The administration of a molecule which inhibits or blocks interaction of a 87 lymphocyte antigen with its natural ligand(s) on immune cells (such as a soluble, monomeric form of a peptide having 87-2 activity alone or in conjunction with a monomeric form of a peptide having an activity of another 8 lymphocyte antigen (e.g., 87-1, B7-3) or blocking antibody), prior to transplantation can lead to the binding of the molecule to the natural ligand(s) on the immune cells without transmitting the corresponding costimulatory signal. Blocking B lymphocyte antigen function in this matter prevents cytokine synthesis by immune cells, such as T cells, and thus acts as an immunosuppressant. Moreover, the lack of costimulation may also be sufficient to anergize the T cells, thereby inducing tolerance in a subject. Induction of long-term tolerance by B lymphocyte antigen-blocking reagents may avoid the necessity of repeated administration of these blocking reagents. To achieve sufficient immunosuppression or tolerance in a subject, it may also be necessary to block the function of a combination of B lymphocyte antigens.

The efficacy of particular blocking reagents in preventing organ transplant rejection or GVHD can be assessed using animal models that are predictive of efficacy in humans. Examples of appropriate systems which can be used include allogeneic cardiac grafts in rats and renogeneic pancreatic islet cell grafts in mice, both of which have been used to examine the immunosuppressive effects of CTLA4Ig fusion proteins in vivo as described in Lenschow et al., Science 257:789-792 (1992) and Turka et al., Proc. Natl. Acad. Sci USA. 89:11102-11105 (1992). In addition, murine models of GVHD (see Paul ed.. Fundamental Immunology, Raven Press, New York, 1989, pp. 846-847) can be used to determine the effect of blocking B lymphocyte antigen function in vivo on the development of that disease.

Blocking antigen function may also be therapeutically useful for treating autoimmune diseases. Many autoimmune disorders are the result of inappropriate activation of T cells that are reactive against self tissue and which promote the production of cytokines and autoantibodies involved in the pathology of the diseases. Preventing the activation of autoreactive T cells may reduce or eliminate disease symptoms. Administration of reagents which block costimulation of T cells by disrupting receptor ligand interactions of B lymphocyte antigens can be used to inhibit T cell activation and prevent production of autoantibodies or T cell-derived cytokines which may be involved in the disease process. Additionally, blocking reagents may induce antigen-specific tolerance of autoreactive T cells which could lead to long-term relief from the disease. The efficacy of blocking reagents in preventing or alleviating autoimmune disorders can be determined using a number of well-characterized animal models of human autoimmune diseases. Examples include murine experimental autoimmune encephalitis, systemic lupus erythmatosis in MRL/pr/pr mice or NZB hybrid mice, murine autoimmuno collagen arthritis, diabetes mellitus in OD mice and BB rats, and murine experimental myasthenia gravis (see Paul ed., Fundamental Immunology, Raven Press, New York, 1989, pp. 840-856).

Upregulation of an antigen function (preferably a B lymphocyte antigen function), as a means of up regulating immune responses, may also be useful in therapy. Upregulation of immune responses may be in the form of enhancing an existing immune response or eliciting an initial immune response. For example, enhancing an immune response through stimulating B lymphocyte antigen function may be useful in cases of viral infection. In addition, systemic viral diseases such as influenza, the common cold, and encephalitis might be alleviated by the administration of stimulatory form of B lymphocyte antigens systemically.

Alternatively, anti-viral immune responses may be enhanced in an infected patient by removing T cells from the patient, costimulating the T cells in vitro with viral antigen-pulsed APCs either expressing a peptide of the present invention or together with a stimulatory form of a soluble peptide of the present invention and reintroducing the in vitro activated T cells into the patient. The infected cells would now be capable of delivering a costimulatory signal to T cells in vivo, thereby activating the T cells.

In another application, up regulation or enhancement of antigen function (preferably B lymphocyte antigen function) may be useful in the induction of tumor immunity. Tumor cells (e.g., sarcoma, melanoma, lymphoma, leukemia, neuroblastoma, carcinoma) transfected with a nucleic acid encoding at least one peptide of the present invention can be administered to a subject to overcome tumor-specific tolerance in the subject. If desired, the tumor cell can be transfected to express a combination of peptides. For example, tumor cells obtained from a patient can be transfected ex vivo with an expression vector directing the expression of a peptide having B7-2-like activity alone, or in conjunction with a peptide having B7-1-like activity and/or B7-3-like activity. The transfected tumor cells are returned to the patient to result in expression of the peptides on the surface of the transfected cell. Alternatively, gene therapy techniques can be used to target a tumor cell for transfection in vivo.

The presence of a peptide having the activity of a B lymphocyte antigen(s) on the surface of the tumor cell provides the necessary costimulation signal to T cells to induce a T cell mediated immune response against the transfected tumor cells. In addition, tumor cells which lack MHC class I or MHC class II molecules, or which fail to reexpress sufficient amounts of MHC class I or MHC class II molecules, can be transfected with nucleic acids encoding all or a portion of (e.g., a cytoplasmic-domain truncated portion) of an MHC class I a chain protein and β₂ macroglobulin protein or an MHC class II a chain protein and an MHC class II β chain protein to thereby express MHC class I or MHC class II proteins on the cell surface. Expression of the appropriate class II or class II MHC in conjunction with a peptide having the activity of a B lymphocyte antigen (e.g.. B7-1, B7-2, B7-3) induces a T cell mediated immune response against the transfected tumor cell. Optionally, a gene encoding an antisense construct which blocks expression of an MHC class II associated protein, such as the invariant chain,can also be cotransfected with a DNA encoding a peptide having the activity of a B lymphocyte antigen to promote presentation of tumor associated antigens and induce tumor specific immunity. Thus, the induction of a T cell mediated immune response in a human subject may be sufficient to overcome tumor-specific tolerance in the subject. Alternatively, as described in more detail below, genes encoding these proteins or nucleic acids regulating the expression of these proteins may be introduced into appropriate host cells to increase or decrease the expression of the proteins as desired.

### EXAMPLE 34

### Assaying the Proteins Expressed from Extended cDNAs or Portions Thereof for Hematopoiesis Regulating Activity

The proteins encoded by the extended cDNAs or portions thereof may also be evaluated for their hematopoiesis regulating activity. For example, the effect of the proteins on embryonic stem cell differentiation may be evaluated. Numerous assays for such activity are familiar to those skilled in the art, including the assays disclosed in the following references: Johansson et al. Cellular Biology 15:141-151, 1995; Keller et al., Molecular and Cellular Biology 13:473-486, 1993; McClanahan et al., Blood 81:2903-2915, 1993.

The proteins encoded by the extended cDNAs or portions thereof may also be evaluated for their influence on the lifetime of stem cells and stem cell differentiation. Numerous assays for such activity are familiar to those skilled in the art, including the assays disclosed in the following references: Freshney, M.G. Methylcellulose Colony Forming Assays, in Culture of Hematopoietic Cells. R.I. Freshney, et al. Eds. pp. 265-268, Wiley-Liss, Inc., New York, NY. 1994; Hirayama et al., Proc. Natl. Acad. Sci. USA 89:5907-5911, 1992; McNiece, I.K. and Briddell, R.A. Primitive Hematopoietic Colony Forming Cells with High Proliferative Potential, in Culture of Hematopoietic Cells. R.I. Freshney, et al. eds. Vol pp. 23-39. Wiley-Liss, Inc., New York, NY. 1994; Neben et al., Experimental Hematology 22:353-359, 1994; Ploemacher, R.E. Cobblestone Area Forming Cell Assay, In Culture of Hematopoietic Cells. R.I. Freshney, et al. Eds. pp. 1-21, Wiley-Liss, Inc., New York, NY. 1994; Spooncer, E., Dexter. M. and Allen, T. Long Term Bone Marrow Cultures in the Presence of Stromal Cells, in Culture of Hematopoietic Cells. R.I. Freshney, et al. Eds. pp. 163-179, Wiley-Liss, Inc., New York, NY. 1994; and Sutherland, H.J. Long Term Culture Initiating Cell Assay, in Culture of Hematopoietic Cells. R.I. Freshney, et al Eds. pp. 139-162, Wiley-Liss, Inc., New York, NY. 1994.

Those proteins which exhibit hematopoiesis regulatory activity may then be formulated as pharmaceuticals and used to treat clinical conditions in which regulation of hematopoeisis is beneficial. For example, a protein may be useful in regulation of hematopoiesis and, consequently, in the treatment of myeloid or lymphoid cell deficiencies. Even marginal biological activity in support of colony forming cells or of factor-dependent cell lines indicates involvement in regulating hematopoiesis, e.g. in supporting the growth and proliferation of erythroid progenitor cells alone or in combination with other cytokines, thereby indicating utility, for example, in treating various anemias or for use in conjunction with irradiation/chemotherapy to stimulate the production of erythroid precursors and/or erythroid cells; in supporting the growth and proliferation of myeloid cells such as granulocytes and monocytes/macrophages (i.e.. traditional CSF activity) useful, for example, in conjunction with chemotherapy to prevent or treat consequent myelo-suppression; in supporting the growth and proliferation of megakaryocytes and consequently of platelets thereby allowing prevention or treatment of various platelet disorders such as thrombocytopenia, and generally for use in place of or complimentary to platelet transfusions; and/or in supporting the growth and proliferation of hematopoietic stem cells which are capable of maturing to any and all of the above-mentioned hematopoietic cells and therefore find therapeutic utility in various stem cell disorders (such as those usually treated with transplantion, including, without limitation, aplastic anemia and paroxysmal nocturnal hemoglobinuria), as well as in repopulating the stem cell compartment post irradiation/chemotherapy, either in-vivo or ex-vivo (i.e., in conjunction with bone marrow transplantation or with peripheral progenitor cell transplantation (homologous or heterologous)) as normal cells or genetically manipulated for gene therapy. Alternatively, as described in more detail below, genes encoding these proteins or nucleic acids regulating the expression of these proteins may be introduced into appropriate host cells to increase or decrease the expression of the proteins as desired.

### EXAMPLE 35

### Assaying the Proteins Expressed from Extended cDNAs or Portions Thereof for Regulation of Tissue Growth

The proteins encoded by the extended cDNAs or portions thereof may also be evaluated for their effect on tissue growth. Numerous assays for such activity are familiar to those skilled in the art, including the assays disclosed in International Patent Publication No. WO95/16035, International Patent Publication No. WO95/05846 and International Patent Publication No. WO91/07491.

Assays for wound healing activity include, without limitation, those described in: Winter. Epidermal Wound Healing, pps. 71-112 (Maibach, H1 and Rovee, DT, eds.). Year Book Medical Publishers, Inc., Chicago, as modified by Eaglstein and Mertz. J. Invest. Dermatol 71:382-84 (1978).

Those proteins which are involved in the regulation of tissue growth may then be formulated as pharmaceuticals and used to treat clinical conditions in which regulation of tissue growth is beneficial. For example, a protein also may have utility in compositions used for bone, cartilage, tendon, ligament and/or nerve tissue growth or regeneration, as well as for wound heating and tissue repair and replacement, and in the treatment of burns, incisions and ulcers.

A protein which induces cartilage and/or bone growth in circumstances where bone is not normally formed, has application in the healing of bone fractures and cartilage damage or defects in humans and other animals. Such a preparation employing a protein may have prophylactic use in closed as well as open fracture reduction and also in the improved fixation of artificial joints. De novo bone formation induced by an osteogenic agent contributes to the repair of congenital, trauma induced, or oncologic resection induced craniofacial defects, and also is useful in cosmetic plastic surgery.

A protein may also be used in the treatment of periodontal disease, and in other tooth repair processes. Such agents may provide an environment to attract bone-forming cells, stimulate growth of bone-forming cells or induce differentiation of progenitors of bone-forming cells. A protein may also be useful in the treatment of osteoporosis or osteoarthritis, such as through stimulation of bone and/or cartilage repair or by blocking inflammation or processes of tissue destruction (collagenase activity, osteoclast activity, etc.) mediated by inflammatory processes.

Another category of tissue regeneration activity that may be attributable to a protein is tendon/ligament formation. A protein which induces tendon/ligament-like tissue or other tissue formation in circumstances where such tissue is not normally formed, has application in the healing of tendon or ligament tears, deformities and other tendon or ligament defects in humans and other animals. Such a preparation employing a tendon/ligament-like tissue inducing protein may have prophylactic use in preventing damage to tendon or ligament tissue, as well as use in the improved fixation of tendon or ligament to bone or other tissues, and in repairing defects to tendon or ligament tissue. De novo tendon/ligament-like tissue formation induced by a composition contributes to the repair of congenital, trauma induced, or other tendon or ligament defects of other origin, and is also useful in cosmetic plastic surgery for attachment or repair of tendons or ligaments. The compositions may provide an environment to attract tendon- or ligament-forming cells, stimulate growth of tendon- or ligament-forming cells, induce differentiation of progenitors of tendon- or ligament-forming cells, or induce growth of tendon/ligament cells or progenitors ex vivo for return in vivo to effect tissue repair. The compositions may also be useful in the treatment of tendinitis, carpal tunnel syndrome and other tendon or ligament defects. The compositions may also include an appropriate matrix and/or sequestering agent as a carrier as is well known in the art.

A protein may also be useful for proliferation of neural cells and for regeneration of nerve and brain tissue, i.e.. for the treatment of central and peripheral nervous system diseases and neuropathies, as well as mechanical and traumatic disorders, which involve degeneration, death or trauma to neural cells or nerve tissue. More specifically, a protein may be used in the treatment of diseases of the peripheral nervous system, such as peripheral nerve injuries, peripheral neuropathy and localized neuropathies, and central nervous system diseases, such as Alzheimer's, Parkinson's disease. Huntington's disease, amyotrophic lateral sclerosis, and Shy-Dragef syndrome. Further conditions which may be treated include mechanical and traumatic disorders, such as spinal cord disorders, head trauma and cerebrovascular diseases such as stroke. Peripheral neuropathies resulting from chemotherapy or other medical therapies may also be treatable using a protein.

Proteins may also be useful to promote better or faster closure of non-healing wounds, including without limitation pressure ulcers, ulcers associated with vascular insufficiency, surgical and traumatic wounds, and the like.

It is expected that a protein may also exhibit activity for generation or regeneration of other tissues, such as organs (including, for example, pancreas, liver, intestine, kidney, skin, endothelium) muscle (smooth, skeletal or cardiac) and vascular (including vascular endothelium) tissue, or for promoting the growth of cells comprising such tissues. Part of the desired effects may be by inhibition or modulation of fibrotic scarring to allow normal tissue to generate. A protein may also exhibit angiogenic activity.

A protein may also be useful for gut protection or regeneration and treatment of lung or liver fibrosis, reperfusion injury in various tissues, and conditions resulting from systemic cytokinc damage.

A protein may also be useful for promoting or inhibiting differentiation of tissues described above from precursor tissues or cells; or for inhibiting the growth of tissues described above.

Alternatively, as described in more detail below, genes encoding these proteins or nucleic acids regulating the expression of these proteins may be introduced into appropriate host cells to increase or decrease the expression of the proteins as desired.

### EXAMPLE 36

### Assaying the Proteins Expressed from Extended cDNAs or Portions Thereof for Regulation of Reproductive Hormones or Cell Movement

The proteins encoded by the extended cDNAs or portions thereof may also be evaluated for their ability to regulate reproductive hormones, such as follicle stimulating hormone. Numerous assays for such activity are familiar to those skilled in the art, including the assays disclosed in the following references: Vale et al., Endocrinology 91:562-572, 1972; Ling et al., Nature 321:779-782, 1986; Vale et al., Nature 321:776-779, 1986; Mason et al., Nature 318:659-663, 1985; Forage et al., Proc. Natl. Acad. Sci. USA 83:3091-3095, 1986. Chapter 6.12 (Measurement of Alpha and Beta Chemokines) Current Protocols in Immunology, J.E. Coligan et al. Eds. Greene Publishing Associates and Wiley-Intersciece : Taub et al. J. Clin. Invest. 95:1370-1376, 1995; Lind et al. APMIS 103:140-146, 1995; Muller et al. Eur. J. Immunol. 25:1744-1748; Gruber et al. J. of Immunol. 152:5860-5867, 1994; Johnston et al. J. of Immunol. 153:1762-1768, 1994.

Those proteins which exhibit activity as reproductive hormones or regulators of cell movement may then be formulated as pharmaceuticals and used to treat clinical conditions in which regulation of reproductive hormones or cell movement are beneficial. For example, a protein may also exhibit activin- or inhibin-related activities. Inhibins are characterized by their ability to inhibit the release of follicle stimulating hormone (FSH), while activins are characterized by their ability to stimulate the release of folic stimulating hormone (FSH). Thus, a protein alone or in heterodimers with a member of the inhibin a family, may be useful as a contraceptive based on the ability of inhibins to decrease fertility in female mammals and decrease spermatogenesis in male mammals. Administration of sufficient amounts of other inhibins can induce infertility in these mammals. Alternatively, the protein , as a homodimer or as a heterodimer with other protein subunits of the inhibin-B group, may be useful as a fertility inducing therapeutic, based upon the ability of activin molecules in stimulating FSH release from cells of the anterior pituitary. See, for example, United States Patent 4,798,885. A protein may also be useful for advancement of the onset of fertility in sexually immature mammals, so as to increase the lifetime reproductive performance of domestic animals such as cows, sheep and pigs.

Alternatively, as described in more detail below, genes encoding these proteins or nucleic acids regulating the expression of these proteins may be introduced into appropriate host cells to increase or decrease the expression of the proteins as desired.

### EXAMPLE 36A

### Assaying the Proteins Expressed from Extended cDNAs or Portions Thereof for Chemotaclic/Chemokinetic Activity

The proteins encoded by the extended cDNAs or portions thereof may also be evaluated for chemotactic/chemokinetic activity. For example, a protein may have chemotactic or chemokinetic activity (e.g., act as a chemokine) for mammalian cells, including, for example, monocytes, fibroblasts, neutrophils, T-cells, mast cells, cosinophils, epithelial and/or endothelial cells. Chemotactic and chmekinetic proteins can be used to mobilize or attract a desired cell population to a desired site of action. Chemotactic or chemokinetic proteins provide particular advantages in treatment of wounds and other trauma to tissues, as well as in treatment of localized infections. For example, attraction of lymphocytes, monocytes or neutrophils to tumors or sites of infection may result in improved immune responses against the tumor or infecting agent.

A protein or peptide has chemotactic activity for a particular cell population if it can stimulate, directly or indirectly, the directed orientation or movement of such cell population. Preferably, the protein or peptide has the ability to directly stimulate directed movement of cells. Whether a particular protein has chemotactic activity for a population of cells can be readily determined by employing such protein or peptide in any known assay for cell chemotaxis.

The activity of a protein of the invention may, among other means, be measured by the following methods: Assays for chemotactic activity (which will identify proteins that induce or prevent chemotaxis) consist of assays that measure the ability of a protein to induce the migration of cells across a membrane as well as the ability of a protein to induce the adhension of one cell population to another cell population. Suitable assays for movement and adhesion include, without limitation, those described in: Current Protocols in Immunology, Ed by J.E. Coligan, A.M. Kruisbeek, D.H. Margulies, E.M. Shevach, W. Strober, Pub. Greene Publishing Associates and Wiley-Interscience (Chapter 6.12, Measurement of alpha and beta Chemokines 6.12.1-6.12.28; Taub et al. J. Clin. Invest. 95:1370-1376, 1995; Lind et aL APMIS 103:140-146, 1995; Mueller et al Eur. J. Immunol. 25:1744-1748; Gruber et al. J. of Immunol. 152:5860-5867, 1994; Johnston et al. J. of Immunol, 153:1762-1768, 1994.

### EXAMPLE 37

### Assaying the Proteins Expressed from Extended cDNAs or Portions Thereof for Regulation of Blood Clotting

The proteins encoded by the extended cDNAs or portions thereof may also be evaluated for their effects on blood clotting. Numerous assays for such activity are familiar to those skilled in the art, including the assays disclosed in the following references: Linet et al. J. Clin. Pharmacol. 26:131-140, 1986; Burdick et al., Thrombosis Res. 45:413-419, 1987; Humphrey et al., Fibrinolysis 5:71-79 (1991); Schaub, Prostaglandins 35:467-474, 1988.

Those proteins which are involved in the regulation of blood clotting may then be formulated as pharmaceuticals and used to treat clinical conditions in which regulation of blood clotting is beneficial. For example, a protein may also exhibit hemostatic or thrombolytic activity. As a result, such a protein is expected to be useful in treatment of various coagulations disorders (including hereditary disorders, such as hemophilias) or to enhance coagulation and other hemostatic events in treating wounds resulting from trauma, surgery or other causes. A protein may also be useful for dissolving or inhibiting formation of thromboses and for treatment and prevention of conditions resulting therefrom (such as, for example, infarction of cardiac and central nervous system vessels (e.g. stroke). Alternatively, as described in more detail below, genes encoding these proteins or nucleic acids regulating the expression of these proteins may be introduced into appropriate host cells to increase or decrease the expression of the proteins as desired.

### EXAMPLE 38

### Assaying the Proteins Expressed from Extended cDNAs or Portions Thereof for Involvement in Receptor/Ligand Interactions

The proteins encoded by the extended cDNAs or a portion thereof may also be evaluated for their involvement in receptor/ligand interactions. Numerous assays for such involvement are familiar to those skilled in the art, including the assays disclosed in the following references: Chapter 7.28 (Measurement of Cellular Adhesion under Static Conditions 7.28.1-7.28.22) in Current Protocols in Immunology, J.E. Coligan et al. Eds. Greene Publishing Associates and Wiley-Interscience; Takai et al., Proc. Natl. Acad. Sci. USA 84:6864-6868, 1987; Bierer et al., J. Exp. Med. 168:1145-1156, 1988; Rosenstein et al., J. Exp. Med. 169:149-160, 1989; Stoltenborg et al., J. Immunol. Methods 175:59-68, 1994; Stitt et al., Cell 80:661-670, 1995; Gyuris et al., Cell 75:791-803, 1993.

For example, the proteins may also demonstrate activity as receptors, receptor ligands or inhibitors or agonists of receptor/ligand interactions. Examples of such receptors and ligands include, without limitation, cytokine receptors and their ligands, receptor kinases and their ligands, receptor phosphatases and their ligands, receptors involved in cell-cell interactions and their ligands (including without limitation, cellular adhesion molecules (such as selectins, integrins and their ligands) and receptor/ligand pairs involved in antigen presentation, antigen recognition and development of cellular and humoral immune respones). Receptors and ligands are also useful for screening of potential peptide or small molecules inhibitors of the relevant receptor/ligand interaction. A protein (including, without limitation, fragments of receptors and ligands) may themselves be useful as inhibitors of receptor/ligand interactions.

### EXAMPLE 38A

### Assaying the Proteins Expressed from Extended cDNAs or Portions Thereof for Anti-Inflamrnalary Activity

The proteins encoded by the extended cDNAs or a portion thereof may also be evaluated for anti-inflammatory activity. The anti-inflammatory activity may be achieved by providing a stimulus to cells involved in the inflammatory response, by inhibiting or promoting cell-cell interactions (such as, for example, cell adhesion), by inhibiting or promoting chemotaxis of cells involved in the inflammatory process, inhibiting or promoting cell extravasation, or by stimulating or suppressing production of other factors which more directly inhibit or promote an inflammatory response. Proteins exhibiting such activities can be used to treat inflammatory conditions including chronic or acute conditions), including without limitation inflammation associated with infection (such as septic shock, sepsis or systemic inflammatory response syndrome (SIRS)),ischemia reperfusioninury, endotoxin lethality, arthritis, complement-mediated hyperacute rejection, nephritis, cytokine or chemokine-induced lung injury, inflammatory bowel disease, Crohn's disease or resulting from over production of cytokines such as TNF or IL-1. Proteins may also be useful to treat anaphylaxis and hypersensitivity to an antigenic substance or material.

### EXAMPLE 38B

### Assaying the Proteins Expressed from Extended cDNAs or Portions Thereof for Tumor Inhibition Activity

The proteins encoded by the extended cDNAs or a portion thereof may also be evaluated for tumor inhibition activity. In addition to the activities described above for immunological treatment or prevention of tumors, a protein may exhibit other anti-tumor activities. A protein may inhibit tumor growth directly or indirectly (such as, for example, via ADCC). A protein may exhibit its tumor inhibitory activity by acting on tumor tissue or tumor precursor tissue, by inhibiting formation of tissues necessary to support tumor growth (such as, for example, by inhibiting angiogenesis), by causing production of other factors, agents or cell types which inhibit tumor growth, or by suppressing, climinating or inhibiting factors, agents or cell types which promote tumor growth.

A protein may also exhibit one or more of the following additional activities or effects: inhibiting the growth, infection or function of, or killing, infectious agents, including, without limitation, bacteria, viruses, fungi and other parasites: effecting (suppressing or enhancing) bodily characteristics, including, without limitation, height, weight, hair color, eye color, skin, fat to lean ratio or other tissue pigmentation, or organ or body part size or shape (such as, for example, breast augmentation or diminution, change in bone form or shape); effecting biorhythms or circadian cycles or rhythms; effecting the fertility of male or female subjects; effecting the metabolism, catabolism, anabolism, processing, utilization, storage or climination of dietary fat, lipid, protein, carbohydrate, vitamins, minerals, cofactors or other nutritional factors or component(s); effecting behavioral characteristics, including, without limitation, appetite, libido, stress, cognition (including cognitive disorders), depression (including depressive disorders) and violent behaviors; providing analgesic effects or other pain reducing effects; promoting differentiation and growth of embryonic stem cells in lineages other than hematopoietic lineages; hormonal or endocrine activity: in the case of enzymes, correcting deficiencies of the enzyme and treating deficiency-related diseases: treatment of hyperproliferative disorders (such as, for example, psoriasis); immunoglobulin-like activity (such as, for example, the ability to bind antigens or complement); and the ability to act as an antigen in a vaccine composition to raise an immune response against such protein or another material or entity which is cross-reactive with such protein.

### EXAMPLE 39

### Identification of Proteins which Interact with Polypeptides Encoded by Extended cDNAs

Proteins which interact with the polypeptides encoded by extended cDNAs or portions thereof, such as receptor proteins, may be identified using two hybrid systems such as the Matchmaker Two Hybrid System 2 (Catalog No. K1604-1, Clontech). As described in the manual accompanying the Matchmaker Two Hybrid System 2 (Catalog No. K1604-1, Clontech), the extended cDNAs or portions thereof, are inserted into an expression vector such that they are in frame with DNA encoding the DNA binding domain of the yeast transcriptional activator GAL4. cDNAs in a cDNA library which encode proteins which might interact with the polypeptides encoded by the extended cDNAs or portions thereof are inserted into a second expression vector such that they are in frame with DNA encoding the activation domain of GAL4. The two expression plasmids are transformed into yeast and the yeast are plated on selection medium which selects for expression of selectable markers on each of the expression vectors as well as GAL4 dependent expression of the HIS3 gene. Transformants capable of growing on medium lacking histidine are screened for GAL4 dependent lacZ expression. Those cells which are positive in both the histidine selection and the lacZ assay contain plasmids encoding proteins which interact with the polypeptide encoded by the extended cDNAs or portions thereof.

Alternatively, the system described in Lustig et al., Methods in Enzymology 283: 83-99 (1997) may be used for identifying molecules which interact with the polypeptides encoded by extended cDNAs. In such systems, *in vitro* transcription reactions are performed on a pool of vectors containing extended cDNA inserts cloned downstream of a promoter which drives *in vitro* transcription. The resulting pools of mRNAs are introduced into *Xenopus laevis* oocytes. The oocytes are then assayed for a desired acitivity.

Alternatively, the pooled *in vitro* transcription products produced as described above may be translated *in vitro.* The pooled *in vitro* translation products can be assayed for a desired activity or for interaction with a known polypeptide.

Proteins or other molecules interacting with polypeptides encoded by extended cDNAs can be found by a variety of additional techniques. In one method, affinity columns containing the polypeptide encoded by the extended cDNA or a portion thereof can be constructed. In some versions, of this method the affinity column contains chimeric, proteins in which the protein encoded by the extended cDNA or a portion thereof is fused to glutathione S-transferase, A mixture of cellular proteins or pool of expressed proteins as described above and is applied to the affinity column. Proteins interacting with the polypeptide attached to the column can then be isolated and analyzed on 2-D electrophoresis gel as described in Ramunsen et al. Electrophoresis, 18, 588-598 (1997). Alternatively, the proteins retained on the affinity column can be purified by electrophoresis based methods and sequenced. The same method can be used to isolate antibodies, to screen phage display products, or to screen phage display human antibodies.

Proteins interacting with polypeptides encoded by extended cDNAs or portions thereof can also be screened by using an Optical Biosensor as described in Edwards & Leatherbarrow, Analytical Biochemistry, 246, 1-6 (1997). The main advantage of the method is that it allows the determination of the association rate between the protein and other interacting molecules. Thus, it is possible to specifically select interacting molecules with a high or low association rate. Typically a target molecule is linked to the sensor surface (through a carboxymethl dextran matrix) and a sample of test molecules is placed in contact with the target molecules. The binding of a test molecule to the target molecule causes a change in the refractive index and/ or thickness. This change is detected by the Biosensor provided it occurs in the evanescent field (which extend a few hundred manometers from the sensor surface). In these screening assays, the target molecule can be one of the polypeptides encoded by extended cDNAs or a portion thereof and the test sample can be a collection of proteins extracted from tissues or cells, a pool of expressed proteins, combinatorial peptide and/ or chemical libraries,or phage displayed peptides. The tissues or cells from which the test proteins are extracted can originate from any species.

In other methods, a target protein is immobilized and the test population is a collection of unique polypeptides encoded by the extended cDNAs or portions thereof.

To study the interaction of the proteins encoded by the extended cDNAs or portions thereof with drugs, the microdialysis coupled to HPLC method described by Wang et al., Chromatographia, 44, 205-208(1997) or the affinity capillary electrophoresis method described by Busch et al., J. Chromatogr. 777:311-328 (1997), can be used.

The system described in U.S. Patent No. 5,654.150 may also be used to identify molecules which interact with the polypeptides encoded by the extended cDNAs. In this system, pools of extended cDNAs are transcribed and translated *in vitro* and the reaction products are assayed for interaction with a known polypeptide or antibody.

It will be appreciated by those skilled in the art that the proteins expressed from the extended cDNAs or portions may be assayed for numerous activities in addition to those specifically enumerated above. For example, the expressed proteins may be evaluated for applications involving control and regulation of inflammation, tumor proliferation at metastasis, infection, or other clinical conditions. In addition, the proteins expressed from the extended cDNAs or portions thereof may be useful as nutritional agents or cosmetic agents.

The proteins expressed from the extended cDNAs or portions thereof may be used to generate antibodies capable of specifically binding to the expressed protein or fragments thereof as described in Example 40 below. The antibodies may be capable of binding a full length protein encoded by the sequence of SEQ ID NOs : 45, a mature protein encoded by the sequence of SEQ ID NO. 45, or a signal peptide encoded by the sequence of SEO 10 No. 45. Alternatively, the antibodies may be capable of binding fragments of the proteins expressed from the extended cDNAs which comprise at least 10 amino acids of the sequence of SEO ID NO: 146. The antibodies may be capable of binding fragments of the proteins expressed from the extended cDNAs which comprise at least 15 amino acids of the sequence of SEQ ID NO: 146. The antibodies may be capable of binding fragments of the proteins expressed from the extended cDNAs which comprise at least 25 amino acids of the sequence of SEQ ID NOs: 146. In further embodiments, the antibodies may be capable of binding fragments of the proteins expressed from the extended cDNAs which comprise at least 40 amino acids of the sequence of SEO ID NO: 146.

### EXAMPLE 40

### Production of an Antibody to a Human Protein

Substantially pure protein or polypeptide is isolated from the transfected or transformed cells as described in Example 30. The concentration of protein in the final preparation is adjusted, for example, by concentration on an Amicon filter device, to the level of a few micrograms/ml. Monoclonal or polyclonal antibody to the protein can then be prepared as follows:

### A. Monoclonal Antibody Production by Hybridoma Fusion

Monoclonal antibody to epitopes of any of the peptides identified and isolated as described can be prepared from murine hybridomas according to the classical method of Kohler, G. and Milstein. C., Nature 256:495 (1975) or derivative methods thereof. Briefly, a mouse is repetitively inoculated with a few micrograms of the selected protein or peptides derived therefrom over a period of a few weeks. The mouse is then sacrificed, and the antibody producing cells of the spleen isolated. The spleen cells are fused by means of polyethylene glycol with mouse myeloma cells, and the excess unfused cells destroyed by growth of the system on selective media comprising aminopterin (HAT media). The successfully fused cells are diluted and aliquots of the dilution placed in wells of a microtiter plate where growth of the culture is continued. Antibody producing clones are identified by detection of antibody in the supernatant fluid of the wells by immunoassay procedures, such as Elisa, as originally described by Engvall, E., Meth. Enzymol. 70:419 (1980), and derivative methods thereof. Selected positive clones can be expanded and their monoclonal antibody product harvested for use. Detailed procedures for monoclonal antibody production are described in Davis, L. et al. Basic Methods in Molecular Biology Elsevier, New York. Section 21-2.

### B. Polyclonal Antibody Production by Immunization

Polyclonal antiserum containing antibodies to heterogenous epitopes of a single protein can be prepared by immunizing suitable animals with the expressed protein or peptides derived therefrom described above, which can be unmodified or modified to enhance immunogerocity. Effective polyclonal antibody production is affected by many factors related both to the antigen and the host species, For example, small molecules tend to be less immunogenic than others and may require the use of carriers and adjuvant. Also, host animals vary in response to site of inoculations and dose, with both inadequate or excessive doses of antigen resulting in low titer antisera. Small doses (ng level) of antigen administered at multiple intradermal sites appears to be most reliable. An effective immunization protocol for rabbits can be found in Vaitukaitis, J. et al. J. Clin. Endocrinol. Metab. 33:988-991 (1971).

Booster injections can be given at regular intervals, and antiserum harvested when antibody titer thereof, as determined semi-quantitatively, for example, by double immunodiffusion in agar against known concentrations of the antigen, begins to fall. See, for example. Ouchterlony, D. et al., Chap.19 in: Handbook of Experimental Immunology' O. Wier (ed) Blackwell (1973). Plateau concentration of antibody is usually in the range of 0.1 to 0.2 mg/ml of serum (about 12 µM). Affinity of the antisera for the antigen is determined by preparing competitive binding curves, as described, for example, by Fisher, D., Chap. 42 in: Manual of Clinical Immunology. 2d Ed. (Rose and Friedman, Eds.) Amer. Soc. For Microbiol., Washington, D.C. (1980).

Antibody preparations prepared according to either protocol are useful in quantitative immunoassays which determine concentrations of antigen-bearing substances in biological samples; they are also used semi-quantitatively or qualitatively to identify the presence of antigen in a biological sample. The antibodies may also be used in therapeutic compositions for killing cells expressing the protein or reducing the levels of the protein in the body.

### V. Use of Extended cDNAs or Portions Thereof as Reagents

The extended cDNAs described herein may be used as reagents in isolation procedures, diagnostic assays, and forensic procedures. For example, sequences from the extended cDNAs (or genomic DNAs obtainable therefrom) may be detectably labeled and used as probes to isolate other sequences capable of hybridizing to them. In addition, sequences from the extended cDNAs (or genomic DNAs obtainable therefrom) may be used to design PCR primers to be used in isolation, diagnostic, or forensic procedures.

### EXAMPLE 41

### Preparation of PCR Primers and Amplification of DNA

The extended cDNAs (or genomic DNAs obtainable therefrom) may be used to prepare PCR primers for a variety of applications, including isolation procedures for cloning nucleic acids capable of hybridizing to such sequences, diagnostic techniques and forensic techniques. The PCR primers are at least 10 bases, and preferably at least 12, 15, or 17 bases in length. More preferably, the PCR primers are at least 20-30 bases in length. In some embodiments, the PCR primers may be more than 30 bases in length. It is preferred that the primer pairs have approximately the same G/C ratio, so that melting temperatures are approximately the same. A variety of PCR techniques are familiar to those skilled in the art. For a review of PCR technology, see Molecular Cloning to Genetic Engineering White, B.A. Ed. in Methods in Molecular Biology 67: Humana Press, Totowa 1997. In each of these PCR procedures, PCR primers on either side of the nucleic acid sequences to be amplified are added to a suitably prepared nucleic acid sample along with dNTPs and a thermostable polymerase such as Taq polymerase. Pfu polymerase, or Vent polymerase. The nucleic acid in the sample is denatured and the PCR primers are specifically hybridized to complementary nucleic acid sequences in the sample. The hybridized primers are extended. Thereafter, another cycle of denaturation, hybridization, and extension is initiated. The cycles are repeated multiple times to produce an amplified fragment containing the nucleic acid sequence between the primer sites.

### EXAMPLE 42

### Use of Extended cDNAs as Probes

Probes derived from extended cDNAs or portions thereof (or genomic DNAs obtainable therefrom) may be labeled with detectable labels familiar to those skilled in the art, including radioisotopes and non radioactive labels, to provide a detectable probe. The detectable probe may be single stranded or double stranded and may be made using techniques known in the art, including in vitro transcription, nick translation, or kinase reactions. A nucleic acid sample containing a sequence capable of hybridizing to the labeled probe is contacted with the labeled probe. If the nucleic acid in the sample is double stranded, it may be denatured prior to contacting the probe. In some applications, the nucleic acid sample may be immobilized on a surface such as a nitrocellulose or nylon membrane. The nucleic acid sample may comprise nucleic acids obtained from a variety of sources, including genomic DNA, cDNA libraries, RNA, or tissue samples.

Procedures used to detect the presence of nucleic acids capable of hybridizing to the detectable probe include well known techniques such as Southern blotting, Northern blotting, dot blotting, colony hybridization, and plaque hybridization. In some applications, the nucleic acid capable of hybridizing to the labeled probe may be cloned into vectors such as expression vectors, sequencing vectors, or in vitro transcription vectors to facilitate the characterization and expression of the hybridizing nucleic acids in the sample. For example, such techniques may be used to isolate and clone sequences in a genomic library or cDNA library which are capable of hybridizing to the detectable probe as described in Example 30 above.

PCR primers made as described in Example 41 above may be used in forensic analyses, such as the DNA fingerprinting techniques described in Examples 43.47 below. Such analyses may utilize detectable probes or primers based on the sequences of the extended cDNAs isolated using the 5' ESTs (or genomic DNAs obtainable therefrom)..

### EXAMPLE 43

### Forensic Matching by DNA Sequencing

In one exemplary method, DNA samples are isolated from forensic specimens of, for example, hair, semen, blood or skin cells by conventional methods. A panel of PCR primers based on a number of the extended cDNAs (or genomic DNAs obtainable therefrom), is then utilized in accordance with Example 41 to amplify DNA of approximately 100-200 bases in length from the forensic specimen Corresponding sequences are obtained from a test subject. Each of these identification DNAs is then sequenced using standard techniques, and a simple database comparison determines the differences, if any, between the sequences from the subject and those from the sample. Statistically significant differences between the suspect's DNA sequences and those from the sample conclusively prove a lack of identity. This lack of identity can be proven, for example, with only one sequence. Identity, on the other hand, should be demonstrated with a large number of sequences, all matching. Preferably, a minimum of 50 statistically identical sequences of 100 bases in length are used to prove identity between the suspect and the sample.

### EXAMPLE 44

### Positive Identification by DNA Sequencing

The technique outlined in the previous example may also be used on a larger scale to provide a unique fingerprint-type identification of any individual. In this technique, primers are prepared from a large number of sequences identified herein. Preferably. 20 to 50 different primers are used. These primers are used to obtain a corresponding number of PCR-generaled DNA segments from the individual in question in accordance with Example 41. Each of these DNA segments is sequenced, using the methods set forth in Example 43. The database of sequences generated through this procedure uniquely identifies the individual from whom the sequences were obtained. The same panel of primers may then be used at any later time to absolutely correlate tissue or other biological specimen with that individual.

### EXAMPLE 45

### Southern Blot Forensic Identification

The procedure of Example 44 is repeated to obtain a panel of at least 10 amplified sequences from an individual and a specimen. Preferably, the panel contains at least 50 amplified sequences. More preferably, the panel contains 100 amplified sequences. In some embodiments, the panel contains 200 amplified sequences. This PCR-generated DNA is then digested with one or a combination of, preferably, four base specific restriction enzymes. Such enzymes are commercially available and known to those of skill in the art. After digestion, the resultant gene fragments are size separated in multiple duplicate wells on an agarose gel and transferred to nitrocellulose using Southern blotting techniques well known to those with skill in the art. For a review of Southern blotting see Davis et at. (Basic Methods in Molecular Biology, 1986, Elsevier Press. pp 62-65).

A panel of probes based on the sequences of the extended cDNAs (or genomic DNAs obtainable therefrom), or fragments thereof of at least 10 bases, are radioactively or colorimetrically labeled using methods known in the art, such as nick translation or end labeling, and hybridized to the Southern blot using techniques known in the art (Davis et al., supra). Preferably, the probe comprises at least 12, 15, or 17 consecutive nucleotides from the extended cDNA (or genomic DNAs obtainable therefrom). More preferably, the probe comprises at least 20-30 consecutive nucleotides from the extended cDNA (or genomic DNAs obtainable therefrom). In some embodiments, the probe comprises more than 30 nucleotides from the extended cDNA (or genomic DNAs obtainable therefrom). In other embodiments, the probe comprises at least 40, at least 50, at least 75, at least 100, at least 150, or at least 200 consecutive nucleotides from the extended cDNA (or genomic DNAs obtainable therefrom).

Preferably, at least 5 to 10 of these labeled probes are used, and more preferably at least about 20 or 30 are used to provide a unique pattern. The resultant bands appearing from the hybridization of a large sample of extended cDNAs (or genomic DNAs obtainable therefrom) will be a unique identifier. Since the restriction enzyme cleavage will be different for every individual, the band pattern on the Southern blot will also be unique. Increasing the number of extended cDNA probes will provide a statistically higher level of confidence in the identification since there will be an increased number of sets of bands used for identification.

### EXAMPLE 46

### Dot Blot Identification Procedure

Another technique for identifying individuals using the extended cDNA sequences disclosed herein utilizes a dot blot hybridization technique.

Genomic DNA is isolated from nuclei of subject to be identified. Oligonucleotide probes of approximately 30 bp in length are synthesized that correspond to at least 10, preferably 50 sequences from the extended cDNAs or genomic DNAs obtainable therefrom. The probes are used to hybridize to the genomic DNA through conditions known to those in the art. The oligonucleotides are end labeled with P³² using polynucleotide kinase (Pharmacia). Dot Blots are created by spotting the genomic DNA onto nitrocellulose or the like using a vacuum dot blot manifold (BioRad, Richmond California). The nitrocellulose filter containing the genomic sequences is baked or UV linked to the filter, prehybridized and hybridized with labeled probe using techniques known in the art (Davis et al. supra). The ³²P labeled DNA fragments are sequentially hybridized with successively stringent conditions to detect minimal differences between the 30 bp sequence and the DNA. Tetramethylammonium chloride is useful for identifying clones containing small numbers of nucleotide mismatches (Wood et al., Proc. Natl. Acad. Sci. USA 82(6):1585-1588 (1985)). A unique pattern of dots distinguishes one individual from another individual.

Extended cDNAs or oligonucleotides containing at least 10 consecutive bases from these sequences can be used as probes in the following alternative fingerprinting technique. Preferably, the probe comprises at least 12, 15, or 17 consecutive nucleotides from the extended cDNA (or genomic DNAs obtainable therefrom). More preferably, the probe comprises at least 20-30 consecutive nucleotides from the extended cDNA (or genomic DNAs obtainable therefrom). In some embodiments, the probe comprises more than 30 nucleotides from the extended cDNA (or genomic DNAs obtainable therefrom). In other embodiments, the probe comprises at least 40, at least 50, at least 75, at least 100, at least 150, or at least 200 consecutive nucleotides from the extended cDNA (or genomic DNAs obtainable therefrom).

Preferably, a plurality of probes having sequences from different genes are used in the alternative fingerprinting technique. Example 47 below provides a representative alternative fingerprinting procedure in which the probes are derived from extended cDNAs.

### EXAMPLE 47

### Alternative "Fingerprint" Identification Technique

20-mer oligonucleotides are prepared from a large number, e.g. 50, 100, or 200, of extended cDNA sequences (or genomic DNAs obtainable therefrom) using commercially available oligonucleotide services such as Genset, Paris, France. Cell samples from the test subject are processed for DNA using techniques well known to those with skill in the art. The nucleic acid is digested with restriction enzymes such as EcoRI and Xbal. Following digestion, samples are applied to wells for electrophoresis. The procedure, as known in the art, may be modified to accommodate polyacrylamide electrophoresis, however in this example, samples containing 5 ug of DNA are loaded into wells and separated on 0.8% agarose gels. The gels are transferred onto nitrocellulose using standard Southern blotting techniques.

10 ng of each of the oligonucleotides are pooled and end-labeled with P³². The nitrocellulose is prehybridized with blocking solution and hybridized with the labeled probes. Following hybridization and washing, the nitrocellulose filter is exposed to X-Omat AR X-ray film. The resulting hybridization pattern will be unique for each individual.

It is additionally contemplated within this example that the number of probe sequences used can be varied for additional accuracy or clarity.

The antibodies generated in Examples 30 and 40 above may be used to identify the tissue type or cell species from which a sample is derived as described above.

### EXAMPLE 48

### Identification of Tissue Types or Cell Species by Means of Labeled Tissue Specific Antibodies

Identification of specific tissues is accomplished by the visualization of tissue specific antigens by means of antibody preparations according to Examples 30 and 40 which are conjugated, directly or indirectly to a detectable marker. Selected labeled antibody species bind to their specific antigen binding partner in tissue sections, cell suspensions, or in extracts of soluble proteins from a tissue sample to provide a pattern for qualitative or semi-qualitative interpretation.

Antisera for these procedures must have a potency exceeding that of the native preparation, and for that reason, antibodies are concentrated to a mg/ml level by isolation of the gamma globulin fraction, for example, by ionexchange chromatography or by ammonium sulfate fractionation. Also, to provide the most specific antisera, unwanted antibodies, for example to common proteins, must be removed from the gamma globulin fraction, for example by means of insoluble immunoabsorbents, before the antibodies are labeled with the marker. Either monoclonal or heterologous antisera is suitable for either procedure.

### A. Immunohistochemical Techniques

Purified, high-titer antibodies, prepared as described above, are conjugated to a detectable marker, as described, for example, by Fudenberg, H., Chap. 26 in: Basic 503 Clinical Immunology. 3rd Ed. Lange, Los Altos, California (1980) or Rose, N. et al., Chap. 12 in: Methods in Immunodiagnosis, 2d Ed. John Wiley 503 Sons, New York (1980).

A fluorescent marker, either fluorescein or rhodamine, is preferred, but antibodies can also be labeled with an enzyme that supports a color producing reaction with a substrate, such as horseradish peroxidase. Markers can be added to tissue-bound antibody in a second step, as described below. Alternatively, the specific antitissue antibodies can be labeled with ferritin or other electron dense particles, and localization of the ferritin coupled antigen-antibody complexes achieved by means of an electron microscope. In yet another approach, the antibodies are radiolabeled, with, for example ¹²⁵I, and detected by overlaying the antibody treated preparation with photographic emulsion.

Preparations to carry out the procedures can comprise monoclonal or polyclonal antibodies to a single protein or peptide identified as specific to a tissue type, for example, brain tissue, or antibody preparations to several antigenically distinct tissue specific antigens can be used in panels, independently or in mixtures, as required.

Tissue sections and cell suspensions are prepared for immunohistochemical examination according to common histological techniques. Multiple cryostat sections (about 4 µm, unfixed) of the unknown tissue and known control, are mounted and each slide covered with different dilutions of the antibody preparation. Sections of known and unknown tissues should also be treated with preparations to provide a positive control, a negative control, for example, pre-immune sera, and a control for non-specific staining, for example, buffer.

Treated sections are incubated in a humid chamber for 30 min at room temperature, rinsed, then washed in buffer for 30-45 min. Excess fluid is blotted away, and the marker developed.

If the tissue specific antibody was not labeled in the first incubation, it can be labeled at this time in a second antibody-antibody reaction, for example, by adding fluorescein- or enzyme-conjugated antibody against the immunoglobulin class of the antiserum-producing species, for example, fluorescein labeled antibody to mouse IgG. Such labeled sera are commercially available.

The antigen found in the tissues by the above procedure can be quantified by measuring the intensity of color or fluorescence on the tissue section, and calibrating that signal using appropriate standards.

### B. Identification of Tissue Specific Soluble Proteins

The visualization of tissue specific proteins and identification of unknown tissues from that procedure is carried out using the labeled antibody reagents and detection strategy as described for immunohistochemistry; however the sample is prepared according to an electrophoretic technique to distribute the proteins extracted from the tissue in an orderly array on the basis of molecular weight for detention.

A tissue sample is homogenized using a Virtis apparatus; cell suspensions are disrupted by Dounce homogenization or osmotic lysis, using detergents in either case as required to disrupt cell membranes, as is the practice in the art. Insoluble cell components such as nuclei, microsomes, and membrane fragments are removed by ultracentrifugation, and the soluble protein-containing fraction concentrated if necessary and reserved for analysis.

A sample of the soluble protein solution is resolved into individual protein species by conventional SDS polyacrylamide electrophoresis as described, for example, by Davis, L. et al., Section 19-2 in: Basic Methods in Molecular Biology (P. Leder, ed), Elsevier, New York (1986), using a range of amounts of polyacrylamide in a set of gels to resolve the entire molecular weight range of proteins to be detected in the sample. A size marker is run in parallel for purposes of estimating molecular weights of the constituent proteins. Sample size for analysis is a convenient volume of from 5 to55 µl, and containing from about 1 to 100 µg protein. An aliquot of each of the resolved proteins is transferred by blotting to a nitrocellulose filter paper, a process that maintains the pattern of resolution. Multiple copies are prepared. The procedure, known as Western Blot Analysis, is well described in Davis, L. et al., (above) Section 19-3. One set of nitrocellulose blots is stained with Coomassie Blue dye to visualize the entire set of proteins for comparison with the antibody bound proteins. The remaining nitrocellulose filters are then incubated with a solution of one or mora specific antisera to tissue specific proteins prepared as described in Examples 30 and 40. In this procedure, as in procedure A above, appropriate positive and negative sample and reagent controls are run.

In either procedure A or 8, a detectable label can be attached to the primary tissue antigen-primary antibody complex according to various strategies and permutations thereof. In a straightforward approach, the primary specific antibody can be labeled; alternatively, the unlabeled complex can be bound by a labeled secondary anti-IgG antibody. In other approaches, either the primary or secondary antibody is conjugated to a biotin molecule, which can, in a subsequent step, bind an avidin conjugated marker. According to yet another strategy, enzyme labeled or radioactive protein A, which has the property of binding to any IgG, is bound in a final step to either the primary or secondary antibody.

The visualization of tissue specific antigen binding at levels above those seen in control tissues to one or more tissue specific antibodies, prepared from the gene sequences identified from extended cONA sequences, can identify tissues of unknown origin, for example, forensic samples, or differentiated tumor tissue that has metastasized to foreign bodily sites.

In addition to their applications in forensics and identification, extended cDNAs (or genomic DNAs obtainable therefrom) may be mapped to their chromosomal locations. Example 49 below describes radiation hybrid (RH) mapping of human chromosomal regions using extended cDNAs. Example 50 below describes a representative procedure for mapping an extended cONA (or a genomic DNA obtainable therefrom) to its location on a human chromosome. Example 51 below describes mapping of extended cDNAs (or genomic DNAs obtainable therefrom) on metaphase chromosomes by Fluorescence In Situ Hybridization (FISH).

### EXAMPLE 49

### Radiation hybrid mapping of Extended cOMAs to the human genome

Radiation hybrid (RH) mapping is a somatic cell genetic approach that can be used for high resolution mapping of the human genome. In this approach, cell lines containing one or more human chromosomes are lethally irradiated, breaking each chromosome into fragments whose size depends on the radiation dose. These fragments are rescued by fusion with cultured rodent cells, yielding subclones containing different portions of the human genome. This technique is described by Benham et al. (Genomics 4:509-517, 1989) and Cox et al., (Science 250:245-250, 1990). The random and independent nature of the subclones permits efficient mapping of any human genome marker. Human DNA isolated from a panel of 80-100 cell lines provides a mapping reagent for ordering extended cONAs (or genomic DNAs obtainable therefrom). In this approach, the frequency of breakage between markers is used to measure distance, allowing construction of fine resolution maps as has been done using conventional ESTs (Schuler et al., Science 274:540-546, 1996).

RH mapping has been used to generate a high-resolution whole genome radiation hybrid map of human chromosome 17q22-q25.3 across the genes for growth hormone (GH) and thymidine kinase (TK) (Foster et al., Genomics 33:185-192, 1996), the region surrounding the Gorlin syndrome gene (Obermayr et al., Eur. J. Hum. Genet. 4:242-245, 1996), 60 loci covering the entire short arm of chromosome 12 (Raeymaekers et al., Genomics 29:170-178, 1995), the region of human chromosome 22 containing the neurofibromatosis type 2 locus (Frazer et al., Genomics 14:574-584, 1992) and 13 loci on the long arm of chromosome 5 (Warrington et al., Genomics 11:701-708, 1991).

### EXAMPLE 50

### Mapping of Extended cONAs to Human Chromosomes using PCR techniques

Extended cONAs (or genomic DNAs obtainable therefrom) may be assigned to human chromosomes using PCR based methodologies. In such approaches, oligonucleotide primer pairs are designed from the extended cONA sequence (or the sequence of a genomic DNA obtainable therefrom) to minimize the chance of amplifying through an intron. Preferably, the oligonucleotide primers are 18-23 bp in length and are designed for PCR amplification. The creation of PCR primers from known sequences is well known to those with skill in the art. For a review of PCR technology see Erlich, H.A., PCR Technology: Principles and Applications for DNA Amplification. 1992. W.H. Freeman and Co., New York.

The primers are used in polymerase chain reactions (PCR) to amplify templates from total human genomic DNA. PCR conditions are as follows: 60 ng of genomic DNA is used as a template for PCR with 80 ng of each oligonucleotide primer, 0.6 unit of Taq polymerase, and 1 µCu of a ³²P-labeled deoxycytidine triphosphate. The PCR is performed in a microplate thermocycler (Techne) under the following conditions: 30 cycles of 94°C, 1.4 min; 55°C, 2 min; and 72°C, 2 min; with a final extension at 72°C for 10 min. The amplified products are analyzed on a 6% polyacrylamide sequencing gel and visualized by autoradiography. If the length of the resulting PCR product is identical to the distance between the ends of the primer sequences in the extended cDNA from which the primers are derived, then the PCR reaction is repeated with DNA templates from two panels of human-rodent somatic cell hybrids, BIOS PCRable DNA (BIOS Corporation) and NIGMS Human-Rodent Somatic Cell Hybrid Mapping Panel Number 1 (NIGMS, Camden, NJ).

PCR is used to screen a series of somatic cell hybrid cell lines containing defined sets of human chromosomes for the presence of a given extended cDNA (or genomic DNA obtainable therefrom). DNA is isolated from the somatic hybrids and used as starting templates for PCR reactions using the primer pairs from the extended cDNAs (or genomic DNAs obtainable therefrom). Only those somatic cell hybrids with chromosomes containing the human gene corresponding to the extended cONA (or genomic DNA obtainable therefrom) will yield an amplified fragment. The extended cDNAs (or genomic DNAs obtainable therefrom) are assigned to a chromosome by analysis of the segregation pattern of PCR products from the somatic hybrid DNA templates. The single human chromosome present in all cell hybrids that give rise to an amplified fragment is the chromosome containing that extended cONA (or genomic DNA obtainable therefrom). For a review of techniques and analysis of results from somatic cell gene mapping experiments. (See Ledbetter et al., Genomics 6:475-981 (1990).)

Alternatively, the extended cDNAs (or genomic DNAs obtainable therefrom) may be mapped to individual chromosomes using FISH as described in Example 51 below.

### EXAMPLE 51

### Mapping of Extended 5' ESTs to Chromosomes Using Fluorescence in situ Hybridization

Fluorescence in situ hybridization allows the extended cDNA (or genomic DNA obtainable therefrom) to be mapped to a particular location on a given chromosome. The chromosomes to be used for fluorescence in situ hybridization techniques may be obtained from a variety of sources including cell cultures, tissues, or whole blood.

In a preferred embodiment, chromosomal localization of an extended cDNA (or genomic DNA obtainable therefrom) is obtained by FISH as described by Cherif et al. (Proc. Natl. Acad Sci. U.S.A., 87:6639-6643, 1990). Metaphase chromosomes are prepared from phytohemagglutinin (PHA)-stimulated blood cell donors. PHA-stimulated lymphocytes from healthy males are cultured for 72 h in RPMI-1640 medium. For synchronization, methotrexate (10 µM) is added for 17 h, followed by addition of 5-bromodeoxyuridine (5-BudR, 0.1 mM) for 6 h. Colcemid (1 µg/ml) is added for the last 15 min before harvesting the cells. Cells are collected, washed in RPMI, incubated with a hypotonic solution of KCl (75 mM) at 37°C for 15 min and fixed in three changes of methanol:acetic acid (3:1). The cell suspension is dropped onto a glass slide and air dried. The extended cDNA (or genomic DNA obtainable therefrom) is labeled with biotin-16 dUTP by nick translation according to the manufacturer's instructions (Bethesda Research Laboratories, Bethesda, MD), purified using a Sephadex G-50 column (Pharmacia, Upssala, Sweden) and precipitated. Just prior to hybridization, the DNA pellet is dissolved in hybridization buffer (50% formamide, 2 X SSC. 10% dextran sulfate, 1 mg/ml sonicated salmon sperm DNA, pH 7) and the probe is denatured at 70°C for 5-10 min.

Slides kept at 20°C are treated for 1 h at 37°C with RNase A (100 µg/ml), rinsed three times in 2 X SSC and dehydrated in an ethanol series. Chromosome preparations are denatured in 70% formamide, 2 X SSC for 2 min at 70°C, then dehydrated at 4°C. The slides are treated with proteinase K (10 µg/100 ml in 20 mM Tris-HCl, 2 mM CaCl₂) at 37°C for 8 min and dehydrated. The hybridization mixture containing the probe is placed on the slide, covered with a coverslip, sealed with rubber cement and incubated overnight in a humid chamber at 37°C. After hybridization and post-hybridization washes, the biotinylated probe is detected by avidin-FITC and amplified with additional layers of biotinylated goat anti-avidin and avidin-FITC. For chromosomal localization, fluorescent R-bands are obtained as previously described (Cherif et al., *supra.*). The slides are observed under a LEICA fluorescence microscope (DMRXA). Chromosomes are counterstained with propidium iodide and the fluorescent signal of the probe appears as two symmetrical yellow-green spots on both chromatids of the fluorescent R-band chromosome (red). Thus, a particular extended cDNA (or genomic DNA obtainable therefrom) may be localized to a particular cytogenetic R-band on a given chromosome.

Once the extended cDNAs (or genomic DNAs obtainable therefrom) have been assigned to particular chromosomes using the techniques described in Examples 49-51 above, they may be utilized to construct a high resolution map of the chromosomes on which they are located or to identify the chromosomes in a sample.

### EXAMPLE 52

### Use of Extended cDNAs to Construct or Expand Chromosome Maps

Chromosome mapping involves assigning a given unique sequence to a particular chromosome as described above. Once the unique sequence has been mapped to a given chromosome, it is ordered relative to other unique sequences located on the same chromosome. One approach to chromosome mapping utilizes a series of yeast artificial chromosomes (YACs) bearing several thousand long inserts derived from the chromosomes of the organism from which the extended cDNAs (or genomic DNAs obtainable therefrom) are obtained. This approach is described in Ramaiah Nagaraja et al. Genome Research 7:210-222. March 1997. Briefly, in this approach each chromosome is broken into overlapping pieces which are inserted into the YAC vector. The YAC inserts are screened using PCR or other methods to determine whether they include the extended cDNA (or genomic DNA obtainable therefrom) whose position is to be determined. Once an insert has been found which includes the extended cDNA (or genomic DNA obtainable therefrom), the insert can be analyzed by PCR or other methods to determine whether the insert also contains other sequences known to be on the chromosome or in the region from which the extended cDNA (or genomic DNA obtainable therefrom) was derived. This process can be repeated for each insert in the YAC library to determine the location of each of the extended cONAs (or genomic DNAs obtainable therefrom) relative to one another and to other known chromosomal markers. In this way, a high resolution map of the distribution of numerous unique markers along each of the organisms chromosomes may be obtained.

As described in Example 53 below extended cDNAs (or genomic DNAs obtainable therefrom) may also be used to identify genes associated with a particular phenotype, such as hereditary disease or drug response.

### EXAMPLE 53

### Identification of genes associated with hereditary diseases or drug response

This example illustrates an approach useful for the association of extended cONAs (or genomic DNAs obtainable therefrom) with particular phenotypic characteristics. In this example, a particular extended cONA (or genomic DNA obtainable therefrom) is used as a test probe to associate that extended cONA (or genomic DNA obtainable therefrom) with a particular phenotypic characteristic.

Extended cDNAs (or genomic DNAs obtainable therefrom) are mapped to a particular location on a human chromosome using techniques such as those described in Examples 49 and 50 or other techniques known in the art. A search of Mendelian Inheritance in Man (V. McKusick, Mendelian Inheritance in Man (available on line through Johns Hopkins University Welch Medical Library) reveals the region of the human chromosome which contains the extended cONA (or genomic DNA obtainable therefrom) to be a very gene rich region containing several known genes and several diseases or phenotypes for which genes have not been identified. The gene corresponding to this extended cONA (or genomic DNA obtainable therefrom) thus becomes an immediate candidate for each of these genetic diseases.

Cells from patients with these diseases or phenotypes are isolated and expanded in culture. PCR primers from the extended cONA (or genomic DNA obtainable therefrom) are used to screen genomic DNA, mRNA or cONA obtained from the patients. Extended cONAs (or genomic DNAs obtainable therefrom) that are not amplified in the patients can be positively associated with a particular disease by further analysis. Alternatively, the PCR analysis may yield fragments of different lengths when the samples are derived from an individual having the phenotype associated with the disease than when the sample is derived from a healthy individual, indicating that the gene containing the extended cDNA may be responsible for the genetic disease.

### VI. Use of Extended cDNAs (or genomic DNAs obtainable therefrom) to Construct Vectors

The present extended cONAs (or genomic DNAs obtainable therefrom) may also be used to construct secretion vectors capable of directing the secretion of the proteins encoded by genes inserted in the vectors. Such secretion vectors may facilitate the purification or enrichment of the proteins encoded by genes inserted therein by reducing the number of background proteins from which the desired protein must be purified or enriched. Exemplary secretion vectors are described in Example 54 below.

### EXAMPLE 54

### Construction of Secretion Vectors

The secretion vectors described herein include a promoter capable of directing gene expression in the host cell, tissue, or organism of interest. Such promoters include the Rous Sarcoma Virus promoter, the SV40 promoter, the human cytomegalovirus promoter, and other promoters familiar to those skilled in the art.

A signal sequence from an extended cONA (or genomic DNA obtainable therefrom), such as the signal sequence in SEQ ID NO: 45 as defined in Table IV above, is operably linked to the promoter such that the mRNA transcribed from the promoter will direct the translation of the signal peptide. The host cell tissue, or organism may be any cell, tissue, or organism which recognizes the signal peptide encoded by the signal sequence in the extended cDNA (or genomic DNA obtainable therefrom). Suitable hosts include mammalian cells, tissues or organisms, avian cells, tissues, or organisms, insect cells, tissues or organisms, or yeast.

In addition, the secretion vector contains cloning sites for inserting genes encoding the proteins which are to be secreted. The cloning sites facilitate the cloning of the insert gene in frame with the signal sequence such that a fusion protein in which the signal peptide is fused to the protein encoded by the inserted gene is expressed from the mRNA transcribed from the promoter. The signal peptide directs the extracellular secretion of the fusion protein.

The secretion vector may be DNA or RNA and may integrate into the chromosome of the host, be stably maintained as an extrachromosomal replicon in the host, be an artificial chromosome, or be transiently present in the host. Many nucleic acid backbones suitable for use as secretion vectors are known to those skilled in the art, including retroviral vectors, SV40 vectors, Bovine Papilloma Virus vectors, yeast integrating plasmids, yeast episomal plasmids, yeast artificial chromosomes, human artificial chromosomes, P element vectors, baculovirus vectors, or bacterial plasmids capable of being transiently introduced into the host.

The secretion vector may also contain a polyA signal such that the polyA signal is located downstream of the gene inserted into the secretion vector.

After the gene encoding the protein for which secretion is desired is inserted into the secretion vector, the secretion vector is introduced into the host cell, tissue, or organism using calcium phosphate precipitation, DEAE. Dextran, electroporation, liposome-mediated transfection, viral particles or as naked DNA. The protein encoded by the inserted gene is then purified or enriched from the supernatant using conventional techniques such as ammonium sulfate precipitation, immunoprecipitation, immunochromatography, size exclusion chromatography, ion exchange chromatography, and hplc. Alternatively, the secreted protein may be in a sufficiently enriched or pure state in the supernatant or growth media of the host to permit it to be used for its intended purpose without further enrichment.

The signal sequences may also be inserted into vectors designed for gene therapy. In such vectors, the signal sequence is operably linked to a promoter such that mRNA transcribed from the promoter encodes the signal peptide. A cloning site is located downstream of the signal sequence such that a gene encoding a protein whose secretion is desired may readily be inserted into the vector and fused to the signal sequence. The vector is introduced into an appropriate host cell. The protein expressed from the promoter is secreted extracellularly, thereby producing a therapeutic effect.

The extended cDNAs or 5' ESTs may also be used to clone sequences located upstream of the extended cDNAs or 5' ESTs which are capable of regulating gene expression, induing promoter sequences, enhancer sequences, and other upstream sequences which influence transcription or translation levels. Once identified and cloned, these upstream regulatory sequences may be used in expression vectors designed to direct the expression of an inserted gene in a desired spatial, temporal, developmental, or quantitative fashion. Example 55 describes a method for cloning sequences upstream of the extended cDNAs or 5' ESTs.

### EXAMPLE 55

### Use of Extended cONAs or 5' ESTs to Clone Upstream Sequences from Genomic DNA

Sequences derived from extended cDNAs or 5' ESTs may be used to isolate the promoters of the corresponding genes using chromosome walking techniques. In one chromosome walking technique, which utilizes the GenomeWalker™ kit available from Clontech, five complete genomic DNA samples are each digested with a different restriction enzyme which has a 6 base recognition site and leaves a blunt end. Following digestion, oligonucleotide adapters are ligated to each end of the resulting genomic DNA fragments.

For each of the five genomic DNA libraries, a first PCR reaction is performed according to the manufacturer's instructions using an outer adaptor primer provided in the kit and an outer gene specific primer. The gene specific primer should be selected to be specific for the extended cONA or 5' EST of interest and should have a melting temperature, length, and location in the extended cDNA or' EST which is consistent with its use in PCR reactions. Each first PCR reaction contains 5ng of genomic DNA, 5 µl of 10X Tth reaction buffer, 0.2 mM of each dNTP, 0.2 µM each of outer adaptor primer and outer gene specific primer, 1.1 mM of Mg(OAc)₂, and 1 µl of the Tth polymerase 50X mix in a total volume of 50 µl. The reaction cycle for the first PCR reaction is as follows: 1 min @ 94°C /2 sec @ 94°C, 3 min @ 72°C (7 cycles) / 2 sec @ 94°C, 3 min @ 67°C (32 cycles) / 5 min @ 67°C.

The product of the first PCR reaction is diluted and used as a template for a second PCR reaction according to the manufacturer's instructions using a pair of nested primers which are located internally on the amplicon resulting from the first PCR reaction. For example, 5 µl of the reaction product of the first PCR reaction mixture may be diluted 180 times. Reactions are made in a 50 µl volume having a composition identical to that of the first PCR reaction except the nested primers are used. The first nested primer is specific for the adaptor, and is provided with the GenomeWalker™ kit. The second nested primer is specific for the particular extended cDNA or 5' EST for which the promoter is to be cloned and should have a melting temperature, length, and location in the extended cDNA or 5' EST which is consistent with its use in PCR reactions. The reaction parameters of the second PCR reaction are as follows:1 min @ 94°C / 2 sec @ 94°C. 3 min @ 72°C (6 cycles) / 2 sec @ 94°C, 3 min @ 67°C (25 cycles) / 5 min @ 67°C

The product of the second PCR reaction is purified, cloned, and sequenced using standard techniques. Alternatively, two or more human genomic DNA libraries can be constructed by using two or more restriction enzymes. The digested genomic DNA is cloned into vectors which can be converted into single stranded, circular, or linear DNA. A biotinylated oligonucleotide comprising at least 15 nucleotides from the extended cONA or 5' EST sequence is hybridized to the single stranded DNA. Hybrids between the biotinylated oligonucleotide and the single stranded DNA containing the extended cDNA or EST sequence are isolated as described in Example 29 above. Thereafter, the single stranded DNA containing the extended cDNA or EST sequence is released from the beads and converted into double stranded DNA using a primer specific for the extended cONA or 5' EST sequence or a primer corresponding to a sequence included in the cloning vector. The resulting double stranded DNA is transformed into bacteria. DNAs containing the 5' EST or extended cDNA sequences are identified by colony PCR or colony hybridization.

Once the upstream genomic sequences have been cloned and sequenced as described above, prospective promoters and transcription start sites within the upstream sequences may be identified by comparing the sequences upstream of the extended cDNAs or 5' ESTs with databases containing known transcription start sites, transcription factor binding sites, or promoter sequences.

In addition, promoters in the upstream sequences may be identified using promoter reporter vectors as described in Example 56.

### EXAMPLE 56

### Identification of Promoters in Cloned Upstream Sequences

The genomic sequences upstream of the extended cDNAs or 5' ESTs are cloned into a suitable promoter reporter vector, such as the pSEAP-Basic, pSEAP-Enhancer, pβgal-Basic, pβgal-Enhancer, or pEGFP-1 Promoter Reporter vectors available from Clontech. Briefly, each of these promoter reporter vectors include multiple cloning sites positioned upstream of a reporter gene encoding a readily assayable protein such as secreted alkaline phosphatase, β galactosidase, or green fluorescent protein. The sequences upstream of the extended cDNAs or 5' ESTs are inserted into the cloning sites upstream of the reporter gene in both orientations and introduced into an appropriate host cell. The level of reporter protein is assayed and compared to the level obtained from a vector which lacks an insert in the cloning site. The presence of an elevated expression level in the vector containing the insert with respect to the control vector indicates the presence of a promoter in the insert. If necessary, the upstream sequences can be cloned into vectors which contain an enhancer for augmenting transcription levels from weak promoter sequences. A significant level of expression above that observed with the vector lacking an insert indicates that a promoter sequence is present in the inserted upstream sequence.

Appropriate host cells for the promoter reporter vectors may be chosen based on the results of the above described determination of expression patterns of the extended cDNAs and ESTs. For example, if the expression pattern analysis indicates that the mRNA corresponding to a particular extended cDNA or 5' EST is expressed in fibroblasts, the promoter reporter vector may be introduced into a human fibroblast cell line.

Promoter sequences within the upstream genomic DNA may be further defined by constructing nested deletions in the upstream DNA using conventional techniques such as Exonuclease III digestion. The resulting deletion fragments can be inserted into the promoter reporter vector to determine whether the deletion has reduced or obliterated promoter activity. In this way, the boundaries of the promoters may be defined. If desired, potential individual regulatory sites within the promoter may be identified using site directed mutagenesis or linker scanning to obliterate potential transcription factor binding sites within the promoter individually or in combination. The effects of these mutations on transcription levels may be determined by inserting the mutations into the cloning sites in the promoter reporter vectors.

### EXAMPLE 57

### Cloning and Identification of Promoters

Using the method described in Example 55 above with 5' ESTs, sequences upstream of several genes were obtained. Using the primer pairs GGG AAG ATG GAG ATA GTA TTG CCT G (SEQ ID NO:29) and CTG CCA TGT ACA TGA TAG AGA GAT TC (SEQ ID NO:30), the promoter having the internal designation P13H2 (SEQ ID NO:31) was obtained.

Using the primer pairs GTA CCA GGGG ACT GTG ACC ATT GC (SEQ ID NO:32) and CTG TGA CCA TTG CTC CCA AGA GAG (SEQ ID NO:33), the promoter having the internal designation P15B4 (SEQ ID NO:34) was obtained.

Using the primer pairs CTG GGA TGG AAG GCA CGG TA (SEQ ID NO:35) and GAG ACC ACA CAG CTA GAC AA (SEQ ID NO:36), the promoter having the internal designation P29B6 (SEQ ID NO:37) was obtained.

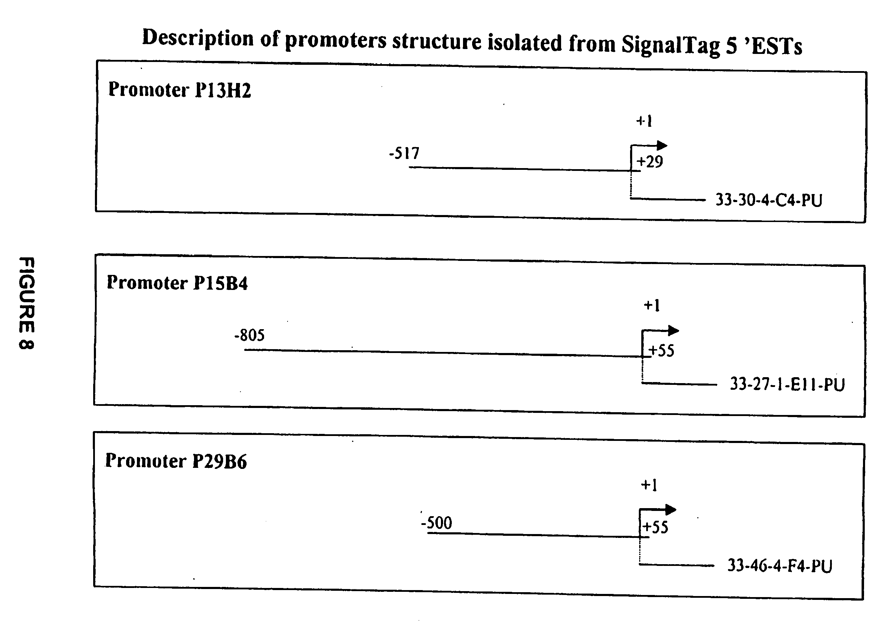

Figure 8 provides a schematic description of the promoters isolated and the way they are assembled with the corresponding 5' tags. The upstream sequences were screened for the presence of motifs resembling transcription factor binding sites or known transcription start sites using the computer program MatInspector release 2.0, August 1996.

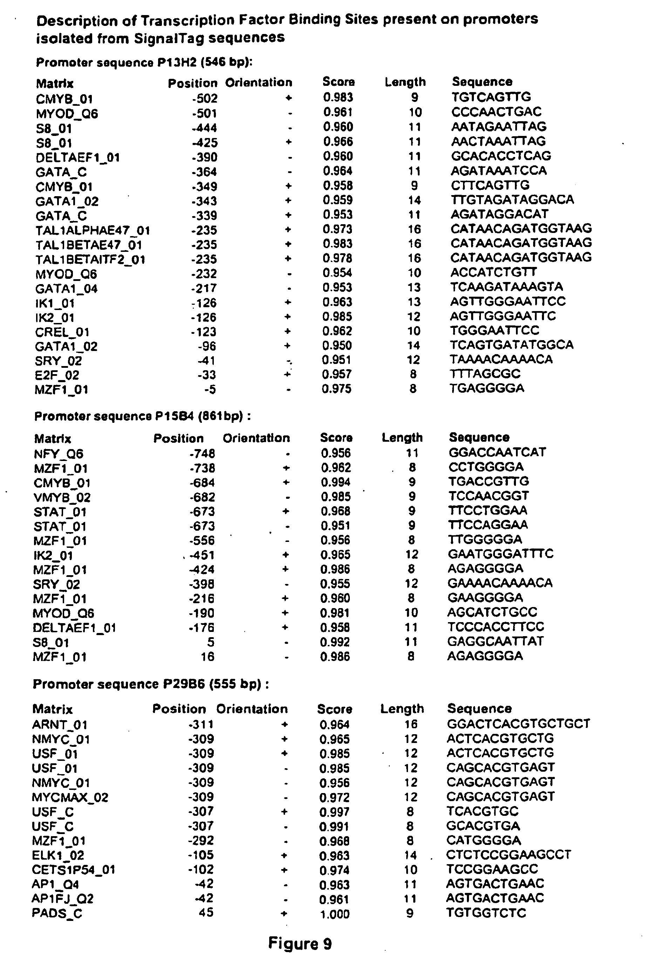

Figure 9 describes the transcription factor binding sites present in each of these promoters. The columns labeled matrice provides the name of the MatInspector matrix used. The column labeled position provides the 5' postion of the promoter site. Numeration of the sequence starts from the transcription site as determined by matching the genomic sequence with the 5' EST sequence. The column labeled "orientation" indicates the DNA strand on which the site is found, with the + strand being the coding strand as determined by matching the genomic sequence with the sequence of the 5' EST. The column labeled "score" provides the MatInspector score found for this site. The column labeled "length" provides the length of the site in nucleotides. The column labeled "sequence" provides the sequence of the site found.

The promoters and other regulatory sequences located upstream of the extended cDNAs or 5' ESTs may be used to design expression vectors capable of directing the expression of an inserted gene in a desired spatial, temporal, developmental, or quantitative manner. A promoter capable of directing the desired spatial; temporal, developmental, and quantitative patterns may be selected using the results of the expression analysis described in Example 26 above. For example, if a promoter which confers a high level of expression in muscle is desired, the promoter sequence upstream of an extended cDNA or 5' EST derived from an mRNA which is expressed at a high level in muscle, as determined by the method of Example 26, may be used in the expression vector.

Preferably, the desired promoter is placed near multiple restriction sites to facilitate the cloning of the desired insert downstream of the promoter, such that the promoter is able to drive expression of the inserted gene. The promoter may be inserted in conventional nucleic acid backbones designed for extrachromosomal replication, integration into the host chromosomes or transient expression. Suitable backbones for the present expression vectors include retroviral backbones, backbones from eukaryotic episomes such as SV40 or Bovine Papilloma Virus, backbones from bacterial episomes, or artificial chromosomes.

Preferably, the expression vectors also include a polyA signal downstream of the multiple restriction sites for directing the polyadenylation of mRNA transcribed from the gene inserted into the expression vector.

Following the identification of promoter sequences using the procedures of Examples 55-57. proteins which interact with the promoter may be identified as described in Example 58 below.

### EXAMPLE 58

### Identification of Proteins Which Interact with Promoter Sequences, Upstream Regulatory Sequences, or mRNA

Sequences within the promoter region which are likely to bind transcription factors may be identified by homology to known transcription factor binding sites or through conventional mutagenesis or deletion analyses of reporter plasmids containing the promoter sequence. For example, deletions may be made in a reporter plasmid containing the promoter sequence of interest operably linked to an assayable reporter gene. The reporter plasmids carrying various deletions within the promoter region are transfected into an appropriate host cell and the effects of the deletions on expression levels is assessed. Transcription factor binding sites within the regions in which deletions reduce expression levels may be further localized using site directed mutagenesis, linker scanning analysis, or other techniques familiar to those skilled in the art. Nucleic acids encoding proteins which interact with sequences in the promoter may be identified using one hybrid systems such as those described in the manual accompanying the Matchmaker One-Hybrid System kit avalilabe from Clontech (Catalog No. K1603-1). Briefly, the Matchmaker One-hybrid system is used as follows. The target sequence for which it is desired to identify binding proteins is cloned upstream of a selectable reporter gene and integrated into the yeast genome. Preferably, multiple copies of the target sequences are inserted into the reporter plasmid in tandem.

A library comprised of fusions between cDNAs to be evaluated for the ability to bind to the promoter and the activation domain of a yeast transcription factor, such as GAL4, is transformed into the yeast strain containing the integrated reporter sequence. The yeast are plated on selective media to select cells expressing the selectable marker linked to the promoter sequence. The colonies which grow on the selective media contain genes encoding proteins which bind the target sequence. The inserts in the genes encoding the fusion proteins are further characterized by sequencing. In addition, the inserts may be inserted into expression vectors or in vitro transcription vectors. Binding of the polypeptides encoded by the inserts to the promoter DNA may be confirmed by techniques familiar to those skilled in the art, such as gel shift analysis or DNAse protection analysis.

### VII. Use of Extended cDNAs (or Genomic DNAs Obtainable Therefrom) in Gene Therapy

The extended cDNAs (or genomic DNAs obtainable therefrom) may also be used in gene therapy strategies, including antisense and triple helix strategies as described in Examples 57 and 58 below. In antisense approaches, nucleic acid sequences complementary to an mRNA are hybridized to the mRNA intracellularly, thereby blocking the expression of the protein encoded by the mRNA. The antisense sequences may prevent gene expression through a variety of mechanisms. For example, the antisense sequences may inhibit the ability of ribosomes to translate the mRNA. Alternatively, the antisense sequences may block transport of the mRNA from the nucleus to the cytoplasm, thereby limiting the amount of mRNA available for translation. Another mechanism through which antisense sequences may inhibit gene expression is by interfering with mRNA splicing. In yet another strategy, the antisense nucleic acid may be incorporated in a ribozyme capable of specifically cleaving the target mRNA.

### EXAMPLE 59

### Preparation and Use of Antisense Oligonucleotides

The antisense nucleic acid molecules to be used in gene therapy may be either DNA or RNA sequences. They may comprise a sequence complementary to the sequence of the extended cDNA (or genomic DNA obtainable therefrom). The antisense nucleic acids should have a length and melting temperature sufficient to permit formation of an intracellular duplex having sufficient stability to inhibit the expression of the mRNA in the duplex. Strategies for designing antisense nucleic acids suitable for use in gene therapy are disclosed in Green et al., Ann. Rev. Biochem. 55:569-597 (1986) and Izant and Weintraub, Cell 36:1007-1015 (1984).

In some strategies, antisense molecules are obtained from a nucleotide sequence encoding a protein by reversing the orientation of the coding region with respect to a promoter so as to transcribe the opposite strand from that which is normally transcribed in the cell. The antisense molecules may be transcribed using in vitro transcription systems such as those which employ T7 or SP6 polymerase to generate the transcript. Another approach involves transcription of the antisense nucleic acids in vivo by operably linking DNA containing the antisense sequence to a promoter in an expression vector.

Alternatively, oligonucleotides which are complementary to the strand normally transcribed in the cell may be synthesized in vitro. Thus, the antisense nucleic acids are complementary to the corresponding mRNA and are capable of hybridizing to the mRNA to create a duplex. In some embodiments, the antisense sequences may contain modified sugar phosphate backbones to increase stability and make them less sensitive to RNase activity. Examples of modifications suitable for use in antisense strategies are described by Rossi et al., Pharmacol. Ther. 50(2):245-254. (1991).

Various types of antisense oligonucleotides complementary to the sequence of the extended cDNA (or genomic DNA obtainable therefrom) may be used. In one preferred example, stable and semi-stable antisense oligonucleotides described in International Application No. PCT WO94/23026 are used. In these moleucles, the 3' end or both the 3' and 5' ends are engaged in intramolecular hydrogen bonding between complementary base pairs. These molecules are better able to withstand exonuclease attacks and exhibit increased stability compared to conventional antisense oligonucleotides.

In another preferred example, the antisense oligodeoxynucleotides against herpes simplex virus types 1 and 2 described in International Application No. WO 95/04141.

In yet another preferred example, the covalently cross-linked antisense oligonucleotides described in International Application No. WO 96/31523 are used. These double- or single-stranded oligonucleotides comprise one or more, respectively, inter- or intra-oligonucleotide covalent cross-linkages, wherein the linkage consists of an amide bond between a primary amine group of one strand and a carboxyl group of the other strand or of the same strand, respectively, the primary amine group being directly substituted in the 2' position of the strand nucleotides monosaccharide ring, and the carboxyl group being carried by an aliphatic spacer group substituted on a nucleotide or nucleotide analog of the other strand or the same strand, respectively.

The antisense-oligodeoxynucleotides and oligonucleotides disclosed in International Application No. WO 92/18522 may also be used. These molecules are stable to degradation and contain at least one transcription control recognition sequence which binds to control proteins and are effective as decoys therefor. These molecules may contain "hairpin" structures, "dumbbell' structures, "modified dumbbell" structures, "cross-linked" decoy structures and "loop" structures.

In another preferred example, the cyclic double-stranded oligonucleotides described in European Patent Application No. 0 572 287 A2 are used. These ligated oligonucleotide "dumbbells" contain the binding site for a transcription factor and inhibit expression of the gene under control of the transcription factor by sequestering the factor.

Use of the closed antisense oligonucleotides disclosed in International Application No. WO 92/19732 is also contemplated. Because these molecules have no free ends, they are more resistant to degradation by exonucleases than are conventional oligonucleotides. These oligonucleotides may be multifunctional, interacting with several regions which are not adjacent to the target mRNA.

The appropriate level of antisense nucleic acids required to inhibit gene expression may be determined using in vitro expression analysis. The antisense molecule may be introduced into the cells by diffusion, injection, infection or transfection using procedures known in the art. For example, the antisense nucleic acids can be introduced into the body as a bare or naked oligonucleotide, oligonucleotide encapsulated in lipid, oligonucleotide sequence encapsidated by viral protein, or as an oligonucleotide operably linked to a promoter contained in an expression vector. The expression vector may be any of a variety of expression vectors known in the art, including retroviral or viral vectors, vectors capable of extrachromosomal replication, or integrating vectors. The vectors may be DNA or RNA.

The antisense molecules are introduced onto cell samples at a number of different concentrations preferably between 1x10⁻¹⁰M to 1x10⁻⁴M. Once the minimum concentration that can adequately control gene expression is identified, the optimized dose is translated into a dosage suitable for use in vivo. For example, an inhibiting concentration in culture of 1x10⁻⁷ translates into a dose of approximately 0.6 mg/kg bodyweight. Levels of oligonucleotide approaching 100 mg/kg bodyweight or higher may be possible after testing the toxicity of the oligonucleotide in laboratory animals. It is additionally contemplated that cells from the vertebrate are removed, treated with the antisense oligonucleotide, and reintroduced into the vertebrate.

It is further contemplated that the antisense oligonucleotide sequence is incorporated into a ribozyme sequence to enable the antisense to specifically bind and cleave its target mRNA. For technical application of ribozyme and antisense oligonucleotides see Rossi et al., *supra*.

In a preferred application, the polypeptide encoded by the gene is first identified, so that the effectiveness of antisense inhibition on translation can be monitored using techniques that include but are not limited to antibody-mediated tests such as RlAs and ELISA, functional assays, or radiolabeling.

The extended cDNAs described herein (or genomic DNAs obtainable therefrom) may also be used in gene therapy approaches based on intracellular triple helix formation. Triple helix oligonucleotides are used to inhibit transcription from a genome. They are particularly useful for studying alterations in cell activity as it is associated with a particular gene. The extended cDNAs for genomic DNAs obtainable therefrom) or more preferably, a portion of those sequences, can be used to inhibit gene expression in individuals having diseases associated with expression of a particular gene. Similarly, a portion of the extended cDNA (or genomic DNA obtainable therefrom) can be used to study the effect of inhibiting transcription of a particular gene within a cell. Traditionally, homopurine sequences were considered the most useful for triple helix strategies. However, homopyrimidine sequences can also inhibit gene expression. Such homopyrimidine oligonucleotides bind to the major groove at homopurine:homopyrimidine sequences. Thus, both types of sequences from the extended cDNA or from the gene corresponding to the extended cDNA are contemplated.

### EXAMPLE 60

### Preparation and use of Triple Helix Probes

The sequences of the extended cDNAs (or genomic DNAs obtainable therefrom) are scanned to identify 10-mer to 20-mer homopyrimidine or homopurine stretches which could be used in triple-helix based strategies for inhibiting gene expression. Following identification of candidate homopyrimidine or homopurine stretches, their efficiency in inhibiting gene expression is assessed by introducing varying amounts of oligonucleotides containing the candidate sequences into tissue culture cells which normally express the target gene. The oligonucleotides may be prepared on an oligonucleotide synthesizer or they may be purchased commercially from a company specializing in custom oligonucleotide synthesis, such as GENSET. Paris, France.

The oligonucleotides may be introduced into the cells using a variety of methods known to those skilled in the art, including but not limited to calcium phosphate precipitation. DEAE-Dextran, electroporation, liposome-mediated transfection or native uptake.

Treated cells are monitored for altered cell function or reduced gene expression using techniques such as Northern blotting, RNase protection assays, or PCR based strategies to monitor the transcription levels of the target gene in cells which have been treated with the oligonucleotide. The cell functions to be monitored are predicted based upon the homologies of the target gene corresponding to the extended cDNA from which the oligonucleotide was derived with known gene sequences that have been associated with a particular function. The cell functions can also be predicted based on the presence of abnormal physiologies within cells derived from individuals with a particular inherited disease, particularly when the extended cDNA is associated with the disease using techniques described in Example 53.

The oligonucleotides which are effective in inhibiting gene expression in tissue culture cells may then be introduced in vivo using the techniques described above and in Example 59 at a dosage calculated based on the in vitro results, as described in Example 59.

In some examples, the natural (beta) anomers of the oligonucleotide units can be replaced with alpha anomers to render the oligonucleotide more resistant to nucleases. Further, an intercalating agent such as ethidium bromide, or the like, can be attached to the 3' end of the alpha oligonucleotide to stabilize the triple helix. For information on the generation of oligonucleotides suitable for triple helix formation see Griffin et al. (Science 245:967. 971 (1989).

### EXAMPLE 61

### Use of Extended cDNAs to Express an Encoded Protein in a Host Organism

The extended cDNAs described herein may also be used to express an encoded protein in a host organism to produce a beneficial effect. In such procedures, the encoded protein may be transiently expressed in the host organism or stably expressed in the host organism. The encoded protein may have any of the activities described above. The encoded protein may be a protein which the host organism lacks or, alternatively, the encoded protein may augment the existing levels of the protein in the host organism.

A full length extended cDNA encoding the signal peptide and the mature protein, or an extended cDNA encoding only the mature protein is introduced into the host organism. The extended cDNA may be introduced into the host organism using a variety of techniques known to those of skill in the art. For example, the extended cDNA may be injected into the host organism as naked DNA such that the encoded protein is expressed in the host organism, thereby producing a beneficial effect.

Alternatively, the extended cDNA may be cloned into an expression vector downstream of a promoter which is active in the host organism. The expression vector may be any of the expression vectors designed for use in gene therapy, including viral or retroviral vectors.

The expression vector may be directly introduced into the host organism such that the encoded protein is expressed in the host organism to produce a beneficial effect. In another approach, the expression vector may be introduced into cells in vitro. Cells containing the expression vector are thereafter selected and introduced into the host organism, where they express the encoded protein to produce a beneficial effect.

### EXAMPLE 62

### Use Of Signal Peptides Encoded By 5' Ests Or Sequences Obtained Therefrom To Import Proteins Into Cells

The short core hydrophobic region (h) of signal peptides encoded by the 5'ESTS or extended cDNAs derived from the 5'ESTs described herein may also be used as a carrier to import a peptide or a protein of interest, so-called cargo, into tissue culture cells (Lin et al., J. Biol. Chem., 270: 14225-14258 (1995): Du et al., J. Peptide Res., 51: 235-243 (1998); Rojas et al., Nature Biotech., 16: 370-375 (1998)).

When cell permeable peptides of limited size (approximately up to 25 amino acids) are to be translocated across cell membrane, chemical synthesis may be used in order to add the h region to either the C-terminus or the N-terminus to the cargo peptide of interest. Alternatively, when longer peptides or proteins are to be imported into cells, nucleic acids can be genetically engineered, using techniques familiar to those skilled in the art, in order to link the extended cDNA sequence encoding the h region to the 5' or the 3' end of a DNA sequence coding for a cargo polypeptide. Such genetically engineered nucleic acids are then translated either *in vitro* or *in vivo* after transfection into appropriate cells, using conventional techniques to produce the resulting cell permeable polypeptide. Suitable hosts cells are then simply incubated with the cell permeable polypeptide which is then translocated across the membrane.

This method may be applied to study diverse intracellular functions and cellular processes. For instance, it has been used to probe functionally relevant domains of intracellular proteins and to examine protein-protein interactions involved in signal transduction pathways (Lin *et al., supra*: Lin et al., J. Biol. Chem., 271: 5305-5308 (1996); Rojas et al., J. Biol. Chem., 271: 27456-27461 (1996); Liu et al., Proc. Natl. Acad. Sci. USA. 93: 11819-11824 (1996); Rojas et al., Bioch. Biophys. Res. Commun., 234: 675-680 (1997)).

Such techniques may be used in cellular therapy to import proteins producing therapeutic effects. For instance, cells isolated from a patient may be treated with imported therapeutic proteins and then re-introduced into the host organism.

Alternatively, the h region of signal peptides described herein could be used in combination with a nuclear localization signal to deliver nucleic acids into cell nucleus. Such oligonucleotides may be antisense oligonucleotides or oligonucleotides designed to form triple helixes, as described in examples 59 and 60 respectively, in order to inhibit processing and maturation of a target cellular RNA.

### EXAMPLE 63

### Reassembling & Resequencing of Clones

Full length cDNA clones obtained by the procedure described in Example 27 were double-sequenced. These sequences were assembled and the resulting consensus sequences were then reanalyzed. Open reading frames were reassigned following essentially the same process as the one described in Example 27.

After this reanalysis process a few abnormalities were revealed. The sequences presented in SEQ ID NOs: 47, 73, 79, 89, 91, 96, 126, 128, 134, and 139 are apparently unlikely to be genuine full length cDNAs. These clones are missing a stop codon and are thus more probably 3' truncated cDNA sequences. Similarly, the sequences presented in SEQ ID NOs: 45, 50, 54, 57, 73, 74, 89, 92, 95, 98, 126, 129, 130, 131 and 139 may also not be genuine full length cDNAs based on homology studies with existing protein sequences. Although both of these sequences encode a potential start methionine each could represent a 5' truncated cDNA.

In addition, SEQ ID NO: 115 was found to be an alternatively spliced transcript and the identities of some of the bases in SEQ ID NO: 131 were corrected.

Finallly, after the reassignment of open reading frames for the clones, new open reading frames were chosen in some instances. For example, in the case of SEO ID NOs: 41, 47, 50, 52, 54-56, 58, 59, 61, 74, 75, 79, 84, 89, 91, 92, 96, 98, 103, 105, 106, 126, 129, 131, and 133 the new open reading frames were no longer predicted to contain a signal peptide.

As discussed above, Table IV provides the location of the full coding sequence in SEO ID NO: 45 (i.e. the nucleotides encoding both the signal peptide and the mature protein, listed under the heading FCS location in Table IV), the locations of the nucleotides in SEQ ID NO: 45 which encode the signal peptides (listed under the heading SigPep Location in Table IV), the locations of the nucleotides in SEQ ID NO: 45 which encode the mature proteins generated by cleavage of the signal peptides (listed under the heading Mature Polypeptide Location in Table IV), the locations in SEQ ID NO: 45 of stop codons (listed under the heading Stop Codon Location in Table IV) the locations in SEQ ID NO: 45 of polyA signals (listed under the heading g PolyA Signal Location in Table IV) and the locations of polyA sites (listed under the heading PolyA Site Location in Table IV).

As discussed above, Table V lists the sequence identification numbers of the polypeptide of SEQ ID NO: 146, the locations of the amino acid residues of SEQ ID NO: 146 in the full length polypeptide (second column), the locations of the amino acid residues of SEQ ID NO: 146 in the signal peptides (third column), and the locations of the amino acid residues of SEQ ID NO: 146 in the mature polypeptide created by cleaving the signal peptide from the fall length polypeptide (fourth column). In Table V, and in the appended sequence listing, the first amino acid of the mature protein resulting from cleavage of the signal peptide is designated as amino acid number 1 and the first amino acid of the signal peptide is designated with the appropriate negative number, in accordance with the regulations governing sequence listings.

### EXAMPLE 64

### Functional Analysis of Predicted Protein Sequences

Following double-sequencing, new contigs were assembled for each of the extended cDNAs and each was compared to known sequences available at the time of filing. These sequences originate from the following databases: Genbank (release 108 and daily releases up to October, 15, 1998). Genseq (release 32) PIR (release 53) and SwissProt (release 35). The predicted proteins matching known proteins were further classified into 3 categories depending on the level of homology.

The first category contains proteins exhibiting more than 70% identical amino acid residues on the whole length of the matched protein. They are clearly close homologues which most probably have the same function or a very similar function as the matched protein.

The second category contains proteins exhibiting more remote homologies (40 to 70% over the whole protein) indicating that the protein may have functions similar to those of the homologous protein.

The third category contains proteins exhibiting homology (90 to 100%) to a domain of a known protein indicating that the matched protein and the protein of the invention may share similar features.

It should be noted that the numbering of amino acids in the protein sequences discussed in (Figures 10 to 15) the first methionine encountered is designated as amino acid number 1. In the appended sequence listing, the first amino acid of the mature protein resulting from cleavage of the signal peptide is designated as amino acid number 1, and the first amino acid of the signal peptide is designated with the appropriate negative number, in accordance with the regulations governing sequence listings.

In addition all of the corrected amino acid sequences (SEQ ED NOs: 141-241 and 378-513) were scanned for the presence of known protein signatures and motifs. This search was performed against the Prosite 15.0 database, using the Proscan software from the GCG package.

### Protein of SEQ ID NO: 146

The protein SEQ ID NO: 146 encoded by the extended cDNA SEQ ID NO: 45 shows homology to human apolipoprotein L (Genbank accession number AFO 19225). The matched protein is a secreted high density lipoprotein associated with apoA-I-containing lipoproteins which play a key role in reverse cholesterol transport.

Taken together, these data suggest that the protein of SEQ ID NO. 146 may play a role in lipid metabolism. Thus, this protein may be useful in diagnosing and/or treating several types of disorders including, but not limited to, hyperlipidemia, hypercholesterolemia, atherosclerosis, cardiovascular disorders such as, coronary heart disease, and neurodegenerative disorders such as Alzheimer's disease or dementia.

Although this invention has been described in terms of certain preferred embodiments, other embodiments which will be apparent to those of ordinary skill in the art in view of the disclosure herein are also within the scope of this invention. Accordingly, the scope of the invention is intended to be defined only by reference to the appended claims.

As discussed above, the extended cDNAs described herein or portions thereof can be used for various purposes. The polynucleotides can be used to express recombinant protein for analysis, characterization or therapeutic use; as markers for tissues in which the corresponding protein is preferentially expressed (either constitutively or at a particular stage of tissue differentiation or development or in disease states): as molecular weight markers on Southern gels; as chromosome markers or tags (when labeled) to identify chromosomes or to map related gene positions: to compare with endogenous DNA sequences in patients to identify potential genetic disorders; as probes to hybridize and thus discover novel, related DNA sequences; as a source of information to derive PCR primers for genetic fingerprinting; for selecting and making oligomers for attachment to a "gene chip" or other support, including for examination for expression patterns; to raise anti-protein antibodies using DNA immunization techniques; and as an antigen to raise anti-DNA antibodies or elicit another immune response. Where the polynucleotide encodes a protein which binds or potentially binds to another protein (such as, for example, in a receptor-ligand interaction), the polynucleotide can also be used in interaction trap assays (such as, for example, that described in Gyuris et al., Cell 75:791-803 (1993)) to identify polynucleotides encoding the other protein with which binding occurs or to identify inhibitors of the binding interaction.

The proteins or polypeptides provided herein can similarly be used in assays to determine biological activity, including in a panel of multiple proteins for high-throughput screening: to raise antibodies or to elicit another immune response; as a reagent (including the labeled reagent) in assays designed to quantitatively determine levels of the protein (or its receptor) in biological fluids; as markers for tissues in which the corresponding protein is preferentially expressed (either constitutively or at a particular stage of tissue differentiation or development or in a disease state); and, of course, to isolate correlative receptors or ligands. Where the protein binds or potentially binds to another protein (such as, for example, in a receptor-ligand interaction), the protein can be used to identify the other protein with which binding occurs or to identify inhibitors of the binding interaction. Proteins involved in these binding interactions can also be used to screen for peptide or small molecule inhibitors or agonists of the binding interaction.

Any or all of these research utilities are capable of being developed into reagent grade or kit format for commercialization as research products.

Methods for performing the uses listed above are well known to those skilled in the art. References disclosing such methods include without limitation "Molecular Cloning; A Laboratory Manual". 2d ed., Cole Spring Harbor Laboratory Press, Sambrook. J., E.F. Fritsch and T. Maniatis eds., 1989, and "Methods in Enzymology; Guide to Molecular Cloning Techniques", Academic Press, Berger. S.L. and A.R. Kimmel eds., 1987.

Polynucleotides and proteins can also be used as nutritional sources or supplements. Such uses include without limitation use as a protein or amino acid supplement, use as a carbon source, use as a nitrogen source and use as a source of carbohydrate. In such causes the protein or polynucleotide can be added to the feed of a particular organism or can be administered as a separate solid or liquid preparation, such as in the form of powder, pills, solutions, suspensions or capsules. In the case of microorganisms, the protein or polynucleotide of can be added to the medium in or on which the microorganism is cultured.

### SEQUENCE LISTING FREE TEXT

The following free text appears in the accompanying Sequence Listing:
In vitro transcription product
oligonucleotide
promoter
transcription start site
Von Heijne matrix
Score
matinspector prediction
name

**TABLE I**

| SEQ ID NO. in Present Application | Provisional Application Disclosing Sequence | SEQ ID NO. in Provisional Application |
|---|---|---|
| 45 | U.S. Provisional Patent Application Serial No. 60/074,121, filed Feb. 9, 1998 | 41 |

**TABLE II: Parameters used for each step of EST analysis**

| | Search Characteristics | | | Selection Characteristics | |
|---|---|---|---|---|---|
| Step | Program | Strand | Parameters | Identity (%)) | Length (bp) |
| Misceflaneous | Blastn | both | S-61 X-16 | 90 | 17 |
| tRNA | Fasta | both | - | 80 | 60 |
| rRNA | Blastn | both | S-108 | 80 | 40 |
| mtRNA | Blastn | both | S-108 | 80 | 40 |
| Procaryotic | Blastn | both | S-144 | 90 | 40 |
| Fungal | Blastn | both | S-144 | 90 | 40 |
| Alu | fasta* | both | - | 70 | 40 |
| L1 | Blastn | both | S-72 | 70 | 40 |
| Repeats | Blastn | both | S-72 | 70 | 40 |
| Promoters | Blastn | top | S-54X-16 | 90 | 15┴ |
| Vertebrate | fasta* | both | S-108 | 90 | 30 |
| EST₃ | Blatsn | both | S-108 X-16 | 90 | 30 |
| Proteins | blastxη | top | E-0.001 | - | - |

| | | | | | |
|---|---|---|---|---|---|
| * use "Quick Fast" Database Scanner ┴ alignment further constrained to begin closer than 10bp to EST15' end η using BLOSUM62 substitution matrix | | | | | |

**TABLE III: Parameters used for each step of extended cDNA analysis**

| | Search characteristics | | Selection characteristics | | | |
|---|---|---|---|---|---|---|
| Step | Program | Strand | parameters | Identity (%) | Length (bp) | Comments |
| misceflaneous* | FASTA | both | - | 90 | 15 | |
| tRNA³ | FASTA | both | - | 80 | 90 | |
| rRHA¹ | BLASTN | both | S-108 | 80 | 40 | |
| mtRNA¹ | BLASTN | both | S-108 | 80 | 40 | |
| Procaryotis¹ | BLASTN | both | S-144 | 90 | 40 | |
| Fungal⁻ | BLASTN | both | S-144 | 90 | 40 | |
| Alu^{*} | BLASTN | both | S-72 | 70 | 40 | max 5 matches, masking |
| L1¹ | BLASTN | both | S-72 | 70 | 40 | max 5 matches masking |
| Repeats' | BLASTN | both | S-72 | 70 | 40 | masking |
| PolyA | BLAST2N | top | W-6,S-10,E-1000 | 90 | 8 | in the last 20 nucleotides |
| Polyadenylati on signal | - | top | AATAAA allowing 1 mismatch | | | in the 50 nucleotides preceding the 5' end of the polA |
| Vertebrate* | BLASTN than FASTA | bath | - | 90 than 70 | 30 | first BLASTN end then FASTA on matching sequences |
| ESTs* | BLAST2N | both | - | 90 | 30 | |
| Geneseq | BLASTN | both | W-8, B-10 | 90 | 30 | |
| ORF | BLASTP | top | W-8, B-10 | - | - | on ORF proteins, max 10 matches |
| Proteins* | BLASTX | top | E-0.001 | 70 | 30 | |

| | | | | | | |
|---|---|---|---|---|---|---|
| ¹ steps common to EST analysis and using the same algorithms and parameters ^{*} steps also used in EST analysis but with different algorithms and/or parameters | | | | | | |

**TABLE IV**

| Id | FCS Location | SigPep Location | Mature Polypeptide Location | Stop Codon Location | PolyA Signal Location | PolyA Site Location |
|---|---|---|---|---|---|---|
| 45 | 276 through 1040 | 276 through 485 | 486 through 1040 | 1041 | - | 2024 through 2036 |

**TABLE V**

| Id | Full Length Polypeptide Location | Signal Peptide Location | Mature Polypeptide Location |
|---|---|---|---|
| 146 | -70 through 185 | -70 through -1 | 1 through 185 |

**TABLE VI**

| Id | Collection Refs | Deposit Name |
|---|---|---|
| 45 | ATCC # 98920 | SignalTaq 67-90 |

**TABLE VII**

| Internal Designation Number | SEQ ID NO | Type of Sequence |
|---|---|---|
| 26-27-3-D7-CL0_1 | 45 | DNA |
| 26-27-3-D7-CL0_1 | 146 | PRT |

## Claims

1. A purified or isolated polypeptide comprising a sequence having at least 80% identity to the polypeptide of SEQ ID NO: 146.

2. The polypeptide of claim 1, wherein said polypeptide comprises SEQ ID NO: 146.

3. The polypeptide of claim 2, wherein said polypeptide consist of SEQ ID NO: 146.

4. A purified or isolated nucleic acid encoding the polypeptide of claim 2 or 3.

5. The nucleic acid of claim 4, wherein said nucleic acid comprises SEQ ID NO: 45.

6. An expression vector comprising the nucleic acid of claim 4 or 5.

7. A host cell containing a recombinant nucleic acid of claim 4 or 5.

8. A method of making the polypeptide of any of claims 1 to 3 comprising the steps of:
a. obtaining a cDNA encoding said polypeptide;
b. inserting said cDNA in an expression vector such that said cDNA is operably linked to a promoter; and
c. introducing said expression vector into a host cell whereby said host cell produces said polypeptide.

9. The method of claim 8, further comprising the step of isolating said polypeptide.

10. The method of claim 9, further comprising the step of purifying said polypeptide using immunochromatography techniques

11. A purified or isolated antibody capable of specifically binding to the polypeptide of any of claims 1 to 3.

## Patentansprüche

1. Gereinigtes oder isoliertes Polypeptid, umfassend eine Sequenz, die wenigstens 80 % Identität mit dem Polypeptid von SEQ ID NO: 146 aufweist.

2. Polypeptid nach Anspruch 1, wobei das Polypeptid SEQ ID NO: 146 umfasst.

3. Polypeptid nach Anspruch 2, wobei das Polypeptid aus SEQ ID NO: 146 besteht.

4. Gereinigte oder isolierte Nukleinsäure, die das Polypeptid nach Anspruch 2 oder 3 codiert.

5. Nukleinsäure nach Anspruch 4, wobei die Nukleinsäure SEQ ID NO: 45 umfasst.

6. Expressionsvektor, umfassend die Nukleinsäure nach Anspruch 4 oder 5.

7. Wirtszelle, enthaltend eine rekombinante Nukleinsäure nach Anspruch 4 oder 5.

8. Verfahren zum Herstellen des Polypeptids nach einem der Ansprüche 1 bis 3, umfassend die Schritte:
a. Erhalten einer cDNA, die das Polypeptid codiert;
b. Einfügen der cDNA in einen Expressionsvektor derart, dass die cDNA mit einem Promoter funktionell verbunden ist; und
c. Einführen des Expressionsvektors in eine Wirtszelle, wodurch die Wirtszelle das Polypeptid produziert.

9. Verfahren nach Anspruch 8, außerdem umfassend den Schritt des Isolierens des Polypeptids.

10. Verfahren nach Anspruch 9, außerdem umfassend den Schritt des Reinigens des Polypeptids unter Verwendung von Immunchromatografiemethoden.

11. Gereinigter oder isolierter Antikörper, der das Polypeptid nach einem der Ansprüche 1 bis 3 spezifisch binden kann.

## Revendications

1. Polypeptide purifié ou isolé comprenant une séquence étant au moins identique à 80% au polypeptide de Séquence n° 146.

2. Polypeptide selon la revendication 1, en quoi ledit polypeptide comprend la Séquence n° 146.

3. Polypeptide selon la revendication 2, en quoi ledit polypeptide est constitué de la Séquence n° 146.

4. Acide nucléique purifié ou isolé codant le polypeptide de la revendication 2 ou 3.

5. Acide nucléique selon la revendication 4, en quoi ledit acide nucléique comprend la Séquence n° 45.

6. Vecteur d'expression comprenant l'acide nucléique de la revendication 4 ou 5.

7. Cellule hôte contenant un acide nucléique recombiné de la revendication 4 ou 5.

8. Procédé de fabrication du polypeptide de l'une quelconque des revendication 1 à 3 comprenant les étapes consistant à:
a. obtenir un ADNc codant ledit polypeptide ;
b. insérer ledit ADNc dans un vecteur d'expression de sorte que ledit ADNc soit lié opérationnellement à un promoteur ;
c. et introduire ledit vecteur d'expression dans une cellule hôte ce par quoi ladite cellule hôte produit ledit polypeptide.

9. Procédé selon la revendication 8 comprenant en outre l'étape d'isolement dudit polypeptide.

10. Procédé selon la revendication 9 comprenant en outre l'étape de purification dudit polypeptide à l'aide de techniques d'immunochromatographie.

11. Anticorps purifié ou isolé capable de se lier spécifiquement au polypeptide de l'une quelconque des revendications 1 à 3.
